(19) Europäisches Patentamt
European Patent Office
Office européen des brevets

(11) EP 2 226 072 A1

(12) EUROPEAN PATENT APPLICATION

(43) Date of publication:
**08.09.2010 Bulletin 2010/36**

(51) Int Cl.:
***A61K 31/167*** (2006.01) ***A61K 31/7068*** (2006.01)
***A61K 45/06*** (2006.01) ***A61P 35/00*** (2006.01)

(21) Application number: **10158227.8**

(22) Date of filing: **12.08.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **29.08.2003 US 498803 P**

(62) Document number(s) of the earlier application(s) in accordance with Art. 76 EPC:
**04780925.6 / 1 667 680**

(71) Applicants:
- **Aton Pharma, Inc.**
  **Boston, MA 02115 (US)**
- **Sloan Kettering Institute For Cancer Research**
  **New York, NY 10021 (US)**

(72) Inventors:
- **Bacopoulos, Nicholas G.**
  **New York, NY 10023 (US)**
- **Chiao, Judy H.**
  **Berkeley Heights, NJ 07922 (US)**
- **Marks, Paul A.**
  **Washington, CT 06793 (US)**
- **Miller, Thomas A.**
  **Brookline, MA 02445 (US)**
- **Paradise, Carolyn M.**
  **New York, NY 10567 (US)**
- **Richon, Victoria M.**
  **Wellesley, MA 02481 (US)**
- **Rifkind, Richard A.**
  **New York, NY 10022 (US)**

(74) Representative: **Bevan, Emma et al**
**Mintz, Levin, Cohn, Ferris, Glovsky and Popeo IP, LLP**
**Alder Castle**
**10 Noble Street**
**London EC2V 7JX (GB)**

Remarks:
This application was filed on 29-03-2010 as a divisional application to the application mentioned under INID code 62.

(54) **Combinations of suberoylanilide hydroxamic acid and antimetbolic agents for treating cancer**

(57) The present invention relates to a method of treating cancer in a subject in need thereof, by administering to a subject in need thereof a first amount of a histone deacetylase (HDAC) inhibitor or a pharmaceutically acceptable salt or hydrate thereof, in a first treatment procedure, and a second amount of an anti-cancer agent in a second treatment procedure. The first and second amounts together comprise a therapeutically effective amount. The effect of the HDAC inhibitor and the anti-cancer agent may be additive or synergistic.

EP 2 226 072 A1

## Description

### FIELD OF THE INVENTION

The present invention relates to a method of treating cancer by administering a histone deacetylase (HDAC) inhibitor in combination with an anti-cancer agent. The first and second amounts together comprise a therapeutically effective amount.

### BACKGROUND OF THE INVENTION

Cancer is a disorder in which a population of cells has become, in varying degrees, unresponsive to the control mechanisms that normally govern proliferation and differentiation.

Therapeutic agents used in clinical cancer therapy can be categorized into six groups: alkylating agents, antibiotic agents, antimetabolic agents, biologic agents, hormonal agents, and plant-derived agents.

Cancer therapy is also being attempted by the induction of terminal differentiation of the neoplastic cells (M. B., Roberts, A. B., and Driscoll, J. S. (1985) in Cancer: Principles and Practice of Oncology, eds. Hellman, S., Rosenberg, S. A., and DeVita, V. T., Jr., Ed. 2, (J. B. Lippincott, Philadelphia), P. 49). In cell culture models, differentiation has been reported by exposure of cells to a variety of stimuli, including: cyclic AMP and retinoic acid (Breitman, T. R., Selonick, S. E., and Collins, S. J. (1980) Proc. Natl. Acad. Sci. USA 77: 2936-2940; Olsson, I. L. and Breitman, T. R. (1982) Cancer Res. 42: 3924-3927), aclarubicin and other anthracyclines (Schwartz, E. L. and Sartorelli, A. C. (1982) Cancer Res. 42: 2651-2655). There is abundant evidence that neoplastic transformation does not necessarily destroy the potential of cancer cells to differentiate (Sporn et al; Marks, P. A., Sheffery, M., and Rifkind, R. A. (1987) Cancer Res. 47: 659; Sachs, L. (1978) Nature (Lond.) 274: 535).

There are many examples of tumor cells which do not respond to the normal regulators of proliferation and appear to be blocked in the expression of their differentiation program, and yet can be induced to differentiate and cease replicating. A variety of agents can induce various transformed cell lines and primary human tumor explants to express more differentiated characteristics. These agents include:

a) Polar compounds (Marks et al (1987); Friend, C., Scher, W., Holland, J. W., and Sato, T. (1971) Proc. Natl. Acad. Sci. (USA) 68: 378-382; Tanaka, M., Levy, J., Terada, M., Breslow, R., Rifkind, R. A., and Marks, P. A. (1975) Proc. Natl. Acad. Sci. (USA) 72: 1003-1006; Reuben, R. C., Wife, R. L., Breslow, R., Rifkind, R. A., and Marks, P. A. (1976) Proc. Natl. Acad. Sci. (USA) 73: 862-866);

b) Derivatives of vitamin D and retinoic acid (Abe, E., Miyaura, C., Sakagami, H., Takeda, M., Konno, K., Yamazaki, T., Yoshika, S., and Suda, T. (1981) Proc. Natl, Acad, Sci. (USA) 78: 4990-4994; Schwartz, E. L., Snoddy, J. R., Kreutter, D., Rasmussen, H., and Sartorelli, A. C. (1983) Proc. Am. Assoc. Cancer Res. 24: 18; Tanenaga, K., Hozumi, M., and Sakagami, Y. (1980) Cancer Res. 40: 914-919);

c) Steroid hormones (Lotem, J. and Sachs, L. (1975) Int. J. Cancer 15: 731-740);

d) Growth factors (Sachs, L. (1978) Nature (Lond.) 274: 535, Metcalf, D. (1985) Science, 229: 16-22);

e) Proteases (Scher, W., Scher, B. M., and Waxman, S. (1983) Exp. Hematol. 11: 490-498; Scher, W., Scher, B. M., and Waxman, S. (1982) Biochem. & Biophys. Res. Comm. 109: 348-354);

f) Tumor promoters (Huberman, E. and Callaham, M. F. (1979) Proc. Natl. Acad. Sci. (USA) 76: 1293-1297; Lottem, J. and Sachs, L. (1979) Proc. Natl. Acad. Sci. (USA) 76: 5158-5162); and

g) inhibitors of DNA or RNA synthesis (Schwartz, E. L. and Sartorelli, A. C. (1982) Cancer Res. 42: 2651-2655, Terada, M., Epner, E., Nudel, U., Salmon, J., Fibach, E., Rifkind, R. A., and Marks, P. A. (1978) Proc. Natl. Acad. Sci. (USA) 75: 2795-2799; Morin, M. J. and Sartorelli, A. C. (1984) Cancer Res. 44: 2807-2812; Schwartz, E. L., Brown, B. J., Nierenberg, M., Marsh, J. C., and Sartorelli, A. C. (1983) Cancer Res. 43: 2725-2730; Sugano, H., Furusawa, M., Kawaguchi, T., and Ikawa, Y. (1973) Bibl. Hematol. 39: 943-954; Ebert, P. S., Wars, I., and Buell, D. N. (1976) Cancer Res. 36: 1809-1813; Hayashi, M., Okabe, J., and Hozumi, M. (1979) Gann 70: 235-238).

Histone deacetylase inhibitors such as suberoylanilide hydroxamide acid (SAHA), belong to this class of agents that have the ability to induce tumor cell growth arrest, differentiation and/or apoptosis (Richon, V.M., Webb, Y., Merger, R., et al. (1996) PNAS 93:5705-8). These compounds are targeted towards mechanisms inherent to the ability of a neoplastic cell to become malignant, as they do not appear to have toxicity in doses effective for inhibition of tumor growth in animals (Cohen, L.A., Amin, S., Marks, P.A., Rifkind, R.A., Desai, D., and Richon, V.M. (1999) Anticancer Research 19:4999-5006). There are several lines of evidence that histone acetylation and deacetylation are mechanisms by which transcriptional regulation in a cell is achieved (Grunstein, M. (1997) Nature 389:349-52). These effects are thought to occur through changes in the structure of chromatin by altering the affinity of histone proteins for coiled DNA in the nucleosome. There are five types of histones that have been identified (designated H1, H2A, H2B, H3 and H4).

Histones H2A, H2B, H3 and H4 are found in the nucleosomes and H1 is a linker located between nucleosomes. Each nucleosome contains two of each histone type within its core, except for H1, which is present singly in the outer portion of the nucleosome structure. It is believed that when the histone proteins are hypoacetylated, there is a greater affinity of the histone to the DNA phosphate backbone This affinity causes DNA to be tightly bound to the histone and renders the DNA inaccessible to transcriptional regulatory elements and machinery. The regulation of acetylated states occurs through the balance of activity between two enzyme complexes, histone acetyl transferase (HAT) and histone deacetylase (HDAC). The hypoacetylated state is thought to inhibit transcription of associated DNA. This hypoacetylated state is catalyzed by large multiprotein complexes that include HDAC enzymes. In particular, HDACs have been shown to catalyze the removal of acetyl groups from the chromatin core histones.

The inhibition of HDAC by SAHA is thought occur through direct interaction with the catalytic site of the enzyme as demonstrated by X-ray crystallography studies (Finnin, M.S., Donigian, J.R., Cohen, A., et al. (1999) Nature 401: 188-193). The result of HDAC inhibition is not believed to have a generalized effect on the genome, but rather, only affects a small subset of the genome (Van Lint, C., Emiliani, S., Verdin, E. (1996) Gene Expression 5:245-53). Evidence provided by DNA microarrays using malignant cell lines cultured with a HDAC inhibitor shows that there are a finite (1-2%) number of genes whose products are altered. For example, cells treated in culture with HDAC inhibitors show a consistent induction of the cyclin-dependent kinase inhibitor p21 (Archer, S. Shufen, M. Shei, A., Hodin, R. (1998) PNAS 95:6791-96). This protein plays an important role in cell cycle arrest. HDAC inhibitors are thought to increase the rate of transcription of p21 by propagating the hyperacetylated state of histones in the region of the p21 gene, thereby making the gene accessible to transcriptional machinery. Genes whose expression is not affected by HDAC inhibitors do not display changes in the acetylation of regional associated histones (Dressel, U., Renkawitz, R., Baniahmad, A. (2000) Anticancer Research 20(2A):1017-22).

It has been shown in several instances that the disruption of HAT or HDAC activity is implicated in the development of a malignant phenotype. For instance, in acute promyelocytic leukemia, the oncoprotein produced by the fusion of PML and RAR alpha appears to suppress specific gene transcription through the recruitment of HDACs (Lin, R.J., Nagy, L., Inoue, S., et al. (1998) Nature 391:811-14). In this manner, the neoplastic cell is unable to complete differentiation and leads to excess proliferation of the leukemic cell line.

U.S. Patent Numbers 5,369,108, 5,932,616, 5,700,811, 6,087,367 and 6,511,990, issued to some of the present inventors, disclose compounds useful for selectively inducing terminal differentiation of neoplastic cells, which compounds have two polar end groups separated by a flexible chain of methylene groups or a by a rigid phenyl group, wherein one or both of the polar end groups is a large hydrophobic group. Some of the compounds have an additional large hydrophobic group at the same end of the molecule as the first hydrophobic group which further increases differentiation activity about 100 fold in an enzymatic assay and about 50 fold in a cell differentiation assay. Methods of synthesizing the compounds used in the methods and pharmaceutical compositions of this invention are fully described the aforementioned patents, the entire contents of which are incorporated herein by reference.

Current tumor therapies are known which consist of the combinatorial treatment of patients with more than one anti-tumor therapeutic reagent. Examples are the combined use of irradiation treatment together with chemotherapeutic and/or cytotoxic reagents and more recently the combination of irradiation treatment with immunological therapies such as the use of tumor cell specific therapeutic antibodies. However, the possibility to combine individual treatments with each other in order to identify such combinations which are more effective than the individual approaches alone, requires extensive preclinical and clinical testing, and it is not possible without such experimentation to predict which combinations show an additive or even synergistic effect.

Besides the aim to increase the therapeutic efficacy, another purpose of combination treatment is the potential decrease of the doses of the individual components in the resulting combinations in order to decrease unwanted or harmful side effects caused by higher doses of the individual components.

There is an urgent need to discover suitable methods for the treatment of cancer, including combination treatments that result in decreased side effects and that are effective at treating and controlling malignancies.

## SUMMARY OF THE INVENTION

The present invention is based on the discovery that histone deacetylase (HDAC) inhibitors, for example suberoylanilide hydroxamic acid (SAHA), can be used in combination with one or more anti-cancer agents, to provide therapeutically effective anticancer effects.

It has been unexpectedly discovered that the combination of a first treatment procedure that includes administration of an HDAC inhibitor, as described herein, and a second treatment procedure using one or more anti-cancer agents, as described herein, can provide therapeutically effective anticancer effects. Each of the treatments (administration of an HDAC inhibitor and administration of the anti-cancer agent) is used in an amount or dose that in combination with the other provides a therapeutically effective treatment.

The combination therapy can act through the induction of cancer cell differentiation, cell growth arrest and/or

apoptosis. Furthermore, the effect of the HDAC inhibitor and the anti-cancer agent may be additive or synergistic. The combination of therapy is particularly advantageous, since the dosage of each agent in a combination therapy can be reduced as compared to monotherapy with the agent, while still achieving an overall anti-tumor effect.

As such, the present invention relates to a method of treating cancer in a subject in need thereof, by administering to a subject in need thereof a first amount of suberoylanilide hydroxamic acid (SAHA) or a pharmaceutically acceptable salt or hydrate thereof, in a first treatment procedure, and a second amount of an anti-cancer agent in a second treatment procedure, wherein the first and second amounts together comprise a therapeutically effective amount.

Treatment of cancer, as used herein, refers to partially or totally inhibiting, delaying or preventing the progression of cancer including cancer metastasis; inhibiting, delaying or preventing the recurrence of cancer including cancer metastasis; or preventing the onset or development of cancer (chemoprevention) in a mammal, for example a human.

The methods of the present invention are useful in the treatment in a wide variety of cancers, including but not limited to solid tumors (e.g., tumors of the lung, breast, colon, prostate, bladder, rectum, brain or endometrium), hematological malignancies (e.g., leukemias, lymphomas, myelomas), carcinomas (e.g. bladder carcinoma, renal carcinoma, breast carcinoma, colorectal carcinoma), neuroblastoma, or melanoma. Non-limiting examples of these cancers include cutaneous T-cell lymphoma (CTCL), noncutaneous peripheral T-cell lymphoma, lymphoma associated with human T-cell lymphotrophic virus (HTLV), adult T-cell leukemia/lymphoma (ATLL), acute lymphocytic leukemia, acute nonlymphocytic leukemia, chronic lymphocytic leukemia, chronic myelogenous leukemia, Hodgkin's disease, non-Hodgkin's lymphoma, multiple myeloma, mesothelioma, childhood solid tumors such as brain neuroblastoma, retinoblastoma, Wilms' tumor, bone cancer and soft-tissue sarcomas, common solid tumors of adults such as head and neck cancers (e.g., oral, laryngeal and esophageal), genito urinary cancers (e.g., prostate, bladder, renal, uterine, ovarian, testicular, rectal and colon), lung cancer, breast cancer, pancreatic cancer, melanoma and other skin cancers, stomach cancer, brain cancer, liver cancer, adrenal cancer, kidney cancer, thyroid cancer, basal cell carcinoma, squamous cell carcinoma of both ulcerating and papillary type, metastatic skin carcinoma, medullary carcinoma, osteo sarcoma, Ewing's sarcoma, veticulum cell sarcoma, Kaposi's sarcoma, neuroblastoma and retinoblastoma.

The method comprises administering to a patient in need thereof a first amount of an HDAC inhibitor, e.g., SAHA, in a first treatment procedure, and a second amount of an anti-cancer agent in a second treatment procedure. The first and second treatments together comprise a therapeutically effective amount.

The invention further relates to pharmaceutical combinations useful for the treatment of cancer. The pharmaceutical combination comprises a first amount of an HDAC inhibitor, e.g., SAHA, and a second amount of an anti-cancer agent. The first and second amount together comprise a therapeutically effective amount.

The invention further relates to the use of a first amount of an HDAC inhibitor and a second amount of an anti-cancer agent for the manufacture of a medicament for treating cancer.

In particular embodiments of this invention, the combination of the HDAC inhibitor and anti-cancer agent is additive, i.e. the combination treatment regimen produces a result that is the additive effect of each constituent when it is administered alone. In accordance with this embodiment, the amount of HDAC inhibitor and the amount of the anti-cancer together constitute an effective amount to treat cancer.

In another particular embodiment of this invention, the combination of the HDAC inhibitor and anti-cancer agent is considered therapeutically synergistic when the combination treatment regimen produces a significantly better anti-cancer result (e.g., cell growth arrest, apoptosis, induction of differentiation, cell death) than the additive effects of each constituent when it is administered alone at a therapeutic dose. Standard statistical analysis can be employed to determine when the results are significantly better. For example, a Mann-Whitney Test or some other generally accepted statistical analysis can be employed.

The treatment procedures can take place sequentially in any order, simultaneously or a combination thereof. For example, the first treatment procedure, administration of an HDAC inhibitor, can take place prior to the second treatment procedure, i.e. the anti-cancer agent, after the second treatment with the anticancer agent, at the same time as the second treatment with the anticancer agent, or a combination thereof. For example, a total treatment period can be decided for the HDAC inhibitor. The anti-cancer agent can be administered prior to onset of treatment with the HDAC inhibitor or following treatment with the HDAC inhibitor. In addition, treatment with the anti-cancer agent can be administered during the period of HDAC inhibitor administration but does not need to occur over the entire HDAC inhibitor treatment period. Similarly, treatment with the HDAC inhibitor can be administered during the period of anti-cancer agent administration but does not need to occur over the entire anti-cancer agent treatment period. In another embodiment, the treatment regimen includes pre-treatment with one agent, either the HDAC inhibitor or the anti-cancer agent, followed by the addition of the second agent for the duration of the treatment period.

In one particular embodiment of the present invention, the HDAC inhibitor can be administered in combination with any one or more of an additional HDAC inhibitor, an alkylating agent, an antibiotic agent, an antimetabolic agent, a hormonal agent, a plant-derived agent, an anti-angiogenic agent, a differentiation inducing agent, a cell growth arrest inducing agent, an apoptosis inducing agent, a cytotoxic agent, a biologic agent, a gene therapy agent, or any combination thereof.

In one particular embodiment of the present invention, the HDAC inhibitor is suberoylanilide hydroxamic acid (SAHA), which can be administered in combination with any one or more of another HDAC inhibitor, an alkylating agent, an antibiotic agent, an antimetabolic agent, a hormonal agent, a plant-derived agent, an anti-angiogenic agent, a differentiation inducing agent, a cell growth arrest inducing agent, an apoptosis inducing agent, a cytotoxic agent, a biologic agent, a gene therapy agent, or any combination thereof.

HDAC inhibitors suitable for use in the present invention, include but are not limited to hydroxamic acid derivatives, Short Chain Fatty Acids (SCFAs), cyclic tetrapeptides, benzamide derivatives, or electrophilic ketone derivatives, as defined herein. Specific non-limiting examples of HDAC inhibitors suitable for use in the methods of the present invention are:

A) HYDROXAMIC ACID DERIVATIVES selected from SAHA, Pyroxamide, CBHA, Trichostatin A (TSA), Trichostatin C, Salicylbishydroxamic Acid, Azelaic Bishydroxamic Acid (ABHA), Azelaic-1-Hydroxamate-9-Anilide (AAHA), 6-(3-Chlorophenylureido) carpoic Hydroxamic Acid (3Cl-UCHA), Oxamflatin, A-161906, Scriptaid, PXD-101, LAQ-824, CHAP, MW2796, and MW2996;

B) CYCLIC TETRAPEPTIDES selected from Trapoxin A, FR901228 (FK 228 or Depsipeptide), FR225497, Apicidin, CHAP, HC-Toxin, WF27082, and Chlamydocin;

C) SHORT CHAIN FATTY ACIDS (SCFAs) selected from Sodium Butyrate, Isovalerate, Valerate, 4 Phenylbutyrate (4-PBA), Phenylbutyrate (PB), Propionate, Butyramide, Isobutyramide, Phenylacetate, 3-Bromopropionate, Tributyrin, Valproic Acid and Valproate;

D) BENZAMIDE DERIVATIVES selected from CI-994, MS-27-275 (MS-275) and a 3'-amino derivative of MS-27-275;

E) ELECTROPHILIC KETONE DERIVATIVES selected from a trifluoromethyl ketone and an α-keto amide such as an N-methyl- α-ketoamide; and

F) Miscellaneous HDAC inhibitors including natural products, psammaplins and Depudecin.

Specific HDAC inhibitors include:

Suberoylanilide hydroxamic acid (SAHA), which is represented by the following structural formula:

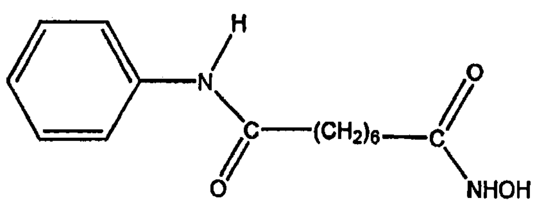

Pyroxamide, which is represented by the following structural formula:

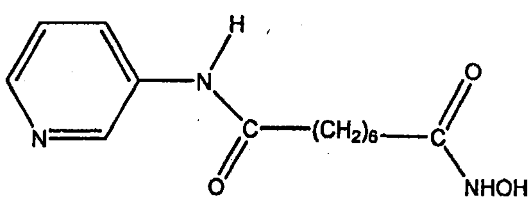

m-Carboxycinnamic acid bishydroxamate (CBHA), which is represented by the structural formula:

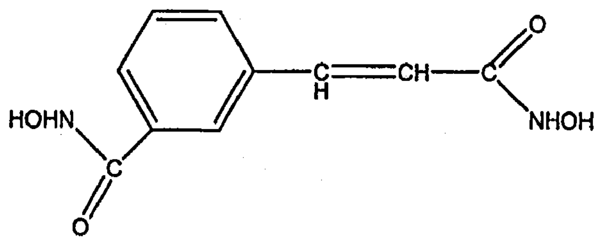

Other non-limiting examples of HDAC inhibitors that are suitable for use in the methods of the present invention are:

A compound represented by the structure:

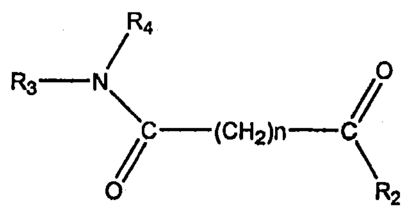

wherein $R_3$ and $R_4$ are independently a substituted or unsubstituted, branched or unbranched alkyl, alkenyl, cycloalkyl, aryl, alkyloxy, aryloxy, arylalkyloxy, or pyridine group, cycloalkyl, aryl, aryloxy, arylalkyloxy, or pyridine group, or $R_3$ and $R_4$ bond together to form a piperidine group; $R_2$ is a hydroxylamino group; and n is an integer from 5 to 8.

A compound represented by the structure:

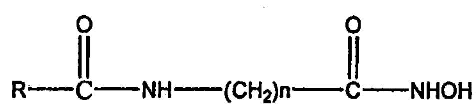

wherein R is a substituted or unsubstituted phenyl, piperidine, thiazole, 2-pyridine, 3- pyridine or 4-pyridine and n is an integer from 4 to 8.

A compound represented by the structure:

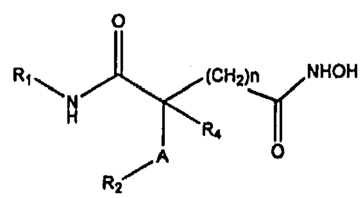

wherein A is an amide moiety, $R_1$ and $R_2$ are each selected from substituted or unsubstituted aryl, arylalkyl, naphthyl, pyridineamino, 9-purine-6-amino, thiazoleamino, aryloxy, arylalkyloxy, pyridyl, quinolinyl or isoquinolinyl; $R_4$ is hydrogen, a halogen, a phenyl or a cycloalkyl moiety and n is an integer from 3 to 10.

Alkylating agents suitable for use in the present invention, include but are not limited to bischloroethylamines (nitrogen mustards, e. g. chlorambucil, cyclophosphamide, ifosfamide, mechlorethamine, melphalan, uracil mustard), aziridines (e. g. thiotepa), alkyl alkone sulfonates (e. g. busulfan), nitrosoureas (e. g. carmustine, lomustine, streptozocin), nonclassic alkylating agents (altretamine, dacarbazine, and procarbazine), platinum compounds (carboplastin and cisplatin).

Antibiotic agents suitable for use in the present invention are anthracyclines (e. g. doxorubicin, daunorubicin, epirubicin, idarubicin and anthracenedione), mitomycin C, bleomycin, dactinomycin, plicatomycin.

Antimetabolic agents suitable for use in the present invention, include but are not limited to, floxuridine, fluorouracil, methotrexate, leucovorin, hydroxyurea, thioguanine, mercaptopurine, cytarabine, pentostatin, fludarabine phosphate, cladribine, asparaginase, and gemcitabine. In a particular embodiment, the antimetabolic agent in gemcitabine.

Hormonal agents suitable for use in the present invention, include but are not limited to, an estrogen, a progestogen, an antiesterogen, an androgen, an antiandrogen, an LHRH analogue, an aromatase inhibitor, diethylstibestrol, tamoxifen, toremifene, fluoxymesterol, raloxifene, bicalutamide, nilutamide, flutamide, aminoglutethimide, tetrazole, ketoconazole, goserelin acetate, leuprolide, megestrol acetate, and mifepristone.

Plant-derived agents suitable for use in the present invention include, but are not limited to vincristine, vinblastine, vindesine, vinzolidine, vinorelbine, etoposide teniposide, paclitaxel and docetaxel.

Biologic agents suitable for use in the present invention include, but are not limited to immuno-modulating proteins, monoclonal antibodies against tumor antigens, tumor suppressor genes, and cancer vaccines. For example, the immuno-modulating protein can be interleukin 2, interleukin 4, interleukin 12, interferon E1 interferon D, interferon alpha, erythropoietin, granulocyte-CSF, granulocyte, macrophage-CSF, bacillus Cahnette-Guerin, levamisole, or octreotide. Furthermore, the tumor suppressor gene can be DPC-4, NF-1, NF-2, RB, p53, WT1, BRCA, or BRCA2.

The HDAC inhibitor (e.g. SAHA), and the anti-cancer agent can be administered by any known administration method known to a person skilled in the art. Examples of routes of administration include but are not limited to oral, parenteral, intraperitoneal, intravenous, intraarterial, transdermal, sublingual, intramuscular, rectal, transbuccal, intranasal, liposomal, via inhalation, vaginal, intraoccular, via local delivery by catheter or stent, subcutaneous, intraadiposal, intraarticular, intrathecal, or in a slow release dosage form.

Of course, the route of administration of SAHA or any one of the other HDAC inhibitors is independent of the route of administration of the anti-cancer agent. A currently preferred route of administration for SAHA is oral administration. Thus, in accordance with this embodiment, SAHA is administered orally, and the second agent (anti-cancer agent) can be administered orally, parenterally, intraperitoneally, intravenously, intraarterially, transdermally, sublingually, intramuscularly, rectally, transbuccally, intranasally, liposomally, via inhalation, vaginally, intraoccularly, via local delivery by catheter or stent, subcutaneously, intraadiposally, intraarticularly, intrathecally, or in a slow release dosage form.

SAHA or any one of the HDAC inhibitors can be administered in accordance with any dose and dosing schedule that, together with the effect of the anti-cancer agent, achieves a dose effective to treat cancer. For example, SAHA or any one of the HDAC inhibitors can be administered in a total daily dose of up to 800 mg, preferably orally, once, twice or three times daily, continuously (every day) or intermittently (e.g., 3-5 days a week).

As such, the present invention relates to a method of treating cancer in a subject in need thereof, by administering to a subject in need thereof a first amount of suberoylanilide hydroxamic acid (SAHA) or a pharmaceutically acceptable salt or hydrate thereof at a total daily dose of up to 800 mg in a first treatment procedure, and a second amount of an anti-cancer agent in a second treatment procedure, wherein the first and second amounts together comprise a therapeutically effective amount.

In one embodiment, the HDAC inhibitor, e.g. SAHA, is administered in a pharmaceutical composition, preferably suited for oral administration. In a currently preferred embodiment, SAHA is administered orally in a gelating capsule, which can comprise excipients such as microcrystalline cellulose, croscarmellose sodium and magnesium stearate.

The HDAC inhibitors can be administered in a total daily dose that may vary from patient to patient, and may be administered at varying dosage schedules. Suitable dosages are total daily dosage of between about 25-4000 $mg/m^2$ administered orally once-daily, twice-daily or three times-daily, continuous (every day) or intermittently (e.g. 3-5 days a week). Furthermore, the compositions may be administered in cycles, with rest periods in between the cycles (e.g. treatment for two to eight weeks with a rest period of up to a week between treatments).

In one embodiment, the composition is administered once daily at a dose of about 200-600 mg. In another embodiment, the composition is administered twice daily at a dose of about 200-400 mg. In another embodiment, the composition is administered twice daily at a dose of about 200-400 mg intermittently, for example three, four or five days per week. In one embodiment, the daily dose is 200 mg which can be administered once-daily, twice-daily or three-times daily. In one embodiment, the daily dose is 300 mg which can be administered once-daily, twice-daily or three-times daily. In one embodiment, the daily dose is 400 mg which can be administered once-daily, twice-daily or three-times daily.

It is apparent to a person skilled in the art that any one or more of the specific dosages and dosage schedules of the HDAC inhibitors, is also applicable to any one or more of the anti-cancer agents to be used in the combination treatment. Moreover, the specific dosage and dosage schedule of the anti-cancer agent can further vary, and the optimal dose, dosing schedule and route of administration will be determined based upon the specific anti-cancer agent that is being used.

The present invention also provides methods for selectively inducing terminal differentiation, cell growth arrest and/or apoptosis of neoplastic cells, thereby inhibiting proliferation of such cells in a subject by administering to the subject a first amount of suberoylanilide hydroxamic acid (SAHA) or a pharmaceutically acceptable salt or hydrate thereof, in a first treatment procedure, and a second amount of an anti-cancer agent in a second treatment procedure, wherein the first and second amounts together comprise an amount effective to induce terminal differentiation, cell

growth arrest of apoptosis of the cells.

The present invention also provides in-vitro methods for selectively inducing terminal differentiation, cell growth arrest and/or apoptosis of neoplastic cells, thereby inhibiting proliferation of such cells, by contacting the cells with a first amount of suberoylanilide hydroxamic acid (SAHA) or a pharmaceutically acceptable salt or hydrate thereof, and a second amount of an anti-cancer agent, wherein the first and second amounts together comprise an amount effective to induce terminal differentiation, cell growth arrest of apoptosis of the cells.

The combination therapy can provide a therapeutic advantage in view of the differential toxicity associated with the two treatment modalities. For example, treatment with HDAC inhibitors can lead to a particular toxicity that is not seen with the anti-cancer agent, and vice versa. As such, this differential toxicity can permit each treatment to be administered at a dose at which said toxicities do not exist or are minimal, such that together the combination therapy provides a therapeutic dose while avoiding the toxicities of each of the constituents of the combination agents. Furthermore, when the therapeutic effects achieved as a result of the combination treatment are enhanced or synergistic, for example, significantly better than additive therapeutic effects, the doses of each of the agents can be reduced even further, thus lowering the associated toxicities to an even greater extent.

## BRIEF DESCRIPTION OF THE DRAWINGS

The foregoing and other objects, features and advantages of the invention will be apparent from the following more particular description of preferred embodiments of the invention, as illustrated in the accompanying drawings in which like reference characters refer to the same parts throughout the different views. The drawings are not necessarily to scale, emphasis instead being placed upon illustrating the principles of the invention.

**FIG.1:** Effect of SAHA and gemcitabine combination in a T24 cell line. Cells were left untreated (□), treated with 2 nM gemcitaine (0), with 5 μM SAHA (○), or treated with a combination of 2 nM gemcitabine and 5 μM SAHA (△) as described in the Experimental Section, for the indicated time points. Fig 1A shows cell proliferation and Fig 1B shows cell viability.

**FIG. 2:** Effect of SAHA and gemcitabine combination in a LnCaP cell line. Cells were left untreated (□), treated with 2 nM gemcitaine (0), with 5 μM SAHA (○), or treated with a combination of 2 nM gemcitabine and 5 μM SAHA (△) as described in the Experimental Section, for the indicated time points. Fig 2A shows cell proliferation and Fig 2B shows cell viability.

**FIG. 3:** Effect of SAHA and 5-azacytidine combination in a T24 cell line. Cells were left untreated (□), treated with 200 nM 5-azacytidine (AZ) (◇), with 5 μM SAHA (○), or treated with a combination of 200 nM 5-azacytidine and 5 μM SAHA (△) as described in the Experimental Section, for the indicated time points. Fig 3A shows cell proliferation and Fig 3B shows cell viability. The asterisk (*) indicates the time point of SAHA addition.

**FIG 4.** Effects of SAHA Combinations on MDA-231 Cell Proliferation. FIG 4A: Cells were pretreated with the indicated concentration of SAHA for 4 hours, washed, and then the second agent was added for 48 hours. FIG 4B: Cells were pretreated with the indicated concentration of SAHA for 48 hours, the second agent was added for 4 hours, and then the cells were washed. Cell growth was quantitated 48 hours later using the MTS assay.

**FIG 5.** Effects of SAHA Combinations on DU145 Cell Proliferation. Cells were pretreated with the indicated concentration of SAHA for 48 hours, the second agent was added for 4 hours, and then the cells were washed. Cell growth was quantitated 48 hours later using the MTS assay.

**FIG 6:** Effects of SAHA Combinations on DU145 Cell Clonogenicity. Cells were treated with SAHA for 48 hours, the second agent was then added for 4 hours and then the cells were washed. Colony formation was evaluated 2-3 weeks later.

**FIG 7:** Effects of SAHA Combinations on MDA-231 Cell Clonogenicity. Cells were treated with SAHA for 48 hours, the second agent was then added for 4 hours and then the cells were washed. Colony formation was evaluated 2-3 weeks later.

**FIG 8:** Effects of SAHA Combinations on U118 Cell Clonogenicity. Cells were treated with SAHA for 48 hours, the second agent was then added for 4 hours and then the cells were washed. Colony formation was evaluated 2-3 weeks later.

**FIG 9:** Percent inhibition of LnCap cells treated with SAHA and Irinotecan. Cells were incubated with SAHA alone, Irinotecan alone, and a combination of SAHA and Irinotecan at the indicated concentrations. The right hand bar of each experiment represents the calculated effect for an additive interaction.

**FIG 10:** Percent inhibition of LnCap cells treated with SAHA and 5-Fluorouracil (5-FU). Cells were incubated with SAHA alone, 5-FU alone, and a combination of SAHA and 5-FU at the indicated concentrations. The right hand bar of each experiment represents the calculated effect for an additive interaction.

**FIG 11:** Percent inhibition of LnCap cells treated with SAHA and Docetaxel. Cells were incubated with SAHA alone, Docetaxel alone, and a combination of SAHA and Docetaxel at the indicated concentrations. The right hand bar of each experiment represents the calculated effect for an additive interaction.

## DETAILED DESCRIPTION OF THE INVENTION

The present invention relates to a method of treating cancer in a subject in need thereof, by administering to a subject in need thereof a first amount of an HDAC inhibitor or a pharmaceutically acceptable salt or hydrate thereof, in a first treatment procedure, and a second amount of an anti-cancer agent in a second treatment procedure, wherein the first and second amounts together comprise a therapeutically effective amount. The effect of the HDAC inhibitor and the anti-cancer agent may be additive or synergistic.

The present invention also relates to a method of treating cancer in a subject in need thereof, by administering to a subject in need thereof a first amount of suberoylanilide hydroxamic acid (SAHA) or a pharmaceutically acceptable salt or hydrate thereof, in a first treatment procedure, and a second amount of an anti-cancer agent in a second treatment procedure, wherein the first and second amounts together comprise a therapeutically effective amount. The effect of SAHA and the anti-cancer agent may be additive or synergistic.

The term "treating" in its various grammatical forms in relation to the present invention refers to preventing (i.e. chemoprevention), curing, reversing, attenuating, alleviating, minimizing, suppressing or halting the deleterious effects of a disease state, disease progression, disease causative agent (e.g., bacteria or viruses) or other abnormal condition. For example, treatment may involve alleviating a symptom (i.e., not necessary all symptoms) of a disease or attenuating the progression of a disease. Because some of the inventive methods involve the physical removal of the etiological agent, the artisan will recognize that they are equally effective in situations where the inventive compound is administered prior to, or simultaneous with, exposure to the etiological agent (prophylactic treatment) and situations where the inventive compounds are administered after (even well after) exposure to the etiological agent.

Treatment of cancer, as used herein, refers to partially or totally inhibiting, delaying or preventing the progression of cancer including cancer metastasis; inhibiting, delaying or preventing the recurrence of cancer including cancer metastasis; or preventing the onset or development of cancer (chemoprevention) in a mammal, for example a human. In addition, the method of the present invention is intended for the treatment of chemoprevention of human patients with cancer. However, it is also likely that the method would be effective in the treatment of cancer in other mammals.

As used herein, the term "therapeutically effective amount" is intended to qualify the combined amount of the first and second treatments in the combination therapy. The combined amount will achieve the desired biological response. In the present invention, the desired biological response is partial or total inhibition, delay or prevention of the progression of cancer including cancer metastasis; inhibition, delay or prevention of the recurrence of cancer including cancer metastasis; or the prevention of the onset or development of cancer (chemoprevention) in a mammal, for example a human.

As used herein, the terms "combination treatment", "combination therapy", "combined treatment" or "combinatorial treatment", used interchangeably, refer to a treatment of an individual with at least two different therapeutic agents. According to the invention, the individual is treated with a first therapeutic agent, preferably SAHA or another HDAC inhibitor as described herein. The second therapeutic agent may be another HDAC inhibitors, or may be any clinically established anti-cancer agent as defined herein. A combinatorial treatment may include a third or even further therapeutic agent.

The method comprises administering to a patient in need thereof a first amount of a histone deacetylase inhibitor, e.g., SAHA, in a first treatment procedure, and a second amount of an anti-cancer agent in a second treatment procedure. The first and second treatments together comprise a therapeutically effective amount.

The invention further relates to pharmaceutical combinations useful for the treatment of cancer. The pharmaceutical combination comprises a first amount of an HDAC inhibitor, e.g., SAHA and a second amount of an anti-cancer agent. The first and second amount together comprise a therapeutically effective amount.

The invention further relates to the use of a first amount of an HDAC inhibitor and a second amount of an anti-cancer agent for the manufacture of a medicament for treating cancer.

In particular embodiments of this invention, the combination of the HDAC inhibitor and anti-cancer agent is

additive, i.e. the combination treatment regimen produces a result that is the additive effect of each constituent when it is administered alone. In accordance with this embodiment, the amount of HDAC inhibitor and the amount of the anti-cancer together constitute an effective amount to treat cancer.

In another particular embodiments of this invention, the combination of the HDAC inhibitor and anti-cancer agent is considered therapeutically synergistic when the combination treatment regimen produces a significantly better anticancer result (e.g., cell growth arrest, apoptosis, induction of differentiation, cell death) than the additive effects of each constituent when it is administered alone at a therapeutic dose. Standard statistical analysis can be employed to determine when the results are significantly better. For example, a Mann-Whitney Test or some other generally accepted statistical analysis can be employed.

The treatment procedures can take place sequentially in any order, simultaneously or a combination thereof. For example, the first treatment procedure, administration of an HDAC inhibitor, can take place prior to the second treatment procedure, i.e., the anti-cancer agent, after the second treatment with the anticancer agent, at the same time as the second treatment with the anticancer agent, or a combination thereof. For example, a total treatment period can be decided for the HDAC inhibitor. The anti-cancer agent can be administered prior to onset of treatment with the HDAC inhibitor or following treatment with the HDAC inhibitor. In addition, treatment with the anti-cancer agent can be administered during the period of HDAC inhibitor administration but does not need to occur over the entire HDAC inhibitor treatment period. In another embodiment, the treatment regimen includes pre-treatment with one agent, either the HDAC inhibitor or the anti-cancer agent, followed by the addition of the second agent.

The methods of the present invention are useful in the treatment in a wide variety of cancers, including but not limited to solid tumors (e.g., tumors of the lung, breast, colon, prostate, bladder, rectum, brain or endometrium), hematological malignancies (e.g., leukemias, lymphomas, myelomas), carcinomas (e.g. bladder carcinoma, renal carcinoma, breast carcinoma, colorectal carcinoma), neuroblastoma, or melanoma. Non-limiting examples of these cancers include cutaneous T-cell lymphoma (CTCL), noncutaneous peripheral T-cell lymphoma, lymphoma associated with human T-cell lymphotrophic virus (HTLV), adult T-cell leukemia/lymphoma (ATLL), acute lymphocytic leukemia, acute nonlymphocytic leukemia, chronic lymphocytic leukemia, chronic myelogenous leukemia, Hodgkin's disease, non-Hodgkin's lymphoma, multiple myeloma, mesothelioma, childhood solid tumors such as brain neuroblastoma, retinoblastoma, Wilms' tumor, bone cancer and soft-tissue sarcomas, common solid tumors of adults such as head and neck cancers (e.g., oral, laryngeal and esophageal), genito urinary cancers (e.g., prostate, bladder, renal, uterine, ovarian, testicular, rectal and colon), lung cancer, breast cancer, pancreatic cancer, melanoma and other skin cancers, stomach cancer, brain cancer, liver cancer, adrenal cancer, kidney cancer, thyroid cancer, basal cell carcinoma, squamous cell carcinoma of both ulcerating and papillary type, metastatic skin carcinoma, medullary carcinoma, osteo sarcoma, Ewing's sarcoma, veticulum cell sarcoma, Kaposi's sarcoma, neuroblastoma and retinoblastoma.

In one particular embodiment of the present invention, the HDAC inhibitor can be administered in combination with an additional HDAC inhibitor. In another particular embodiment of the present invention, the HDAC inhibitor can be administered in combination with an alkylating agent. In another particular embodiment of the present invention, the HDAC inhibitor can be administered in combination with an antibiotic agent. In another particular embodiment of the present invention, In another particular embodiment of the present invention, the HDAC inhibitor can be administered in combination with an antimetabolic agent. In another particular embodiment of the present invention, the HDAC inhibitor can be administered in combination with a hormonal agent. In another particular embodiment of the present invention, the HDAC inhibitor can be administered in combination with a plant-derived agent. In another particular embodiment of the present invention, the HDAC inhibitor can be administered in combination with an anti-angiogenic agent. In another particular embodiment of the present invention, the HDAC inhibitor can be administered in combination with a differentiation inducing agent. In another particular embodiment of the present invention, the HDAC inhibitor can be administered in combination with a cell growth arrest inducing agent. In another particular embodiment of the present invention, the HDAC inhibitor can be administered in combination with an apoptosis inducing agent. In another particular embodiment of the present invention, the HDAC inhibitor can be administered in combination with a cytotoxic agent. In another particular embodiment of the present invention, the HDAC inhibitor can be administered in combination with a biologic agent. In another particular embodiment of the present invention, the HDAC inhibitor can be administered in combination with any combination of an additional HDAC inhibitor, an alkylating agent, an antibiotic agent, an antimetabolic agent, a hormonal agent, a plant-derived agent, an anti-angiogenic agent, a differentiation inducing agent, a cell growth arrest inducing agent, an apoptosis inducing agent, a cytotoxic agent or a biologic agent.

In one particular embodiment of the present invention, the HDAC inhibitor is SAHA, which can be administered in combination with any one or more of another HDAC inhibitor, an alkylating agent, an antibiotic agent, an antimetabolic agent, a hormonal agent, a plant-derived agent, an anti-angiogenic agent, a differentiation inducing agent, a cell growth arrest inducing agent, an apoptosis inducing agent, a cytotoxic agent, a biologic agent, a gene therapy agent, or any combination thereof.

The combination therapy can provide a therapeutic advantage in view of the differential toxicity associated with the two treatment modalities. For example, treatment with HDAC inhibitors can lead to a particular toxicity that is not

seen with the anti-cancer agent, and vice versa. As such, this differential toxicity can permit each treatment to be administered at a dose at which said toxicities do not exist or are minimal, such that together the combination therapy provides a therapeutic dose while avoiding the toxicities of each of the constituents of the combination agents. Furthermore, when the therapeutic effects achieved as a result of the combination treatment are enhanced or synergistic, for example, significantly better than additive therapeutic effects, the doses of each of the agents can be reduced even further, thus lowering the associated toxicities to an even greater extent.

## Histone Deacetylases and Histone Deacetylase Inhibitors

Histone deacetylases (HDACs), as that term is used herein, are enzymes that catalyze the removal of acetyl groups from lysine residues in the amino terminal tails of the nucleosomal core histones. As such, HDACs together with histone acetyl transferases (HATs) regulate the acetylation status of histones. Histone acetylation affects gene expression and inhibitors of HDACs, such as the hydroxamic acid-based hybrid polar compound suberoylanilide hydroxamic acid (SAHA) induce growth arrest, differentiation and/or apoptosis of transformed cells *in vitro* and inhibit tumor growth *in vivo.* HDACs can be divided into three classes based on structural homology. Class I HDACs (HDACs 1, 2, 3 and 8) bear similarity to the yeast RPD3 protein, are located in the nucleus and are found in complexes associated with transcriptional co-repressors. Class II HDACs (HDACs 4, 5, 6, 7 and 9) are similar to the yeast HDA1 protein, and have both nuclear and cytoplasmic subcellular localization. Both Class I and II HDACs are inhibited by hydroxamic acid-based HDAC inhibitors, such as SAHA. Class III HDACs form a structurally distant class of NAD dependent enzymes that are related to the yeast SIR2 proteins and are not inhibited by hydroxamic acid-based HDAC inhibitors.

Histone deacetylase inhibitors or HDAC inhibitors, as that term is used herein are compounds that are capable of inhibiting the deacetylation of histones *in vivo, in vitro* or both. As such, HDAC inhibitors inhibit the activity of at least one histone deacetylase. As a result of inhibiting the deacetylation of at least one histone, an increase in acetylated histone occurs and accumulation of acetylated histone is a suitable biological marker for assessing the activity of HDAC inhibitors. Therefore, procedures that can assay for the accumulation of acetylated histones can be used to determine the HDAC inhibitory activity of compounds of interest. It is understood that compounds that can inhibit histone deacetylase activity can also bind to other substrates and as such can inhibit other biologically active molecules such as enzymes. It is also to be understood that the compounds of the present invention are capable of inhibiting any of the histone deacetylases set forth above, or any other histone deacetylases.

For example, in patients receiving HDAC inhibitors, the accumulation of acetylated histones in peripheral mononuclear cells as well as in tissue treated with HDAC inhibitors can be determined against a suitable control.

HDAC inhibitory activity of a particular compound can be determined *in vitro* using, for example, an enzymatic assays which shows inhibition of at least one histone deacetylase. Further, determination of the accumulation of acetylated histones in cells treated with a particular composition can be determinative of the HDAC inhibitory activity of a compound.

Assays for the accumulation of acetylated histones are well known in the literature. See, for example, Marks, P.A. et al., J. Natl. Cancer Inst., 92:1210-1215, 2000, Butler, L.M. et al., Cancer Res. 60:5165-5170 (2000), Richon, V. M. et al., Proc. Natl. Acad. Sci., USA, 95:3003-3007, 1998, and Yoshida, M. et al., J. Biol. Chem., 265:17174-17179, 1990.

For example, an enzymatic assay to determine the activity of an HDAC inhibitor compound can be conducted as follows. Briefly, the effect of an HDAC inhibitor compound on affinity purified human epitope-tagged (Flag) HDAC1 can be assayed by incubating the enzyme preparation in the absence of substrate on ice for about 20 minutes with the indicated amount of inhibitor compound. Substrate ([$^3$H]acetyl-labelled murine erythroleukemia cell-derived histone) can be added and the sample can be incubated for 20 minutes at 37°C in a total volume of 30 $\mu$L. The reaction can then be stopped and released acetate can be extracted and the amount of radioactivity release determined by scintillation counting. An alternative assay useful for determining the activity of an HDAC inhibitor compound is the "HDAC Fluorescent Activity Assay; Drug Discovery Kit-AK-500" available from BIOMOL® Research Laboratories, Inc., Plymouth Meeting, PA.

In vivo studies can be conducted as follows. Animals, for example, mice, can be injected intraperitoneally with an HDAC inhibitor compound. Selected tissues, for example, brain, spleen, liver etc, can be isolated at predetermined times, post administration. Histones can be isolated from tissues essentially as described by Yoshida et al., J. Biol. Chem. 265:17174-17179, 1990. Equal amounts of histones (about 1 $\mu$g) can be electrophoresed on 15% SDS-polyacrylamide gels and can be transferred to Hybond-P filters (available from Amersham). Filters can be blocked with 3% milk and can be probed with a rabbit purified polyclonal anti-acetylated histone H4 antibody ($\alpha$Ac-H4) and anti-acetylated histone H3 antibody ($\alpha$Ac-H3) (Upstate Biotechnology, Inc.). Levels of acetylated histone can be visualized using a horseradish peroxidase-conjugated goat anti-rabbit antibody (1:5000) and the SuperSignal chemiluminescent substrate (Pierce). As a loading control for the histone protein, parallel gels can be run and stained with Coomassie Blue (CB).

In addition, hydroxamic acid-based HDAC inhibitors have been shown to up regulate the expression of the $p21^{WAFI}$ gene. The $p21^{WAFI}$ protein is induced within 2 hours of culture with HDAC inhibitors in a variety of transformed cells using standard methods. The induction of the $p21^{WAFI}$ gene is associated with accumulation of acetylated histones

in the chromatin region of this gene. Induction of p21$^{WAFI}$ can therefore be recognized as involved in the G1 cell cycle arrest caused by HDAC inhibitors in transformed cells.

HDAC inhibitors are effective at treating a broader range of diseases characterized by the proliferation of neoplastic diseases, such as any one of the cancers described herein. However, the therapeutic utility of HDAC inhibitors is not limited to the treatment of cancer. Rather, there is a wide range of diseases for which HDAC inhibitors have been found useful.

For example, HDAC inhibitors, in particular SAHA, have been found to be useful in the treatment of a variety of acute and chronic inflammatory diseases, autoimmune diseases, allergic diseases, diseases associated with oxidative stress, and diseases characterized by cellular hyperproliferation. Non-limiting examples are inflammatory conditions of a joint including and rheumatoid arthritis (RA) and psoriatic arthritis; inflammatory bowel diseases such as Crohn's disease and ulcerative colitis; spondyloarthropathies; scleroderma; psoriasis (including T-cell mediated psoriasis) and inflammatory dermatoses such an dermatitis, eczema, atopic dermatitis, allergic contact dermatitis, urticaria; vasculitis (e.g., necrotizing, cutaneous, and hypersensitivity vasculitis); eosinphilic myositis, eosinophilic fasciitis; cancers with leukocyte infiltration of the skin or organs, ischemic injury, including cerebral ischemia (e.g., brain injury as a result of trauma, epilepsy, hemorrhage or stroke, each of which may lead to neurodegeneration); HIV, heart failure, chronic, acute or malignant liver disease, autoimmune thyroiditis; systemic lupus erythematosus, Sjorgren's syndrome, lung diseases (e.g., ARDS); acute pancreatitis; amyotrophic lateral sclerosis (ALS); Alzheimer's disease; cachexia/anorexia; asthma; atherosclerosis; chronic fatigue syndrome, fever; diabetes (e.g., insulin diabetes or juvenile onset diabetes); glomerulonephritis; graft versus host rejection (e.g., in transplantation),; hemohorragic shock; hyperalgesia: inflammatory bowel disease; multiple sclerosis; myopathies (e.g., muscle protein metabolism, esp. in sepsis); osteoporosis; Parkinson's disease; pain; pre-term labor; psoriasis; reperfusion injury; cytokine-induced toxicity (e.g., septic shock, endotoxic shock); side effects from radiation therapy, temporal mandibular joint disease, tumor metastasis; or an inflammatory condition resulting from strain, sprain, cartilage damage, trauma such as burn, orthopedic surgery, infection or other disease processes. Allergic diseases and conditions, include but are not limited to respiratory allergic diseases such as asthma, allergic rhinitis, hypersensitivity lung diseases, hypersensitivity pneumonitis, eosinophilic pneumonias (e.g., Loeffler's syndrome, chronic eosinophilic pneumonia), delayed-type hypersentitivity, interstitial lung diseases (ILD) (e.g., idiopathic pulmonary fibrosis, or ILD associated with rheumatoid arthritis, systemic lupus erythematosus, ankylosing spondylitis, systemic sclerosis, Sjogren's syndrome, polymyositis or dermatomyositis); systemic anaphylaxis or hypersensitivity responses, drug allergies (e.g., to penicillin, cephalosporins), insect sting allergies, and the like.

For example, HDAC inhibitors, and in particular SAHA, have been found to be useful in the treatment of a variety of neurodegenerative diseases, a non-exhaustive list of which is:

I. Disorders characterized by progressive dementia in the absence of other prominent neurologic signs, such as Alzheimer's disease; Senile dementia of the Alzheimer type; and Pick's disease (lobar atrophy).

II. Syndromes combining progressive dementia with other prominent neurologic abnormalities such as A) syndromes appearing mainly in adults (e.g., Huntington's disease, Multiple system atrophy combining dementia with ataxia and/ormanifestations of Parkinson's disease, Progressive supranuclear palsy (Steel-Richardson-Olszewski), diffuse Lewy body disease, and corticodentatonigral degeneration); and B) syndromes appearing mainly in children or young adults (e.g., Hallervorden-Spatz disease and progressive familial myoclonic epilepsy).

III. Syndromes of gradually developing abnormalities of posture and movement such as paralysis agitans (Parkinson's disease), striatonigral degeneration, progressive supranuclear palsy, torsion dystonia (torsion spasm; dystonia musculorum deformans), spasmodic torticollis and other dyskinesis, familial tremor, and Gilles de la Tourette syndrome.

IV. Syndromes of progressive ataxia such as cerebellar degenerations (e.g., cerebellar cortical degeneration and olivopontocerebellar atrophy (OPCA)); and spinocerebellar degeneration (Friedreich's atazia and related disorders).

V. Syndrome of central autonomic nervous system failure (Shy-Drager syndrome).

VI. Syndromes of muscular weakness and wasting without sensory changes (motorneuron disease such as amyotrophic lateral sclerosis, spinal muscular atrophy (e.g., infantile spinal muscular atrophy (Werdnig-Hoffman), juvenile spinal muscular atrophy (Wohlfart-Kugelberg-Welander) and other forms of familial spinal muscular atrophy), primary lateral sclerosis, and hereditary spastic paraplegia.

VII. Syndromes combining muscular weakness and wasting with sensory changes (progressive neural muscular atrophy; chronic familial polyneuropathies) such as peroneal muscular atrophy (Charcot-Marie-Tooth), hypertrophic interstitial polyneuropathy (Dejerine-Sottas), and miscellaneous forms of chronic progressive neuropathy.

VIII. Syndromes of progressive visual loss such as pigmentary degeneration of the retina (retinitis pigmentosa), and hereditary optic atrophy (Leber's disease).

Typically, HDAC inhibitors fall into five general classes: 1) hydroxamic acid derivatives; 2) Short-Chain Fatty Acids (SCFAs); 3) cyclic tetrapeptides; 4) benzamides; and 5) electrophilic ketones.

Thus, the present invention includes within its broad scope compositions comprising HDAC inhibitors which are 1) hydroxamic acid derivatives; 2) Short-Chain Fatty Acids (SCFAs); 3) cyclic tetrapeptides; 4) benzamides; 5) electrophilic ketones; and/or any other class of compounds capable of inhibiting histone deacetylases, for use in inhibiting histone deacetylase, inducing terminal differentiation, cell growth arrest and/or apoptosis in neoplastic cells, and/or inducing differentiation, cell growth arrest and/or apoptosis of tumor cells in a tumor.

Non-limiting examples of such HDAC inhibitors are set forth below. It is understood that the present invention includes any salts, crystal structures, amorphous structures, hydrates, derivatives, metabolites, stereoisomers, structural isomers and prodrugs of the HDAC inhibitors described herein.

A. **Hydroxamic Acid Derivatives** such as suberoylanilide hydroxamic acid (SARA) (Richon et al., Proc. Natl. Acad. Sci. USA 95,3003-3007 (1998)); m-carboxycinnamic acid bishydroxamide (CBHA) (Richon *et al*., supra); pyroxamide; trichostatin analogues such as trichostatin A (TSA) and trichostatin C (Koghe et al. 1998. Biochem. Pharmacol. 56: 1359-1364); salicylbishydroxamic acid (Andrews et al., International J. Parasitology 30,761-768 (2000)); suberoyl bishydroxamic acid (SBHA) (U.S. Patent No. 5,608,108); azelaic bishydroxamic acid (ABHA) (Andrews *et al*., supra); azelaic-1-hydroxamate-9-anilide (AAHA) (Qiu et al., Mol. Biol. Cell 11, 2069-2083 (2000)); 6-(3-chlorophenylureido) carpoic hydroxamic acid (3Cl-UCHA); oxamflatin [(2E)-5-[3-[(phenylsufonyl) aminol phenyl]-pent-2-en-4-ynohydroxamic acid] (Kim et al. Oncogene, 18: 2461 2470 (1999)); A-161906, Scriptaid (Su et al. 2000 Cancer Research, 60: 3137-3142); PXD-101 (Prolifix); LAQ-824; CHAP; MW2796 (Andrews *et al*., supra); MW2996 (Andrews *et al*., supra); or any of the hydroxamic acids disclosed in U.S. Patent Numbers 5,369,108, 5,932,616, 5,700,811, 6,087,367 and 6,511, 990.

B. **Cyclic Tetrapeptides** such as trapoxin A (TPX)-cyclic tetrapeptide (cyclo-(L-phenylalanyl-L-phenylalanyl-D-pipecolinyl-L-2-amino-8-oxo-9,10-epoxy decanoyl)) (Kijima et al., J Biol. Chem. 268,22429-22435 (1993)); FR901228 (FK 228, depsipeptide) (Nakajima et al., Ex. Cell Res. 241,126-133 (1998)); FR225497 cyclic tetrapeptide (H. Mori et al., PCT Application WO 00/08048 (17 February 2000)); apicidin cyclic tetrapeptide [cyclo(N-O-methyl-L-tryptophanyl-L -isoleucinyl-D-pipecolinyl-L-2-amino-8-oxodecanoyl)] (Darkin-Rattray et al., Proc. Natl. Acad. Sci. USA 93,1314313147 (1996)); apicidin Ia, apicidin Ib, apicidin Ic, apicidin IIa, and apicidin IIb (P. Dulski *et al*., PCT Application WO 97/11366); CHAP, HC-toxin cyclic tetrapeptide (Bosch et al., Plant Cell 7, 1941-1950 (1995)); WF27082 cyclic tetrapeptide (PCT Application WO 98/48825); and chlamydocin (Bosch *et al*., supra).

C. **Short chain fatty acid (SCFA) derivatives** such as: sodium butyrate (Cousens et al., J. Biol. Chem. 254,1716-1723 (1979)); isovalerate (McBain et al., Biochem. Pharm. 53: 1357-1368 (1997)); valerate (McBain *et al.,* supra) ; 4-phenylbutyrate (4-PBA) (Lea and Tulsyan, Anticancer Research, 15,879-873 (1995)); phenylbutyrate (PB) (Wang et al., Cancer Research, 59, 2766-2799 (1999)); propionate (McBain *et al*., supra); butyramide (Lea and Tulsyan, supra); isobutyramide (Lea and Tulsyan, supra); phenylacetate (Lea and Tulsyan, supra); 3-bromopropionate (Lea and Tulsyan, supra); tributyrin (Guan et al., Cancer Research, 60,749-755 (2000)); valproic acid, valproate and Pivanex™.

D. **Benzamide derivatives** such as CI-994; MS-275 [N- (2-aminophenyl)-4- [N-(pyridin-3-yl methoxycarbonyl) aminomethyl] benzamide] (Saito et al., Proc. Natl. Acad. Sci. USA 96, 4592-4597 (1999)); and 3'-amino derivative of MS-275 (Saito *et al*., supra).

E. **Electrophilic ketone derivatives** such as trifluoromethyl ketones (Frey et al, Bioorganic & Med. Chem. Lett. (2002), 12, 3443-3447; U.S. 6,511,990) and $\alpha$-keto amides such as N-methyl- $\alpha$-ketoamides

F. **Other HDAC Inhibitors** such as natural products, psammaplins and Depudecin (Kwon et al. 1998. PNAS 95: 3356-3361).

Preferred hydroxamic acid based HDAC inhibitors are suberoylanilide hydroxamic acid (SAHA), m-carboxycinnamic acid bishydroxamate (CBHA) and pyroxamide. SAHA has been shown to bind directly in the catalytic pocket of the histone deacetylase enzyme. SAHA induces cell cycle arrest, differentiation and/or apoptosis of transformed cells in culture and inhibits tumor growth in rodents. SAHA is effective at inducing these effects in both solid tumors and hematological cancers. It has been shown that SAHA is effective at inhibiting tumor growth in animals with no toxicity to the animal. The SAHA-induced inhibition of tumor growth is associated with an accumulation of acetylated histones in the tumor. SAHA is effective at inhibiting the development and continued growth of carcinogen-induced (N-methylnitrosourea) mammary tumors in rats. SAHA was administered to the rats in their diet over the 130 days of the study. Thus, SAHA is a nontoxic, orally active antitumor agent whose mechanism of action involves the inhibition of histone deacetylase activity.

Preferred HDAC inhibitors are those disclosed in U.S. Patent Numbers 5,369,108, 5,932,616, 5,700,811, 6,087,367 and 6,511, 990, issued to some of the present inventors disclose compounds, the entire contents of which are incorporated herein by reference, non-limiting examples of which are set forth below:

In one embodiment, the HDAC inhibitor useful in the methods of the present invention is represented by the structure of formula 1, or a pharmaceutically acceptable salt or hydrate thereof, and a pharmaceutically acceptable carrier or excipient.

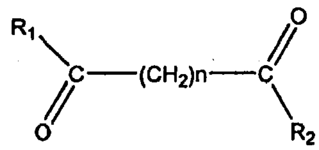

(1)

wherein $R_1$ and $R_2$ can be the same or different; when $R_1$ and $R_2$ are the same, each is a substituted or unsubstituted arylamino, cycloalkylamino, pyridineamino, piperidino, 9-purine-6-amine or thiazoleamino group; when $R_1$ and $R_2$ are different $R_1$=$R_3$-N-$R_4$, wherein each of $R_3$ and $R_4$ are independently the same as or different from each other and are a hydrogen atom, a hydroxyl group, a substituted or unsubstituted, branched or unbranched alkyl, alkenyl, cycloalkyl, aryl alkyloxy, aryloxy, arylalkyloxy or pyridine group, or $R_3$ and $R_4$ are bonded together to form a piperidine group, $R_2$ is a hydroxylamino, hydroxyl, amino, alkylamino, dialkylamino or alkyloxy group and n is an integer from about 4 to about 8.

In a particular embodiment of formula 1, $R_1$ and $R_2$ are the same and are a substituted or unsubstituted thiazoleamino group; and n is an integer from about 4 to about 8.

In one embodiment, the HDAC inhibitor useful in the methods of the present invention is represented by the structure of formula 2, or a pharmaceutically acceptable salt or hydrate thereof, and a pharmaceutically acceptable carrier or excipient.

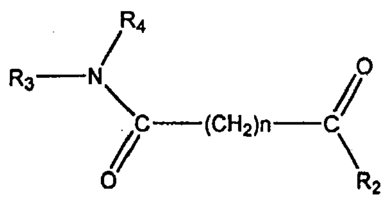

(2)

wherein each of $R_3$ and $R_4$ are independently the same as or different from each other and are a hydrogen atom, a hydroxyl group, a substituted or unsubstituted, branched or unbranched alkyl, alkenyl, cycloalkyl, arylalkyloxy, aryloxy, arylalkyloxy or pyridine group, or $R_3$ and $R_4$ are bonded together to form a piperidine group, $R_2$ is a hydroxylamino, hydroxyl, amino, alkylamino, dialkylamino or alkyloxy group and n is an integer from about 4 to about 8.

In a particular embodiment of formula 2, each of $R_3$ and $R_4$ are independently the same as or different from each other and are a hydrogen atom, a hydroxyl group, a substituted or unsubstituted, branched or unbranched alkyl, alkenyl, cycloalkyl, aryl, alkyloxy, aryloxy, arylalkyloxy, or pyridine group, or $R_3$ and $R_4$ bond together to form a piperidine group; $R_2$ is a hydroxylamino, hydroxyl, amino, alkylamino, or alkyloxy group; n is an integer from 5 to 7; and $R_3$-N-$R_4$ and $R_2$ are different.

In another particular embodiment of formula 2, n is 6. In yet another embodiment of formula 2, $R_4$ is a hydrogen atom, $R_3$ is a substituted or unsubstituted phenyl and n is 6. In yet another embodiment of formula 2, $R_4$ is a hydrogen atom, $R_3$ is a substituted phenyl and n is 6, wherein the phenyl substituent is selected from the group consisting of a methyl, cyano, nitro, trifluoromethyl, amino, aminocarbonyl, methylcyano, chloro, fluoro, bromo, iodo, 2,3-difluoro, 2,4-difluoro, 2,5-difluoro, 3,4-difluoro, 3,5-difluoro, 2,6-difluoro, 1,2,3-trifluoro, 2,3,6-trifluoro, 2,4,6-trifluoro, 3,4,5-trifluoro, 2,3,5,6-tetrafluoro, 2,3,4,5,6-pentafluoro, azido, hexyl, t-butyl, phenyl, carboxyl, hydroxyl, methoxy, phenyloxy, benzyloxy, phenylaminooxy, phenylaminocarbonyl, methoxycarbonyl, methylaminocarbonyl, dimethylamino, dimethylamino carbonyl, or hydroxylaminocarbonyl group.

In another embodiment of formula 2, n is 6, $R_4$ is a hydrogen atom and $R_3$ is a cyclohexyl group. In another embodiment of formula 2, n is 6, $R_4$ is a hydrogen atom and $R_3$ is a methoxy group. In another embodiment of formula 2, n is 6 and $R_3$ and $R_4$ bond together to form a piperidine group. In another embodiment of formula 2, n is 6, $R_4$ is a hydrogen atom and $R_3$ is a benzyloxy group. In another embodiment of formula 2, $R_4$ is a hydrogen atom and $R_3$ is a

γ-pyridine group. In another embodiment of formula 2, $R_4$ is a hydrogen atom and $R_3$ is a β-pyridine group. In another embodiment of formula 2, $R_4$ is a hydrogen atom and $R_3$ is an α-pyridine group. In another embodiment of formula 2, n is 6, and $R_3$ and $R_4$ are both methyl groups. In another embodiment of formula 2, n is 6, $R_4$ is a methyl group and $R_3$ is a phenyl group.

In one embodiment, the HDAC inhibitor useful in the methods of the present invention is represented by the structure of formula 3, or a pharmaceutically acceptable salt or hydrate thereof, and a pharmaceutically acceptable carrier or excipient.

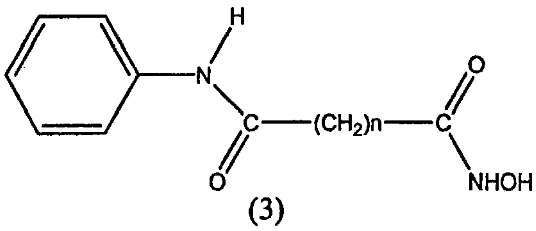

(3)

wherein n is an integer from 5 to about 8.

In a preferred embodiment of formula 3, n is 6. In accordance with this embodiment, the HDAC inhibitor is SAHA (4), or a pharmaceutically acceptable salt or hydrate thereof, and a pharmaceutically acceptable carrier or excipient. SAHA can be represented by the following structural formula:

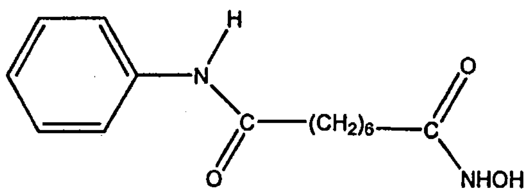

(4)

In one embodiment, the HDAC inhibitor useful in the methods of the present invention is represented by the structure of formula 5, or a pharmaceutically acceptable salt or hydrate thereof, and a pharmaceutically acceptable carrier or excipient.

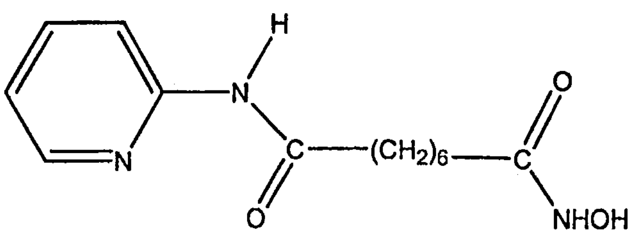

(5)

In one embodiment, the HDAC inhibitor useful in the methods of the present invention is represented by the structure of formula 6 (pyroxamide), or a pharmaceutically acceptable salt or hydrate thereof, and a pharmaceutically acceptable carrier or excipient.

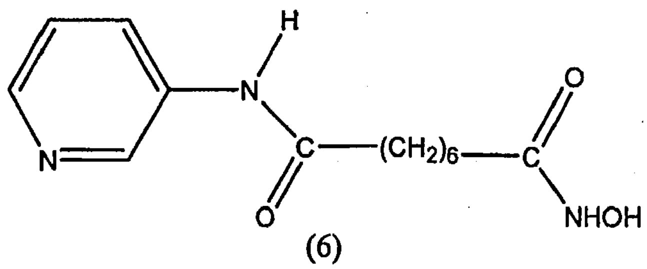

(6)

In one embodiment, the HDAC inhibitor useful in the methods of the present invention is represented by the structure of formula 7, or a pharmaceutically acceptable salt or hydrate thereof, and a pharmaceutically acceptable carrier or excipient.

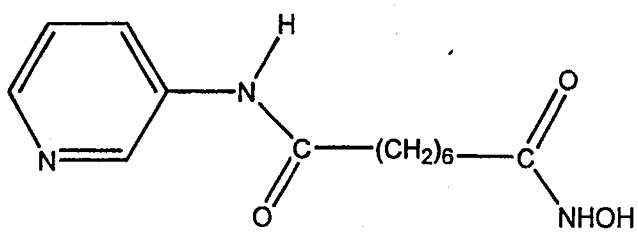

(7)

In one embodiment, the HDAC inhibitor useful in the methods of the present invention is represented by the structure of formula 8, or a pharmaceutically acceptable salt or hydrate thereof, and a pharmaceutically acceptable carrier or excipient.

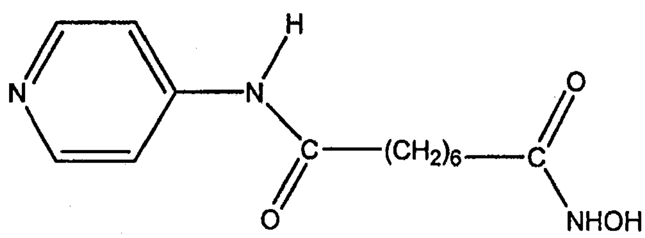

(8)

In one embodiment, the HDAC inhibitor useful in the methods of the present invention is represented by the structure of formula 9, or a pharmaceutically acceptable salt or hydrate thereof, and a pharmaceutically acceptable carrier or excipient.

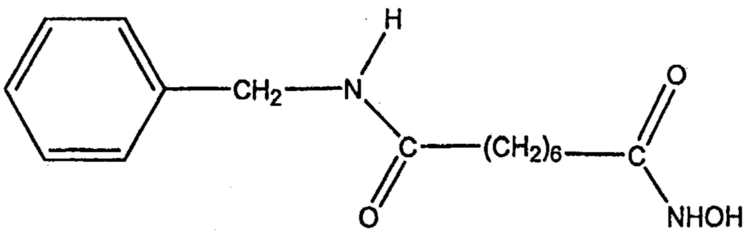

(9)

In one embodiment, the HDAC inhibitor useful in the methods of the present invention is represented by the structure of formula 10, or a pharmaceutically acceptable salt or hydrate thereof, and a pharmaceutically acceptable carrier or excipient.

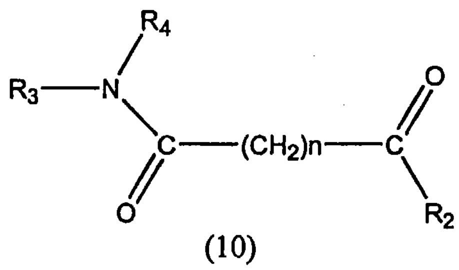

(10)

wherein $R_3$ is hydrogen and $R_4$ cycloalkyl, aryl, aryloxy, arylalkyloxy, or pyridine group, or $R_3$ and $R_4$ bond together to form a piperidine group; $R_2$ is a hydroxylamino group; and n is an integer from 5 to about 8.

In one embodiment, the HDAC inhibitor useful in the methods of the present invention is represented by the structure of formula 11, or a pharmaceutically acceptable salt or hydrate thereof, and a pharmaceutically acceptable carrier or excipient.

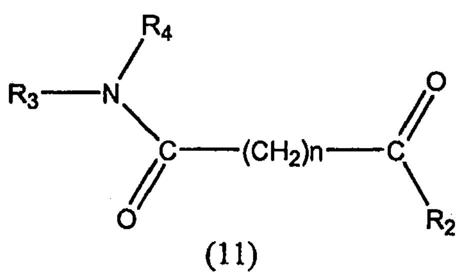

(11)

wherein $R_3$ and $R_4$ are independently a substituted or unsubstituted, branched or unbranched alkyl, alkenyl, cycloalkyl, aryl, alkyloxy, aryloxy, arylalkyloxy, or pyridine group, cycloalkyl, aryl, aryloxy, arylalkyloxy, or pyridine group, or $R_3$ and $R_4$ bond together to form a piperidine group; $R_2$ is a hydroxylamino group; and n is an integer from 5 to about 8.

In one embodiment, the HDAC inhibitor useful in the methods of the present invention is represented by the structure of formula 12, or a pharmaceutically acceptable salt or hydrate thereof, and a pharmaceutically acceptable carrier or excipient.

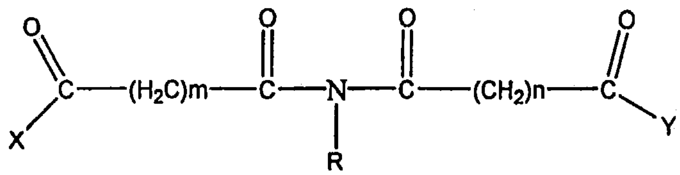

(12)

wherein each of X and Y are independently the same as or different from each other and are a hydroxyl, amino or hydroxylamino group, a substituted or unsubstituted alkyloxy, alkylamino, dialkylamino, arylamino, alkylarylamino, alkyloxyamino, aryloxyamino, alkyloxyalkylamino, or aryloxyalkylamino group; R is a hydrogen atom, a hydroxyl, group, a substituted or unsubstituted alkyl, arylalkyloxy, or aryloxy group; and each of m and n are independently the same as or different from each other and are each an integer from about 0 to about 8.

In a particular embodiment, the HDAC inhibitor is a compound of formula 12 wherein X, Y and R are each hydroxyl and both m and n are 5.

In one embodiment, the HDAC inhibitor useful in the methods of the present invention is represented by the structure of formula 13, or a pharmaceutically acceptable salt or hydrate thereof, and a pharmaceutically acceptable carrier or excipient.

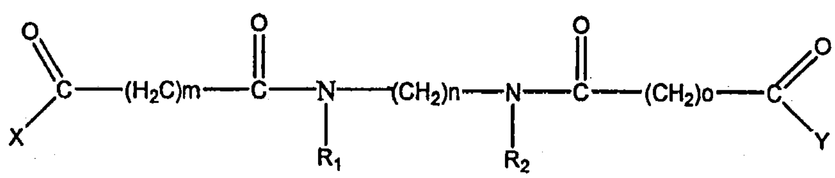

(13)

wherein each of X and Y are independently the same as or different from each other and are a hydroxyl, amino or hydroxylamino group, a substituted or unsubstituted alkyloxy, alkylamino, dialkylamino, arylamino, alkylarylamino, alkyloxyamino, aryloxyamino, alkyloxyalkylamino or aryloxyalkylamino group; each of $R_1$ and $R_2$ are independently the same as or different from each other and are a hydrogen atom, a hydroxyl group, a substituted or unsubstituted alkyl, aryl, alkyloxy, or aryloxy group; and each of m, n and o are independently the same as or different from each other and are each an integer from about 0 to about 8.

In one particular embodiment of formula 13, each of X and Y is a hydroxyl group and each of $R_1$ and $R_2$ is a methyl group. In another particular embodiment of formula 13, each of X and Y is a hydroxyl group, each of $R_1$ and $R_2$ is a methyl group, each of n and o is 6, and m is 2.

In one embodiment, the HDAC inhibitor useful in the methods of the present invention is represented by the structure of formula 14, or a pharmaceutically acceptable salt or hydrate thereof, and a pharmaceutically acceptable carrier or excipient.

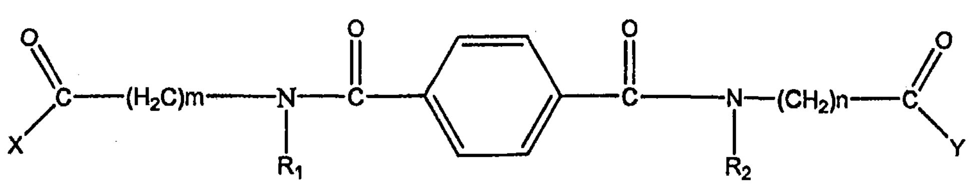

(14)

wherein each of X and Y are independently the same as or different from each other and are a hydroxyl, amino or hydroxylamino group, a substituted or unsubstituted alkyloxy, alkylamino, dialkylamino, arylamino, alkylarylamino, alkyloxyamino, aryloxyamino, alkyloxyalkylamino or aryloxyalkylamino group; each of $R_1$ and $R_2$ are independently the same as or different from each other and are a hydrogen atom, a hydroxyl group, a substituted or unsubstituted alkyl, aryl, alkyloxy, or aryloxy group; and each of m and n are independently the same as or different from each other and are each an integer from about 0 to about 8.

In one embodiment, the HDAC inhibitor useful in the methods of the present invention is represented by the structure of formula 15, or a pharmaceutically acceptable salt or hydrate thereof, and a pharmaceutically acceptable carrier or excipient.

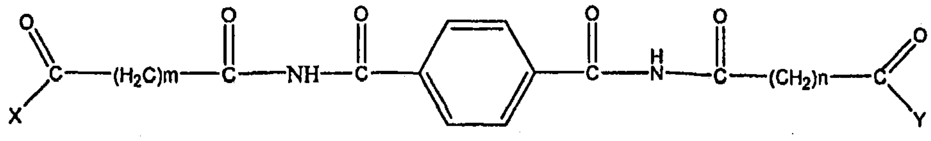

(15)

wherein each of X and Y are independently the same as or different from each other and are a hydroxyl, amino or hydroxylamino group, a substituted or unsubstituted alkyloxy, alkylamino, dialkylamino, arylamino, alkylarylamino, alkyloxyamino, aryloxyamino, alkyloxyalkylamino or aryloxyalkylamino group; and each of m and n are independently the same as or different from each other and are each an integer from about 0 to about 8.

In one particular embodiment of formula 15, each of X and Y is a hydroxyl group and each of m and n is 5.

In one embodiment, the HDAC inhibitor useful in the methods of the present invention is represented by the structure of formula 16, or a pharmaceutically acceptable salt or hydrate thereof, and a pharmaceutically acceptable carrier or excipient.

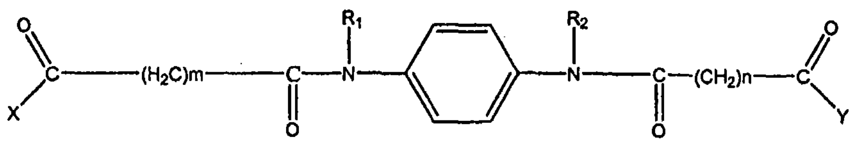

(16)

wherein each of X and Y are independently the same as or different from each other and are a hydroxyl, amino or hydroxylamino group, a substituted or unsubstituted alkyloxy, alkylamino, dialkylamino, arylamino, alkylarylamino, alkyloxyamino, aryloxyamino, alkyloxyalkylamino or aryloxyalkylamino group; $R_1$ and $R_2$ are independently the same as or different from each other and are a hydrogen atom, a hydroxyl group, a substituted or unsubstituted alkyl, arylalkyloxy or aryloxy group; and each of m and n are independently the same as or different from each other and are each an integer from about 0 to about 8.

In one embodiment, the HDAC inhibitor useful in the methods of the present invention is represented by the structure of formula 17, or a pharmaceutically acceptable salt or hydrate thereof, and a pharmaceutically acceptable carrier or excipient.

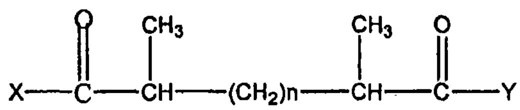

(17)

wherein each of X an Y are independently the same as or different from each other and are a hydroxyl, amino or hydroxylamino group, a substituted or unsubstituted alkyloxy, alkylamino, dialkylamino, arylamino, alkylarylamino, or aryloxyalkylamino group; and n is an integer from about 0 to about 8.

In one particular embodiment of formula 17, each of X and Y is a hydroxylamino group; $R_1$ is a methyl group, $R_2$ is a hydrogen atom; and each of m and n is 2. In another particular embodiment of formula 17, each of X and Y is a hydroxylamino group; $R_1$ is a carbonylhydroxylamino group, $R_2$ is a hydrogen atom; and each of m and n is 5. In another particular embodiment of formula 17, each of X and Y is a hydroxylamino group; each of $R_1$ and $R_2$ is a fluoro group; and each of m and n is 2.

In one embodiment, the HDAC inhibitor useful in the methods of the present invention is represented by the structure of formula 18, or a pharmaceutically acceptable salt or hydrate thereof, and a pharmaceutically acceptable carrier or excipient.

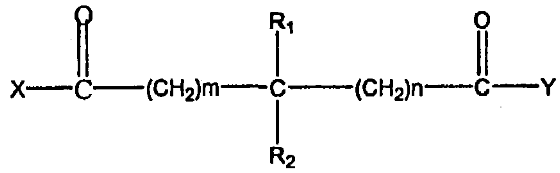

(18)

wherein each of X and Y are independently the same as or different from each other and are a hydroxyl, amino or hydroxylamino group, a substituted or unsubstituted alkyloxy, alkylamino, dialkylamino, arylamino, alkylarylamino, alkyloxyamino, aryloxyamino, alkyloxyalkyamino or aryloxyalkylamino group; each of $R_1$ and $R_2$ are independently the same as or different from each other and are a hydrogen atom, a hydroxyl group, a substituted or unsubstituted alkyl, aryl, alkyloxy, aryloxy, carbonylhydroxylamino or fluoro group; and each of m and n are independently the same as or different from each other and are each an integer from about 0 to about 8.

In one embodiment, the HDAC inhibitor useful in the methods of the present invention is represented by the structure of formula 19, or a pharmaceutically acceptable salt or hydrate thereof, and a pharmaceutically acceptable carrier or excipient.

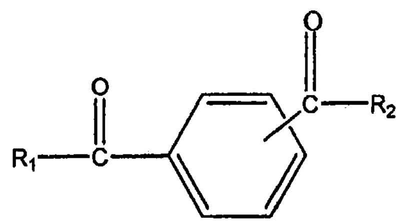

(19)

wherein each of $R_1$ and $R_2$ are independently the same as or different from each other and are a hydroxyl, alkyloxy, amino, hydroxylamino, alkylamino, dialkylamino, arylamino, alkylarylamino, alkyloxyamino, aryloxyamino, alkyloxyalkylamino, or aryloxyalkylamino group. In a particular embodiment, the HDAC inhibitor is a compound of structural formula 19 wherein $R_1$ and $R_2$ are both hydroxylamino.

In one embodiment, the HDAC inhibitor useful in the methods of the present invention is represented by the structure of formula 20, or a pharmaceutically acceptable salt or hydrate thereof, and a pharmaceutically acceptable carrier or excipient.

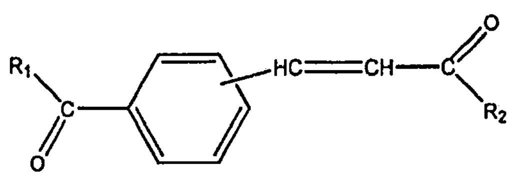

(20)

wherein each of $R_1$ and $R_2$ are independently the same as or different from each other and are a hydroxyl, alkyloxy, amino, hydroxylamino, alkylamino, dialkylamino, arylamino, alkylarylamino, alkyloxyamino, aryloxyamino, alkyloxyalkylamino, or aryloxyalkylamino group. In a particular embodiment, the HDAC inhibitor is a compound of structural formula 20 wherein $R_1$ and $R_2$ are both hydroxylamino.

In one embodiment, the HDAC inhibitor useful in the methods of the present invention is represented by the structure of formula 21, or a pharmaceutically acceptable salt or hydrate thereof, and a pharmaceutically acceptable carrier or excipient.

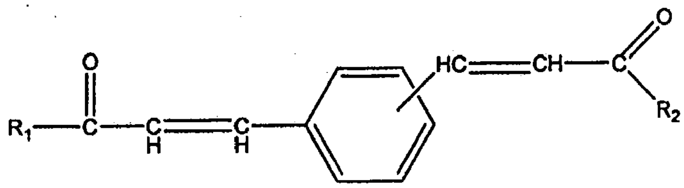

(21)

wherein each of $R_1$ and $R_2$ are independently the same as or different from each other and are a hydroxyl, alkyloxy, amino, hydroxylamino, alkylamino, dialkylamino, arylamino, alkylarylamino, alkyloxyamino, aryloxyamino, alkyloxyalkylamino, or aryloxyalkylamino group.

In a particular embodiment, the HDAC inhibitor is a compound of structural formula 21 wherein $R_1$ and $R_2$ are both hydroxylamino

In one embodiment, the HDAC inhibitor useful in the methods of the present invention is represented by the structure of formula 22, or a pharmaceutically acceptable salt or hydrate thereof, and a pharmaceutically acceptable carrier or excipient.

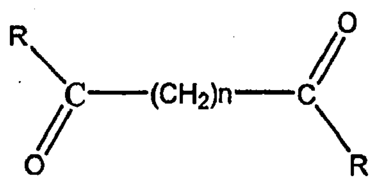

(22)

wherein R is a phenylamino group substituted with a cyano, methylcyano, nitro, carboxyl, aminocarbonyl, methylaminocarbonyl, dimethylaminocarbonyl, trifluoromethyl, hydroxylaminocarbonyl, N-hydroxylaminocarbonyl, methoxycarbonyl, chloro, fluoro, methyl, methoxy, 2,3-difluoro, 2,4-difluoro, 2,5-difluoro, 2,6-difuloro, 3,5-difluoro, 2,3,6-trifluoro, 2,4,6-trifluoro, 1,2,3-trifluoro, 3,4,5-trifluoro, 2,3,4,5-tetrafluoro, or 2,3,4,5,6-pentafluoro group; and n is an integer from 4 to 8.

In one embodiment, the HDAC inhibitor useful in the methods of the present invention is represented by the structure of formula 23 (m-carboxycinnamic acid bishydroxamide - CBHA), or a pharmaceutically acceptable salt or hydrate thereof, and a pharmaceutically acceptable carrier or excipient.

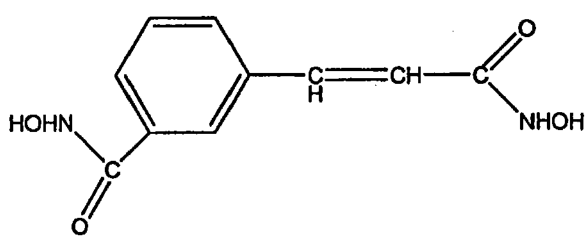

(23)

In one embodiment, the HDAC inhibitor useful in the methods of the present invention is represented by the structure of formula 24, or a pharmaceutically acceptable salt or hydrate thereof, and a pharmaceutically acceptable carrier or excipient.

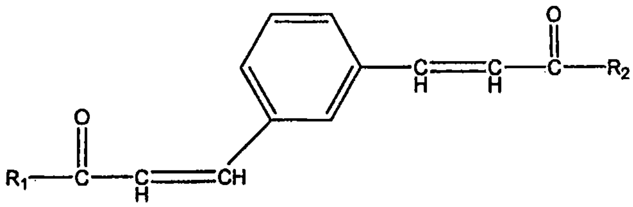

(24)

In one embodiment, the HDAC inhibitor useful in the methods of the present invention is represented by the structure of formula 25, or a pharmaceutically acceptable salt or hydrate thereof, and a pharmaceutically acceptable carrier or excipient.

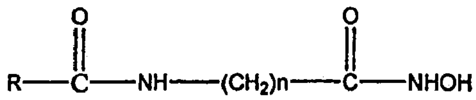

(25)

wherein R is a substituted or unsubstituted phenyl, piperidine, thiazole, 2-pyridine, 3-pyridine or 4-pyridine and n is an integer from about 4 to about 8.

In one particular embodiment of formula 25, R is a substituted phenyl group. In another particular embodiment of formula 25, R is a substituted phenyl group, where the substituent is selected from the group consisting of methyl, cyano, nitro, thio, trifluoromethyl, amino, aminocarbonyl, methylcyano, chloro, fluoro, bromo, iodo, 2,3-difluoro, 2,4-difluoro, 2,5-difluoro, 3,4-difluoro, 3,5-difluoro, 2,6-difluoro, 1,2,3-trifluoro, 2,3,6-trifluoro, 2,4,6-trifluoro, 3,4,5-trifluoro, 2,3,5,6-tetrafluoro, 2,3,4,5,6-pentafluoro, azido, hexyl, t-butyl, phenyl, carboxyl, hydroxyl, methyloxy, phenyloxy, benzyloxy, phenylaminooxy, phenylaminocarbonyl, methyloxycarbonyl, methylaminocarbonyl, dimethylamino, dimethylaminocarbonyl, or hydroxylaminocarbonyl group.

In another particular embodiment of formula 25, R is a substituted or unsubstituted 2-pyridine, 3-pyridine or 4-pyridine and n is an integer from about 4 to about 8.

In one embodiment, the HDAC inhibitor useful in the methods of the present invention is represented by the structure of formula 26, or a pharmaceutically acceptable salt or hydrate thereof, and a pharmaceutically acceptable carrier or excipient.

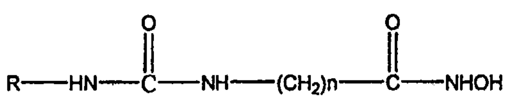

(26)

wherein R is a substituted or unsubstituted phenyl, pyridine, piperidine or thiazole group and n is an integer from about 4 to about 8 or a pharmaceutically acceptable salt thereof.

In a particular embodiment of formula 26, R is a substituted phenyl group. In another particular embodiment of formula 26, R is a substituted phenyl group, where the substituent is selected from the group consisting of methyl, cyano, nitro, thio, trifluoromethyl, amino, aminocarbonyl, methylcyano, chloro, fluoro, bromo, iodo, 2,3-difluoro, 2,4-difluoro, 2,5-difluoro, 3,4-difluoro, 3,5-difluoro, 2,6-difluoro, 1,2,3-trifluoro, 2,3,6-trifluoro, 2,4,6-trifluoro, 3,4,5-trifluoro, 2,3,5,6-tetrafluoro, 2,3,4,5,6-pentafluoro, azido, hexyl, t-butyl, phenyl, carboxyl, hydroxyl, methyloxy, phenyloxy, benzyloxy, phenylaminooxy, phenylaminocarbonyl, methyloxycarbonyl, methylaminocarbonyl, dimethylamino, dimethylaminocarbonyl, or hydroxylaminocarbonyl group.

In another particular embodiment of formula 26, R is phenyl and n is 5. In another embodiment, n is 5 and R is 3-chlorophenyl.

In one embodiment, the HDAC inhibitor useful in the methods of the present invention is represented by the

structure of formula 27, or a pharmaceutically acceptable salt or hydrate thereof, and a pharmaceutically acceptable carrier or excipient.

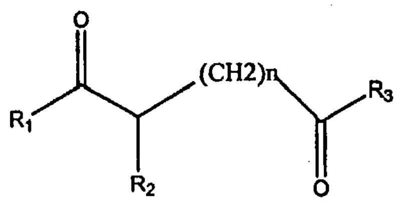

(27)

wherein each of $R_1$ and $R_2$ is directly attached or through a linker and is substituted or unsubstituted, aryl (e.g., phenyl), arylalkyl (e.g., benzyl), naphthyl, cycloalkyl, cycloalkylamino, pyridineamino, piperidino, 9-purine-6-amino, thiazoleamino, hydroxyl, branched or unbranched alkyl, alkenyl, alkyloxy, aryloxy, arylalkyloxy, pyridyl, or quinolinyl or isoquinolinyl; n is an integer from about 3 to about 10 and $R_3$ is a hydroxamic acid, hydroxylamino, hydroxyl, amino, alkylamino or alkyloxy group. The linker can be an amide moiety, e.g., O-, -S-, -NH-, $NR_5$, $-CH_2-$, $-(CH_2)_m-$, -(CH=CH)-, phenylene, cycloalkylene, or any combination thereof, wherein $R_5$ is a substitute or unsubstituted $C_1$-$C_5$ alkyl.

In certain embodiments of formula 27, $R_1$ is $-NH-R_4$ wherein $R_4$ is substituted or unsubstituted, aryl (e.g., phenyl), arylalkyl (e.g., benzyl), naphthyl, cycloalkyl, cycloalkylamino, pyridineamino, piperidino, 9-purine-6-amino, thiazoleamino, hydroxyl, branched or unbranched alkyl, alkenyl, alkyloxy, aryloxy, arylalkyloxy, pyridyl, quinolinyl or isoquinolinyl

In one embodiment, the HDAC inhibitor useful in the methods of the present invention is represented by the structure of formula 28, or a pharmaceutically acceptable salt or hydrate thereof, and a pharmaceutically acceptable carrier or excipient.

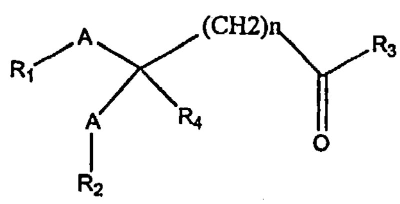

(28)

wherein each of $R_1$ and $R_2$ is, substituted or unsubstituted, aryl (e.g., phenyl), arylalkyl (e.g., benzyl), naphthyl, cycloalkyl, cycloalkylamino, pyridineamino, piperidino, 9-purine-6-amino, thiazoleamino, hydroxyl, branched or unbranched alkyl, alkenyl, alkyloxy, aryloxy, arylalkyloxy, pyridyl, quinolinyl or isoquinolinyl; $R_3$ is hydroxamic acid, hydroxylamino, hydroxyl, amino, alkylamino or alkyloxy group; $R_4$ is hydrogen, halogen, phenyl or a cycloalkyl moiety; and A can be the same or different and represents an amide moiety, O-, -S-, -NH-, $NR_5$, $-CH_2-$, $-(CH_2)_m-$, -(CH=CH)-, phenylene, cycloalkylene, or any combination thereof wherein $R_5$ is a substitute or unsubstituted $C_1$-$C_5$ alkyl; and n and m are each an integer from 3 to 10.

In further particular embodiment compounds having a more specific structure within the scope of compounds 27 or 28 are:

In one embodiment, the HDAC inhibitor useful in the methods of the present invention is represented by the structure of formula 29:

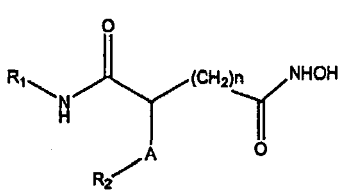

(29)

wherein A is an amide moiety, $R_1$ and $R_2$ are each selected from substituted or unsubstituted aryl (e.g., phenyl), arylalkyl (e.g., benzyl), naphthyl, pyridineamino, 9-purine-6-amino, thiazoleamino, aryloxy, arylalkyloxy, pyridyl, quinolinyl or isoquinolinyl; and n is an integer from 3 to 10.

For example, the compound of formula 29 can have the structure 30 or 31:

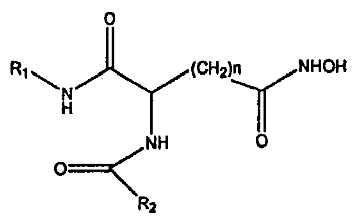
(30)

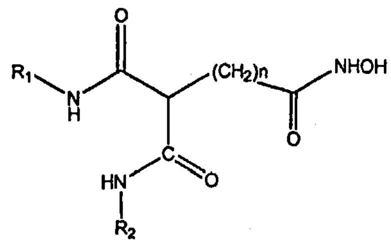
(31)

wherein $R_1$, $R_2$ and n have the meanings of formula 29.

In one embodiment, the HDAC inhibitor useful in the methods of the present invention is represented by the structure of formula 32:

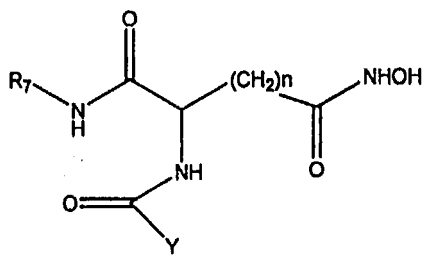
(32)

wherein $R_7$ is selected from substituted or unsubstituted aryl (e.g., phenyl), arylalkyl (e.g., benzyl), naphthyl, pyridineamino, 9-purine-6-amino, thiazoleamino, aryloxy, arylalkyloxy, pyridyl, quinolinyl or isoquinolinyl; n is an integer from 3 to 10 and Y is selected from:

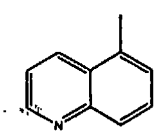
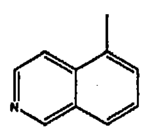
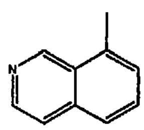
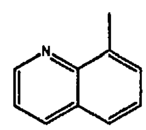

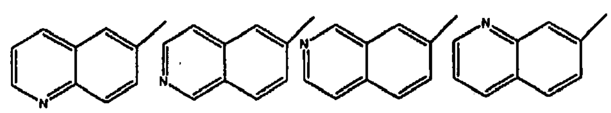

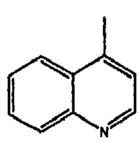
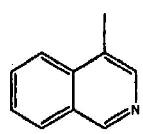
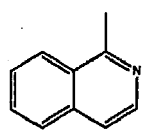
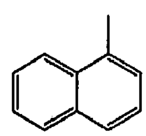

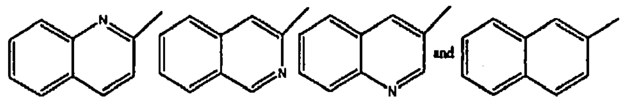
and

In one embodiment, the HDAC inhibitor useful in the methods of the present invention is represented by the structure of formula 33:

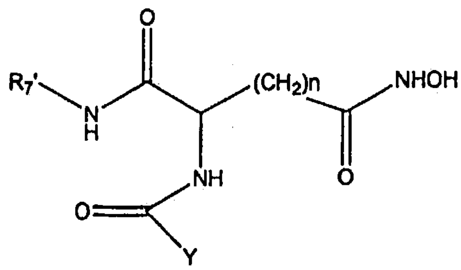

(33)

wherein n is an integer from 3 to 10, Y is selected from

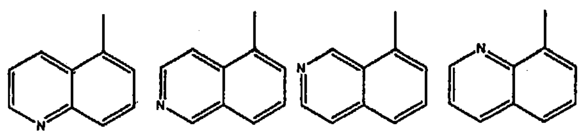

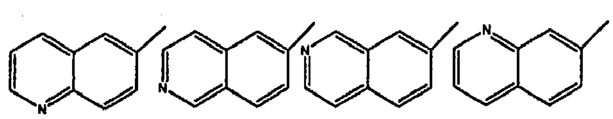

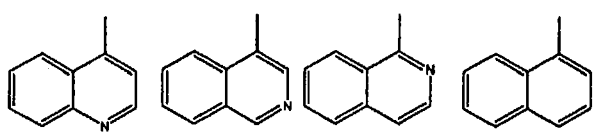

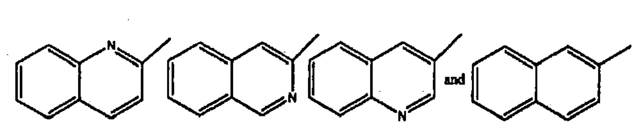
and

and $R_7$' is selected from

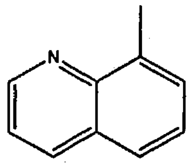
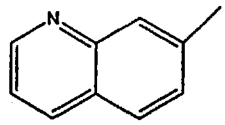
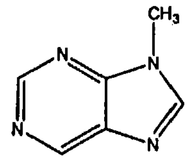
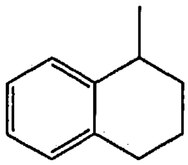
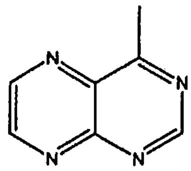

In one embodiment, the HDAC inhibitor useful in the methods of the present invention is represented by the structure of formula 34:

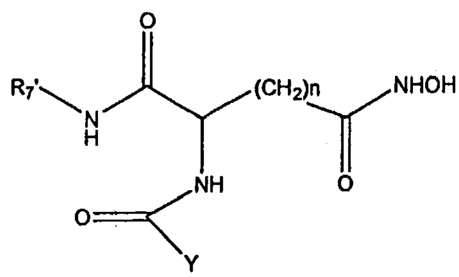

(34)

aryl (e.g., phenyl), arylalkyl (e.g., benzyl), naphthyl, pyridineamino, 9-purine-6-amino, thiazoleamino, aryloxy, arylalkyloxy, pyridyl, quinolinyl or isoquinolinyl; n is an integer from 3 to 10 and $R_7$' is selected from

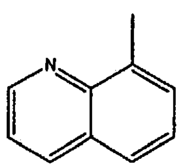
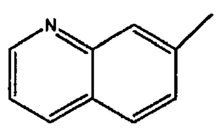
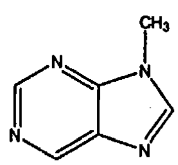
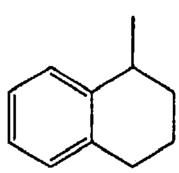
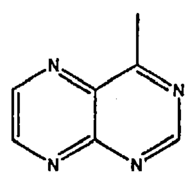

In one embodiment, the HDAC inhibitor useful in the methods of the present invention is represented by the structure of formula 35:

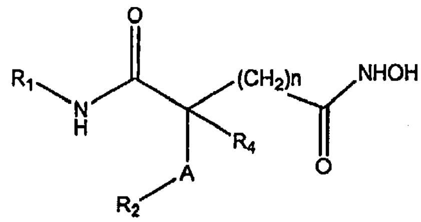

(35)

wherein A is an amide moiety, $R_1$ and $R_2$ are each selected from substituted or unsubstituted aryl (e.g., phenyl), arylalkyl (e.g., benzyl), naphthyl, pyridineamino, 9-purine-6-amino, thiazoleamino, aryloxy, arylalkyloxy, pyridyl, quinolinyl or isoquinolinyl; $R_4$ is hydrogen, a halogen, a phenyl or a cycloalkyl moiety and n is an integer from 3 to 10.

For example, the compound of formula 35 can have the structure 36 or 37:

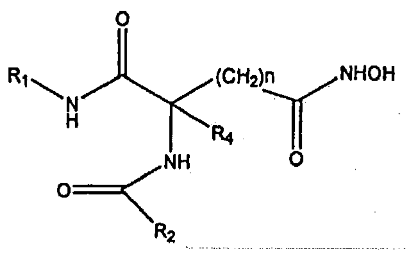

(36)

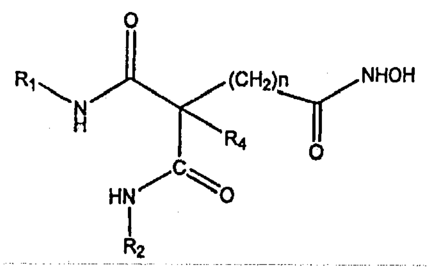

(37)

wherein $R_1$, $R_2$, $R_4$ and n have the meanings of formula 35.

In one embodiment, the HDAC inhibitor useful in the methods of the present invention is represented by the structure of formula 38:

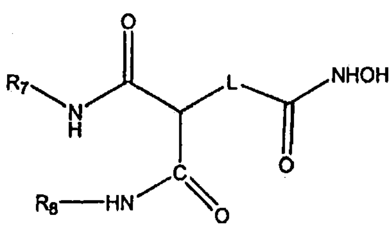

(38)

wherein L is a linker selected from the group consisting of an amide moiety, O-, -S-, -NH-, $NR_5$, $-CH_2-$, $-(CH_2)_m-$, -(CH=CH)-, phenylene, cycloalkylene, or any combination thereof wherein $R_5$ is a substitute or unsubstituted $C_1$-$C_5$ alkyl; and wherein each of $R_7$ and $R_8$ are independently a substituted or unsubstituted aryl (e.g., phenyl), arylalkyl (e.g., benzyl), naphthyl, pyridineamino, 9-purine-6-amino, thiazoleamino, aryloxy, arylalkyloxy, pyridyl, quinolinyl or isoquinolinyl;, n is an integer from 3 to 10 and m is an integer from 0-10.

For example, a compound of formula 38 can be represented by the structure of formula (39):

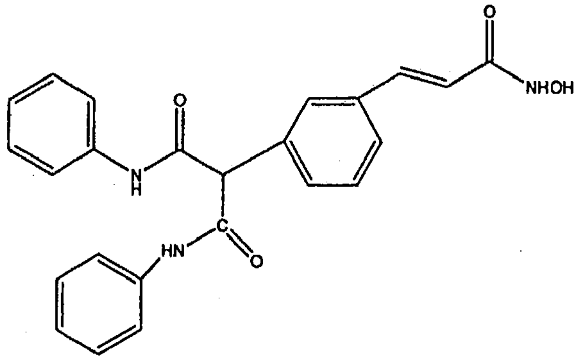

(39)

Other HDAC inhibitors suitable for use in the methods of the present invention include those shown in the following more specific formulas:

A compound represented by the structure:

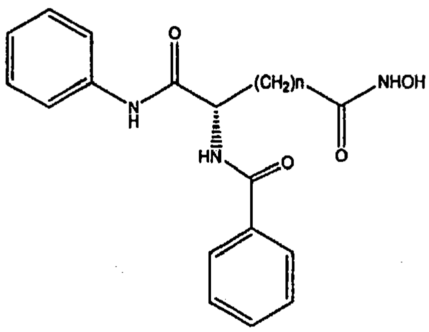

(40)

wherein n is an integer from 3 to 10, or an enantiomer thereof. In one particular embodiment of formula 40, n=5.

A compound represented by the structure:

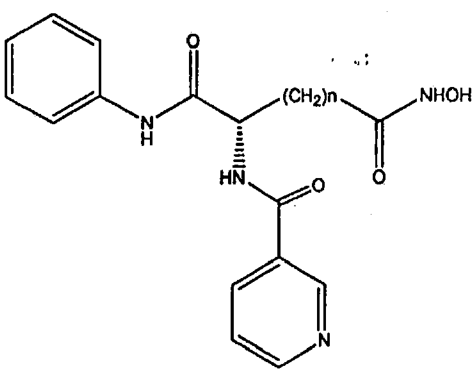

(41)

wherein n is an integer from 3 to 10, or an enantiomer thereof. In one particular embodiment of formula 41, n=5.

A compound represented by the structure:

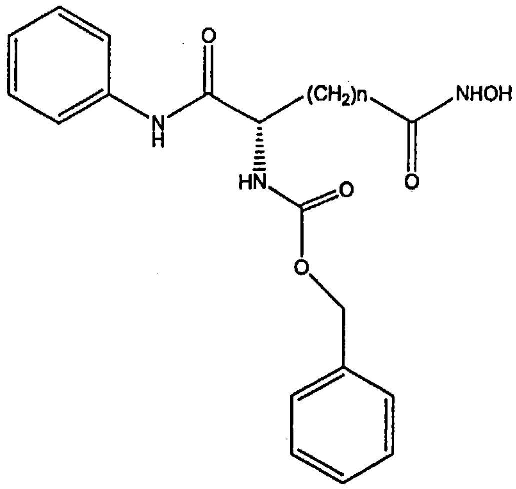

(42)

wherein n is an integer from 3 to 10 or an enantiomer thereof. In one particular embodiment of formula 42, n=5.

A compound represented by the structure:

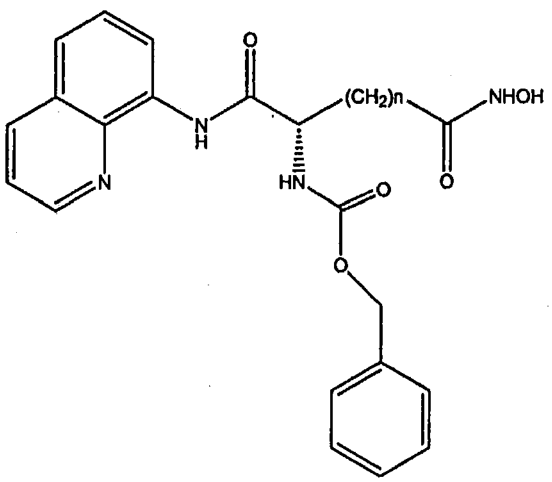

(43)

wherein n is an integer from 3 to 10, or an enantiomer thereof. In one particular embodiment of formula 43, n=5.

A compound represented by the structure:

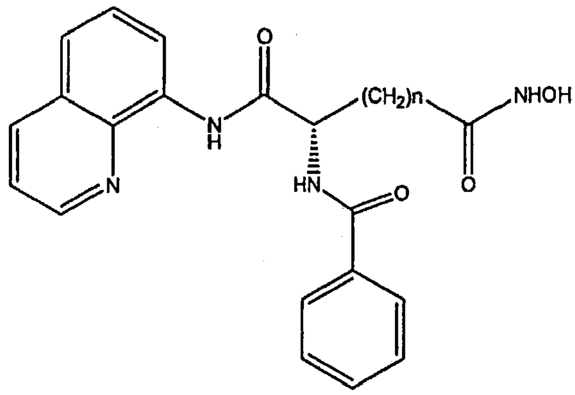

(44)

wherein n is an integer from 3 to 1,0 or an enantiomer thereof. In one particular embodiment of formula 44, n=5.

A compound represented by the structure:

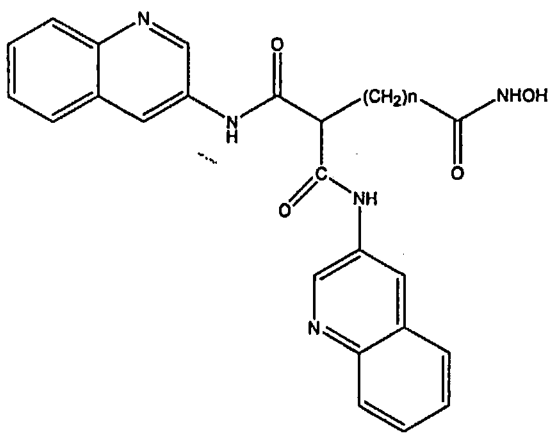

(45)

wherein n is an integer from 3 to 10, or an enantiomer thereof. In one particular embodiment of formula 45, n=5.

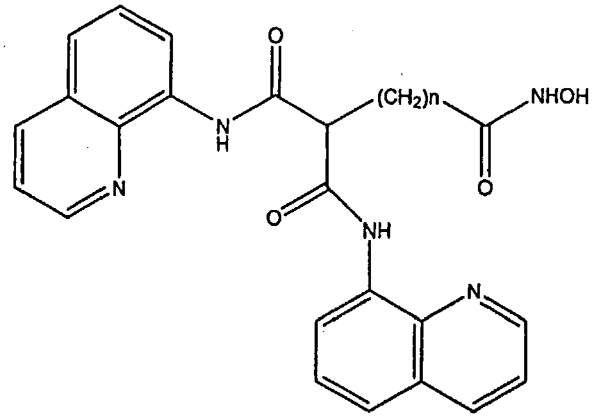

(46)

wherein n is an integer from 3 to 10 or an enantiomer thereof. In one particular embodiment of formula 46, n=5.

A compound represented by the structure:

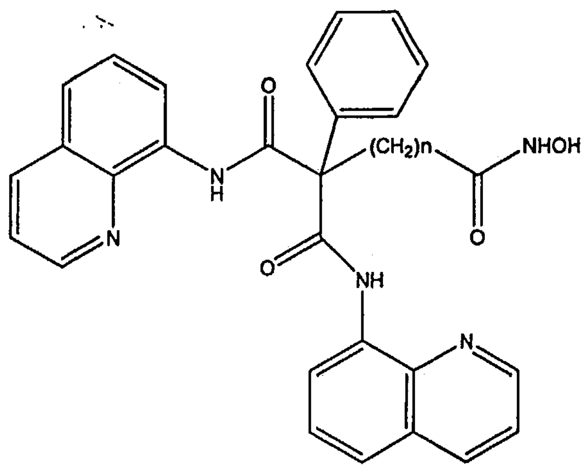

(47)

wherein n is an integer from 3 to 10, or an enantiomer thereof. In one particular embodiment of formula 47, n=5.

A compound represented by the structure:

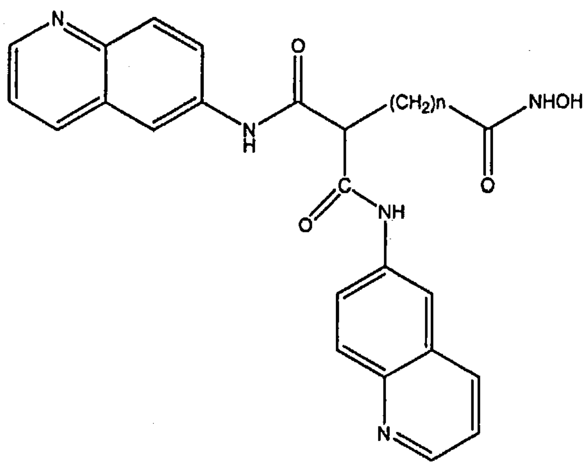

(48)

wherein n is an integer from 3 to 10, or an enantiomer thereof. In one particular embodiment of formula 48, n=5.

A compound represented by the structure:

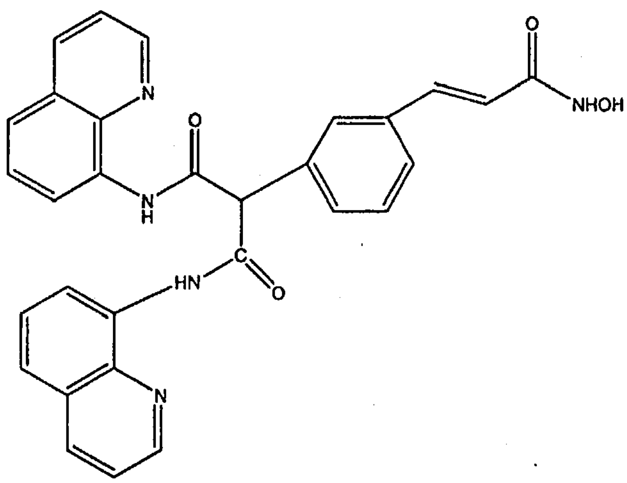

(49)

wherein n is an integer from 3 to 10, or an enantiomer thereof. In one particular

embodiment of formula 49, n=5.

A compound represented by the structure:

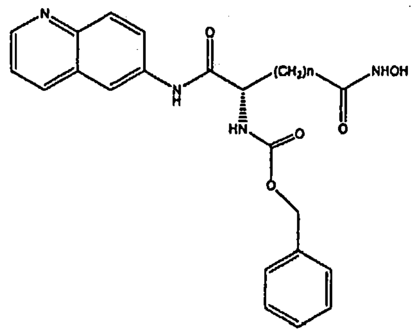

(50)

wherein n is an integer from 3 to 10, or an enantiomer thereof. In one particular embodiment of formula 50, n=5.

A compound represented by the structure:

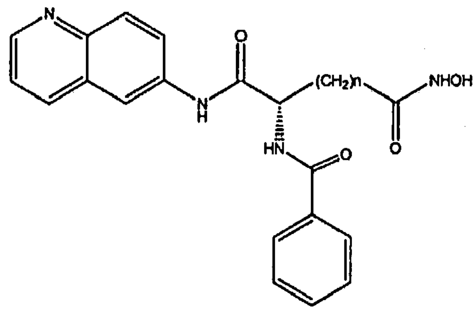

(51)

wherein n is an integer from 3 to 10, or an enantiomer thereof. In one particular embodiment of formula 51, n=5.

Other examples of such compounds and other HDAC inhibitors can be found in U.S. Patent No. 5,369,108, issued on November 29, 1994, U.S. Patent No. 5,700,811, issued on December 23, 1997, U.S. Patent No. 5,773,474, issued on June 30, 1998, U.S.

Patent No. 5,932,616, issued on August 3, 1999 and U.S. Patent No. 6,511,990, issued January 28, 2003, all to Breslow *et al.*; U.S. Patent No. 5,055,608, issued on October 8, 1991, U.S. Patent No. 5,175,191, issued on December 29, 1992 and U.S. Patent No. 5,608,108, issued on March 4, 1997, all to Marks *et al.*; as well as Yoshida, M., et al., Bioassays 17, 423-430 (1995); Saito, A., et al., PNAS USA 96, 4592-4597, (1999); Furamai R. et al., PNAS USA 98 (1), 87-92 (2001); Komatsu, Y., et al., Cancer Res. 61(11), 4459-4466 (2001); Su, G.H., et al., Cancer Res. 60, 3137-3142 (2000); Lee, B.I. et al., Cancer Res. 61(3), 931-934; Suzuki, T., et al., J. Med. Chem. 42(15), 3001-3003 (1999); published PCT Application WO 01/18171 published on March 15, 2001 to Sloan-Kettering Institute for Cancer Research and The Trustees of Columbia University; published PCT Application WO02/246144 to Hofmiann-La Roche; published PCT Application WO02/22577 to Novartis; published PCT Application WO02/30879 to Prolifix; published PCT Applications WO 01/38322 (published May 31, 2001), WO 01/70675 (published on September 27, 2001) and WO 00/71703 (published on November 30, 2000) all to Methylgene, Inc.; published PCT Application WO 00/21979 published on October 8, 1999 to Fujisawa Pharmaceutical Co., Ltd.; published PCT Application WO 98/40080 published on March 11, 1998 to Beacon Laboratories, L.L.C.; and Curtin M. (Current patent status of HDAC inhibitors Expert Opin. Ther. Patents (2002) 12(9): 1375-1384 and references cited therein).

SAHA or any of the other HDACs can be synthesized according to the methods outlined in the Experimental Details Section, or according to the method set forth in U.S. Patent Nos. 5,369,108, 5,700,811, 5,932,616 and 6,511,990, the contents of which are incorporated by reference in their entirety, or according to any other method known to a person skilled in the art.

Specific non-limiting examples of HDAC inhibitors are provided in the Table below. It should be noted that the present invention encompasses any compounds which are structurally similar to the compounds represented below, and which are capable of inhibiting histone deacetylases.

| Title | |
|---|---|
| **MS-275** | 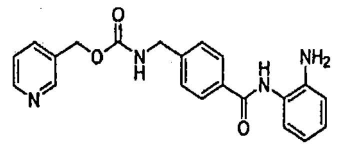 |
| **DEPSIPEPTIDE** | 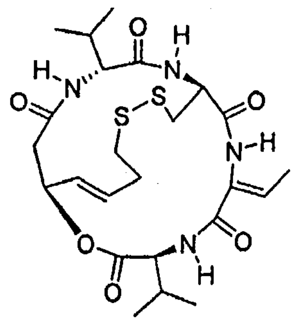 |
| **CI-994** | 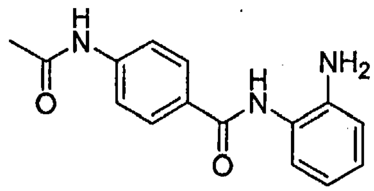 |
| **Apicidin** | 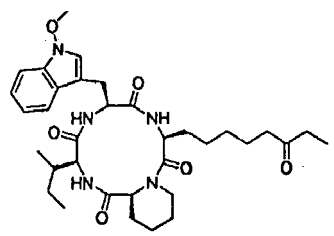 |
| **A-161906** | 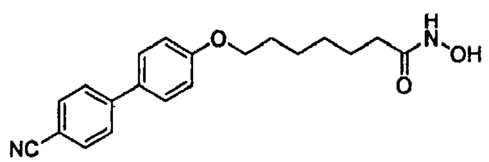 |
| **Scriptaid** | 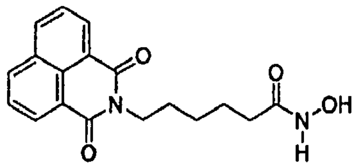 |
| **PXD-101** | 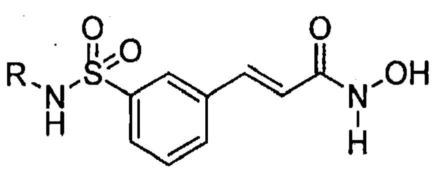 |

(continued)

| Title | |
|---|---|
| **CHAP** | 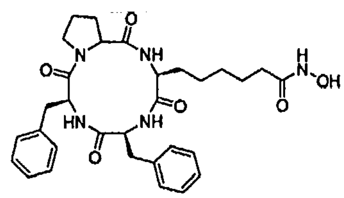 |
| **LAQ-824** | 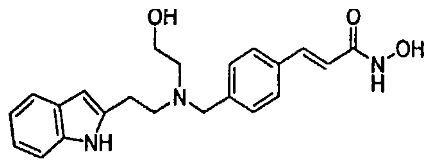 |
| **Butyric Acid** | 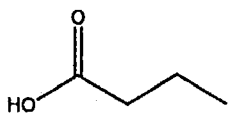 |
| **Depudecin** | 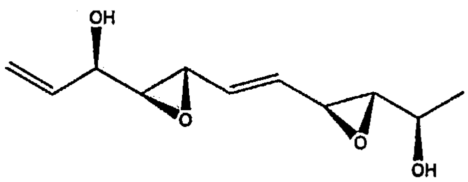 |
| **Oxamflatin** | 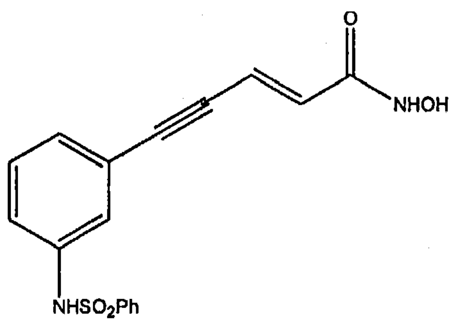 |
| ***Trichostatin C*** | 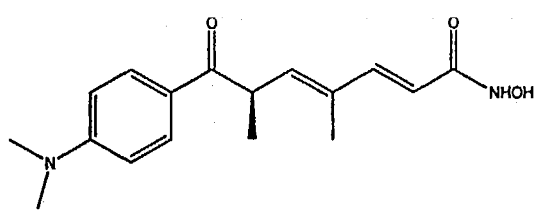 |

## Chemical Definitions

An "aliphatic group" is non-aromatic, consists solely of carbon and hydrogen and can optionally contain one or more units of unsaturation, e.g., double and/or triple bonds. An aliphatic group can be straight chained, branched or cyclic. When straight chained or branched, an aliphatic group typically contains between about 1 and about 12 carbon atoms, more typically between about 1 and about 6 carbon atoms. When cyclic, an aliphatic group typically contains between about 3 and about 10 carbon atoms, more typically between about 3 and about 7 carbon atoms. Aliphatic groups are preferably $C_1$-$C_{12}$ straight chained or branched alkyl groups (i.e., completely saturated aliphatic groups), more preferably $C_1$-$C_6$ straight chained or branched alkyl groups. Examples include methyl, ethyl, n-propyl, iso-propyl, n-butyl, sec-butyl and tert-butyl.

An "aromatic group" (also referred to as an "aryl group") as used herein includes carbocyclic aromatic groups, heterocyclic aromatic groups (also referred to as "heteroaryl") and fused polycyclic aromatic ring system as defined herein.

A "carbocyclic aromatic group" is an aromatic ring of 5 to 14 carbons atoms, and includes a carbocyclic aromatic group fused with a 5-or 6-membered cycloalkyl group such as indan. Examples of carbocyclic aromatic groups include, but are not limited to, phenyl, naphthyl, e.g., 1-naphthyl and 2-naphthyl; anthracenyl, e.g., 1-anthracenyl, 2-anthracenyl; phenanthrenyl; fluorenonyl, e.g., 9-fluorenonyl, indanyl and the like. A carbocyclic aromatic group is optionally substituted with a designated number of substituents, described below.

A "heterocyclic aromatic group" (or "heteroaryl") is a monocyclic, bicyclic or tricyclic aromatic ring of 5- to 14-ring atoms of carbon and from one to four heteroatoms selected from O, N, or S. Examples of heteroaryl include, but are not limited to pyridyl, e.g., 2-pyridyl (also referred to as "α-pyridyl), 3-pyridyl (also referred to as β-pyridyl) and 4-pyridyl (also referred to as (γ-pyridyl); thienyl, e.g., 2-thienyl and 3-thienyl; furanyl, e.g., 2-furanyl and 3-furanyl; pyrimidyl, e.g., 2-pyrimidyl and 4-pyrimidyl; imidazolyl, e.g., 2-imidazolyl; pyranyl, e.g., 2-pyranyl and 3-pyranyl; pyrazolyl, e.g., 4-pyrazolyl and 5-pyrazolyl; thiazolyl, e.g., 2-thiazolyl, 4-thiazolyl and 5-thiazolyl; thiadiazolyl; isothiazolyl; oxazolyl, e.g., 2-oxazoyl, 4-oxazoyl and 5-oxazoyl; isoxazoyl; pyrrolyl; pyridazinyl; pyrazinyl and the like. Heterocyclic aromatic (or heteroaryl) as defined above may be optionally substituted with a designated number of substituents, as described below for aromatic groups.

A "fused polycyclic aromatic" ring system is a carbocyclic aromatic group or heteroaryl fused with one or more other heteroaryl or nonaromatic heterocyclic ring. Examples include, quinolinyl and isoquinolinyl, e.g., 2-quinolinyl, 3-quinolinyl, 4-quinolinyl, 5-quinolinyl, 6-quinolinyl, 7-quinolinyl and 8-quinolinyl, 1-isoquinolinyl, 3-quinolinyl, 4-isoquinolinyl, 5-isoquinolinyl, 6-isoquinolinyl, 7-isoquinolinyl and 8-isoquinolinyl; benzofuranyl e.g., 2-benzofuranyl and 3-benzofuranyl; dibenzofuranyl.e.g., 2,3-dihydrobenzofuranyl; dibenzothiophenyl; benzothienyl, e.g., 2-benzothienyl and 3-benzothienyl; indolyl, e.g., 2-indolyl and 3-indolyl; benzothiazolyl, e.g., 2-benzothiazolyl; benzooxazolyl, e.g., 2-benzooxazolyl; benzimidazolyl, e.g., 2-benzoimidazolyl; isoindolyl, e.g., 1-isoindolyl and 3-isoindolyl; benzotriazolyl; purinyl; thianaphthenyl and the like. Fused polycyclic aromatic ring systems may optionally be substituted with a designated number of substituents, as described herein.

An "aralkyl group" (arylalkyl) is an alkyl group substituted with an aromatic group, preferably a phenyl group. A preferred aralkyl group is a benzyl group. Suitable aromatic groups are described herein and suitable alkyl groups are described herein. Suitable substituents for an aralkyl group are described herein.

An "aryloxy group" is an aryl group that is attached to a compound via an oxygen (e.g., phenoxy).

An "alkoxy group"(alkyloxy), as used herein, is a straight chain or branched $C_1$-$C_{12}$ or cyclic $C_3$-$C_{12}$ alkyl group that is connected to a compound via an oxygen atom. Examples of alkoxy groups include but are not limited to methoxy, ethoxy and propoxy.

An "arylalkoxy group" (arylalkyloxy) is an arylalkyl group that is attached to a compound via an oxygen on the alkyl portion of the arylalkyl (e.g., phenylmethoxy).

An "arylamino group" as used herein, is an aryl group that is attached to a compound via a nitrogen.

As used herein, an "arylalkylamino group" is an arylalkyl group that is attached to a compound via a nitrogen on the alkyl portion of the arylalkyl.

As used herein, many moieties or groups are referred to as being either "substituted or unsubstituted". When a moiety is referred to as substituted, it denotes that any portion of the moiety that is known to one skilled in the art as being available for substitution can be substituted. For example, the substitutable group can be a hydrogen atom which is replaced with a group other than hydrogen (i.e., a substituent group). Multiple substituent groups can be present. When multiple substituents are present, the substituents can be the same or different and substitution can be at any of the substitutable sites. Such means for substitution are well-known in the art. For purposes of exemplification, which should not be construed as limiting the scope of this invention, some examples of groups that are substituents are: alkyl groups (which can also be substituted, with one or more substituents, such as $CF_3$), alkoxy groups (which can be substituted, such as $OCF_3$), a halogen or halo group (F, Cl, Br, I), hydroxy, nitro, oxo, -CN, -COH, -COOH, amino, azido, N-alkylamino or N,N-dialkylamino (in which the alkyl groups can also be substituted), esters (-C(O)-OR, where R can be a group such as alkyl, aryl, etc., which can be substituted), aryl (most preferred is phenyl, which can be substituted), arylalkyl (which can be substituted) and aryloxy.

**Stereochemistry**

Many organic compounds exist in optically active forms having the ability to rotate the plane of plane-polarized light. In describing an optically active compound, the prefixes D and L or R and S are used to denote the absolute configuration of the molecule about its chiral center(s). The prefixes d and 1 or (+) and (-) are employed to designate the sign of rotation of plane-polarized light by the compound, with (-) or meaning that the compound is levorotatory. A compound prefixed with (+) or d is dextrorotatory. For a given chemical structure, these compounds, called stereoisomers, are identical except that they are non-superimposable mirror images of one another. A specific stereoisomer can also

be referred to as an enantiomer, and a mixture of such isomers is often called an enantiomeric mixture. A 50:50 mixture of enantiomers is referred to as a racemic mixture. Many of the compounds described herein can have one or more chiral centers and therefore can exist in different enantiomeric forms. If desired, a chiral carbon can be designated with an asterisk (*). When bonds to the chiral carbon are depicted as straight lines in the formulas of the invention, it is understood that both the (R) and (S) configurations of the chiral carbon, and hence both enantiomers and mixtures thereof, are embraced within the formula. As is used in the art, when it is desired to specify the absolute configuration about a chiral carbon, one of the bonds to the chiral carbon can be depicted as a wedge (bonds to atoms above the plane) and the other can be depicted as a series or wedge of short parallel lines is (bonds to atoms below the plane). The Cahn-Inglod-Prelog system can be used to assign the (R) or (S) configuration to a chiral carbon.

When the HDAC inhibitors of the present invention contain one chiral center, the compounds exist in two enantiomeric forms and the present invention includes both enantiomers and mixtures of enantiomers, such as the specific 50:50 mixture referred to as a racemic mixtures. The enantiomers can be resolved by methods known to those skilled in the art, for example by formation of diastereoisomeric salts which may be separated, for example, by crystallization (see, CRC Handbook of Optical Resolutions via Diastereomeric Salt Formation by David Kozma (CRC Press, 2001)); formation of diastereoisomeric derivatives or complexes which may be separated, for example, by crystallization, gas-liquid or liquid chromatography; selective reaction of one enantiomer with an enantiomer-specific reagent, for example enzymatic esterification; or gas-liquid or liquid chromatography in a chiral environment, for example on a chiral support for example silica with a bound chiral ligand or in the presence of a chiral solvent. It will be appreciated that where the desired enantiomer is converted into another chemical entity by one of the separation procedures described above, a further step is required to liberate the desired enantiomeric form. Alternatively, specific enantiomers may be synthesized by asymmetric synthesis using optically active reagents, substrates, catalysts or solvents, or by converting one enantiomer into the other by asymmetric transformation.

Designation of a specific absolute configuration at a chiral carbon of the compounds of the invention is understood to mean that the designated enantiomeric form of the compounds is in enantiomeric excess (ee) or in other words is substantially free from the other enantiomer. For example, the "R" forms of the compounds are substantially free from the "S" forms of the compounds and are, thus, in enantiomeric excess of the "S" forms. Conversely, "S" forms of the compounds are substantially free of "R" forms of the compounds and are, thus, in enantiomeric excess of the "R" forms. Enantiomeric excess, as used herein, is the presence of a particular enantiomer at greater than 50%. For example, the enantiomeric excess can be about 60% or more, such as about 70% or more, for example about 80% or more, such as about 90% or more. In a particular embodiment when a specific absolute configuration is designated, the enantiomeric excess of depicted compounds is at least about 90%. In a more particular embodiment, the enantiomeric excess of the compounds is at least about 95%, such as at least about 97.5%, for example, at least 99% enantiomeric excess.

When a compound of the present invention has two or more chiral carbons it can have more than two optical isomers and can exist in diastereoisomeric forms. For example, when there are two chiral carbons, the compound can have up to 4 optical isomers and 2 pairs of enantiomers ((S,S)/(R,R) and (R,S)/(S,R)). The pairs of enantiomers (e.g., (S,S)/(R,R)) are mirror image stereoisomers of one another. The stereoisomers which are not mirror-images (e.g., (S, S) and (R,S)) are diastereomers. The diastereoisomeric pairs may be separated by methods known to those skilled in the art, for example chromatography or crystallization and the individual enantiomers within each pair may be separated as described above. The present invention includes each diastereoisomer of such compounds and mixtures thereof.

As used herein, "a," an" and "the" include singular and plural referents unless the context clearly dictates otherwise. Thus, for example, reference to "an active agent" or "a pharmacologically active agent" includes a single active agent as well a two or more different active agents in combination, reference to "a carrier" includes mixtures of two or more carriers as well as a single carrier, and the like.

This invention is also intended to encompass pro-drugs of the HDAC inhibitors disclosed herein. A prodrug of any of the compounds can be made using well known pharmacological techniques.

This invention, in addition to the above listed compounds, is intended to encompass the use of homologs and analogs of such compounds. In this context, homologs are molecules having substantial structural similarities to the above-described compounds and analogs are molecules having substantial biological similarities regardless of structural similarities.

**Alkylating Agents**

Alkylating agents react with nucleophilic residues, such as the chemical entities on the nucleotide precursors for DNA production. They affect the process of cell division by alkylating these nucleotides and preventing their assembly into DNA.

Examples of alkylating agents include, but are not limited to, bischloroethylamines (nitrogen mustards, e.g., chlorambucil, cyclophosphamide, ifosfamide, mechlorethamine, melphalan, uracil mustard), aziridines (e.g., thiotepa), alkyl alkone sulfonates (e.g., busulfan), nitrosoureas (e.g., carmustine, lomustine, streptozocin), nonclassic alkylating

agents (altretamine, dacarbazine, and procarbazine), platinum compounds (carboplastin and cisplatin). These compounds react with phosphate, amino, hydroxyl, sulfihydryl, carboxyl, and imidazole groups.

Under physiological conditions, these drugs ionize and produce positively charged ion that attach to susceptible nucleic acids and proteins, leading to cell cycle arrest and/or cell death. The alkylating agents are cell cycle phasenonspecific agents because they exert their activity independently of the specific phase of the cell cycle. The nitrogen mustards and alkyl alkone sulfonates are most effective against cells in the G1 or M phase. Nitrosoureas, nitrogen mustards, and aziridines impair progression from the G1 and S phases to the M phases. Chabner and Collins eds. (1990) "Cancer Chemotherapy: Principles and Practice", Philadelphia: JB Lippincott.

The alkylating agents are active against wide variety of neoplastic diseases, with significant activity in the treatment of leukemias and lymphomas as well as solid tumors. Clinically this group of drugs is routinely used in the treatment of acute and chronic leukemias; Hodgkin's disease; non-Hodgkin's lymphoma; multiple myeloma; primary brain tumors; carcinomas of the breast, ovaries, testes, lungs, bladder, cervix, head and neck, and malignant melanoma.

The major toxicity common to all of the alkylating agents is myelosuppression. Additionally, Gastrointestinal adverse effects of variable severity occur commonly and various organ toxicities are associated with specific compounds. Black and Livingston (1990) Drugs 39: 489-501 ; and 39: 652-673.

## Antibiotics

Antibiotics (e.g., cytotoxic antibiotics) act by directly inhibiting DNA or RNA synthesis and are effective throughout the cell cycle. Examples of antibiotic agents include anthracyclines (e.g., doxorubicin, daunorubicin, epirubicin, idarubicin and anthracenedione), mitomycin C, bleomycin, dactinomycin, plicatomycin. These antibiotic agents interferes with cell growth by targeting different cellular components. For example, anthracyclines are generally believed to interfere with the action of DNA topoisomerase II in the regions of transcriptionally active DNA, which leads to DNA strand scissions.

Bleomycin is generally believed to chelate iron and forms an activated complex, which then binds to bases of DNA, causing strand scissions and cell death.

The antibiotic agents have been used as therapeutics across a range of neoplastic diseases, including carcinomas of the breast, lung, stomach and thyroids, lymphomas, myelogenous leukemias, myelomas, and sarcomas. The primary toxicity of the anthracyclines within this group is myelosuppression, especially granulocytopenia. Mucositis often accompanies the granulocytopenia and the severity correlates with the degree of myelosuppression. There is also significant cardiac toxicity associated with high dosage administration of the anthracyclines.

## Antimetabolic Agents

Antimetabolic agents (i.e., antimetabolites) are a group of drugs that interfere with metabolic processes vital to the physiology and proliferation of cancer cells. Actively proliferating cancer cells require continuous synthesis of large quantities of nucleic acids, proteins, lipids, and other vital cellular constituents.

Many of the antimetabolites inhibit the synthesis of purine or pyrimidine nucleosides or inhibit the enzymes of DNA replication. Some antimetabolites also interfere with the synthesis of ribonucleosides and RNA and/or amino acid metabolism and protein synthesis as well. By interfering with the synthesis of vital cellular constituents, antimetabolites can delay or arrest the growth of cancer cells. Examples of antimetabolic agents include, but are not limited to, fluorouracil (5-FU), floxuridine (5-FUdR), methotrexate, leucovorin, hydroxyurea, thioguanine (6-TG), mercaptopurine (6-MP), cytarabine, pentostatin, fludarabine phosphate, cladribine (2-CDA), asparaginase, and gemcitabine.

Antimetabolic agents have widely used to treat several common forms of cancer including carcinomas of colon, rectum, breast, liver, stomach and pancreas, malignant melanoma, acute and chronic leukemia and hair cell leukemia. Many of the adverse effects of antimetabolite treatment result from suppression of cellular proliferation in mitotically active tissues, such as the bone marrow or gastrointestinal mucosa. Patients treated with these agents commonly experience bone marrow suppression, stomatitis, diarrhea, and hair loss. Chen and Grem (1992) Curr. Opin. Oncol. 4: 1089-1098.

## Hormonal Agents

The hormonal agents are a group of drug that regulate the growth and development of their target organs. Most of the hormonal agents are sex steroids and their derivatives and analogs thereof, such as estrogens, progestogens, anti-estrogens, androgens, antiandrogens and progestins. These hormonal agents may serve as antagonists of receptors for the sex steroids to down regulate receptor expression and transcription of vital genes. Examples of such hormonal agents are synthetic estrogens (e.g., diethylstibestrol), antiestrogens (e.g., tamoxifen, toremifene, fluoxymesterol and raloxifene), antiandrogens (bicalutamide, nilutamide, flutamide), aromatase inhibitors (e.g., aminoglutethimide, anastrozole and tetrazole), luteinizing hormone release hormone (LHRH) analogues, ketoconazole, goserelin acetate, leuprolide, megestrol acetate and mifepristone.

Hormonal agents are used to treat breast cancer, prostate cancer, melanoma and meningioma. Because the major action of hormones is mediated through steroid receptors, 60% receptor-positive breast cancer responded to first-line hormonal therapy; and less than 10% of receptor-negative tumors responded. The main side effect associated with hormonal agents is flare. The frequent manifestations are an abrupt increase of bony pain, erythema around skin lesions, and induced hypercalcemia.

Specifically, progestogens are used to treat endometrial cancers, since these cancers occur in women that are exposed to high levels of oestrogen unopposed by progestogen.

Antiandrogens are used primarily for the treatment of prostate cancer, which is hormone dependent. They are used to decrease levels of testosterone, and thereby inhibit growth of the tumor.

Hormonal treatment of breast cancer involves reducing the level of oestrogen-dependent activation of oestrogen receptors in neoplastic breast cells. Anti-oestrogens act by binding to oestrogen receptors and prevent the recruitment of coactivators, thus inhibiting the oestrogen signal.

LHRH analogues are used in the treatment of prostate cancer to decrease levels of testosterone and so decrease the growth of the tumor.

Aromatase inhibitors act by inhibiting the enzyme required for hormone synthesis. In post-menopausal women, the main source of oestrogen is through the conversion of androstenedione by aromatase.

## Plant-derived Agents

Plant-derived agents are a group of drugs that are derived from plants or modified based on the molecular structure of the agents. They inhibit cell replication by preventing the assembly of the cell's components that are essential to cell division.

Examples of plant derived agents include vinca alkaloids (e.g., vincristine, vinblastine, vindesine, vinzolidine and vinorelbine), podophyllotoxins (e.g., etoposide (VP-16) and teniposide (VM-26)), taxanes (e.g., paclitaxel and docetaxel). These plant-derived agents generally act as antimitotic agents that bind to tubulin and inhibit mitosis. Podophyllotoxins such as etoposide are believed to interfere with DNA synthesis by interacting with topoisomerase II, leading to DNA strand scission.

Plant-derived agents are used to treat many forms of cancer. For example, vincristine is used in the treatment of the leukemias, Hodgkin's and non-Hodgkin's lymphoma, and the childhood tumors neuroblastoma, rhabdomyosarcoma, and Wilms' tumor. Vinblastine is used against the lymphomas, testicular cancer, renal cell carcinoma, mycosis fungoides, and Kaposi's sarcoma. Doxetaxel has shown promising activity against advanced breast cancer, non-small cell lung cancer (NSCLC), and ovarian cancer.

Etoposide is active against a wide range of neoplasms, of which small cell lung cancer, testicular cancer, and NSCLC are most responsive.

The plant-derived agents cause significant side effects on patients being treated. The vinca alkaloids display different spectrum of clinical toxicity. Side effects of vinca alkaloids include neurotoxicity, altered platelet function, myelosuppression, and leukopenia. Paclitaxel causes dose-limiting neutropenia with relative sparing of the other hematopoietic cell lines. The major toxicity of the epipophyllotoxins is hematologic (neutropenia and thrombocytopenia).

Other side effects include transient hepatic enzyme abnormalities, alopecia, allergic reactions, and peripheral neuropathy.

## Biologic Agents

Biologic agents are a group of biomolecules that elicit cancer/tumor regression when used alone or in combination with chemotherapy and/or radiotherapy. Examples of biologic agents include immuno-modulating proteins such as cytokines, monoclonal antibodies against tumor antigens, tumor suppressor genes, and cancer vaccines.

Cytokines possess profound immunomodulatory activity. Some cytokines such as interleukin-2 (IL-2, aldesleukin) and interferon-a (IFN-a) demonstrated antitumor activity and have been approved for the treatment of patients with metastatic renal cell carcinoma and metastatic malignant melanoma. IL-2 is a T-cell growth factor that is central to T-cell-mediated immune responses. The selective antitumor effects of IL-2 on some patients are believed to be the result of a cell-mediated immune response that discriminate between self and nonself.

Interferon-α includes more than 23 related subtypes with overlapping activities. IFN-a has demonstrated activity against many solid and hematologic malignancies, the later appearing to be particularly sensitive.

Examples of interferons include, interferon-α, interferon-β (fibroblast interferon) and interferon-γ (fibroblast interferon). Examples of other cytokines include erythropoietin (epoietin- α), granulocyte-CSF (filgrastin), and granulocyte, macrophage-CSF (sargramostim). Other immuno-modulating agents other than cytokines include bacillus Calmette-Guerin, levamisole, and octreotide, a long-acting octapeptide that mimics the effects of the naturally occuring

hormone somatostatin.

Furthermore, the anti-cancer treatment can comprise treatment by immunotherapy with antibodies and reagents used in tumor vaccination approaches. The primary drugs in this therapy class are antibodies, alone or carrying e.g. toxins or chemostherapeutics/cytotoxics to cancer cells. Monoclonal antibodies against tumor antigens are antibodies elicited against antigens expressed by tumors, preferably tumor-specific antigens. For example, monoclonal antibody HERCEPTIN® (trastuzumab) is raised against human epidermal growth factor receptor2 (HER2) that is overexpressed in some breast tumors including metastatic breast cancer. Overexpression of HER2 protein is associated with more aggressive disease and poorer prognosis in the clinic. HERCEPTIIV® is used as a single agent for the treatment of patients with metastatic breast cancer whose tumors over express the HER2 protein.

Another example of monoclonal antibodies against tumor antigens is RITUXAN® (rituximab) that is raised against CD20 on lymphoma cells and selectively deplete normal and malignant CD20+ pre-B and mature B cells.

RITUXAN is used as single agent for the treatment of patients with relapsed or refractory low-grade or follicular, CD20+, B cell non-Hodgkin's lymphoma. MYELOTARG® (gemtuzumab ozogamicin) and CAMPATH® (alemtuzumab) are further examples of monoclonal antibodies against tumor antigens that may be used.

Tumor suppressor genes are genes that function to inhibit the cell growth and division cycles, thus preventing the development of neoplasia. Mutations in tumor suppressor genes cause the cell to ignore one or more of the components of the network of inhibitory signals, overcoming the cell cycle checkpoints and resulting in a higher rate of controlled cell growth-cancer. Examples of the tumor suppressor genes include Duc-4, NF-1, NF-2, RB, p53, WT1, BRCA1 and BRCA2.

DPC4 is involved in pancreatic cancer and participates in a cytoplasmic pathway that inhibits cell division. NF-1 codes for a protein that inhibits Ras, a cytoplasmic inhibitory protein. NF-1 is involved in neurofibroma and pheochromocytomas of the nervous system and myeloid leukemia. NF-2 encodes a nuclear protein that is involved in meningioma, schwanoma, and ependymoma of the nervous system. RB codes for the pRB protein, a nuclear protein that is a major inhibitor of cell cycle. RB is involved in retinoblastoma as well as bone, bladder, small cell lung and breast cancer. P53 codes for p53 protein that regulates cell division and can induce apoptosis. Mutation and/or inaction of p53 is found in a wide ranges of cancers. WTI is involved in Wilms' tumor of the kidneys. BRCA1 is involved in breast and ovarian cancer, and BRCA2 is involved in breast cancer. The tumor suppressor gene can be transferred into the tumor cells where it exerts its tumor suppressing functions.

Cancer vaccines are a group of agents that induce the body's specific immune response to tumors. Most of cancer vaccines under research and development and clinical trials are tumor-associated antigens (TAAs). TAAs are structures (i.e., proteins, enzymes or carbohydrates) that are present on tumor cells and relatively absent or diminished on normal cells. By virtue of being fairly unique to the tumor cell, TAAs provide targets for the immune system to recognize and cause their destruction. Examples of TAAs include gangliosides (GM2), prostate specific antigen (PSA), $\alpha$-fetoprotein (AFP), carcinoembryonic antigen (CEA) (produced by colon cancers and other adenocarcinomas, e.g., breast, lung, gastric, and pancreatic cancers), melanoma-associated antigens (MART-1, gap 100, MAGE 1,3 tyrosinase), papillomavirus E6 and E7 fragments, whole cells or portions/lysates of autologous tumor cells and allogeneic tumor cells.

## Other Therapies

Recent developments have introduced, in addition to the traditional cytotoxic and hormonal therapies used to treat cancer, additional therapies for the treatment of cancer.

For example, many forms of gene therapy are undergoing preclinical or clinical trials.

In addition, approaches are currently under development that are based on the inhibition of tumor vascularization (angiogenesis). The aim of this concept is to cut off the tumor from nutrition and oxygen supply provided by a newly built tumor vascular system.

In addition, cancer therapy is also being attempted by the induction of terminal differentiation of the neoplastic cells. Suitable differentiation agents include the compounds disclosed in any one or more of the following references, the contents of which are incorporated by reference herein.

a) Polar compounds (Marks et al (1987); , Friend, C., Scher, W., Holland, J. W., and Sato, T. (1971) Proc. Natl. Acad. Sci. (USA) 68: 378-382; Tanaka, M., Levy, J., Terada, M., Breslow, R., Rifkind, R. A., and Marks, P. A. (1975) Proc. Natl. Acad. Sci. (USA) 72: 1003-1006; Reuben, R. C., Wife, R. L., Breslow, R., Rifkind, R. A., and Marks, P. A. (1976) Proc. Natl. Acad. Sci. (USA) 73: 862-866);

b) Derivatives of vitamin D and retinoic acid (Abe, E., Miyaura, C., Sakagami, H., Takeda, M., Konno, K., Yamazaki, T., Yoshika, S., and Suda, T. (1981) Proc. Natl. Acad. Sci. (USA) 78: 4990-4994; Schwartz, E. L., Snoddy, J. R., Kreutter, D., Rasmussen, H., and Sartorelli, A. C. (1983) Proc. Am. Assoc. Cancer Res. 24: 18; Tanenaga, K., Hozumi, M., and Sakagami, Y. (1980) Cancer Res. 40: 914-919);

c) Steroid hormones (Lotem, J. and Sachs, L. (1975) Int. J. Cancer 15: 731-740);

d) Growth factors (Sachs, L. (1978) Nature (Lond.) 274: 535, Metcalf, D. (1985) Science, 229: 16-22);
e) Proteases (Scher, W., Scher, B. M., and Waxman, S. (1983) Exp. Hematol. 11: 490-498; Scher, W., Scher, B. M., and Waxman, S. (1982) Biochem. & Biophys. Res. Comm. 109: 348-354);
f) Tumor promoters (Huberman, E. and Callaham, M. F. (1979) Proc. Natl. Acad Sci. (USA) 76: 1293-1297; Lottem, J. and Sachs, L. (1979) Proc. Natl. Acad. Sci. (USA) 76: 5158-5162); and
g) inhibitors of DNA or RNA synthesis (Schwartz, E. L. and Sartorelli, A. C. (1982) Cancer Res. 42: 2651-2655, Terada, M., Epner, E., Nudel, U., Salmon, J., Fibach, E., Rifkind, R. A., and Marks, P. A. (1978) Proc. Natl. Acad. Sci. (USA) 75: 2795-2799; Morin, M. J. and Sartorelli, A. C. (1984) Cancer Res. 44: 2807-2812; Schwartz, E. L., Brown, B. J., Nierenberg, M., Marsh, J. C., and Sartorelli, A. C. (1983) Cancer Res. 43: 2725-2730; Sugano, H., Furusawa, M., Kawaguchi, T., and Ikawa, Y. (1973) Bibl. Hematol. 39: 943-954; Ebert, P. S., Wars, I., and Buell, D. N. (1976) Cancer Res. 36: 1809-1813; Hayashi, M., Okabe, J., and Hozumi, M. (1979) Gann 70: 235-238),

The use of all of these approaches in combination with HDAC inhibitors, e.g. SAHA, are within the scope of the present invention.

## Modes and Doses of Administration

The methods of the present invention comprise administering to a patient in need thereof a first amount of an HDAC inhibitor, e.g., SAHA, in a first treatment procedure, and a second amount of an anti-cancer agent in a second treatment procedure. The first and second treatments together comprise a therapeutically effective amount.

"Patient" as that term is used herein, refers to the recipient of the treatment. Mammalian and non-mammalian patients are included. In a specific embodiment, the patient is a mammal, such as a human, canine, murine, feline, bovine, ovine, swine or caprine. In a particular embodiment, the patient is a human.

## Administration of the HDAC Inhibitor

### Routes of Administration

The HDAC inhibitor (e.g. SAHA), can be administered by any known administration method known to a person skilled in the art. Examples of routes of administration include but are not limited to oral, parenteral, intraperitoneal, intravenous, intraarterial, transdermal, sublingual, intramuscular, rectal, transbuccal, intranasal, liposomal, via inhalation, vaginal, intraoccular, via local delivery by catheter or stent, subcutaneous, intraadiposal, intraarticular, intrathecal, or in a slow release dosage form.

For example, the HDAC inhibitors of the invention can be administered in such oral forms as tablets, capsules (each of which includes sustained release or timed release formulations), pills, powders, granules, elixirs, tinctures, suspensions, syrups, and emulsions. Likewise, the HDAC inhibitors can be administered in intravenous (bolus or infusion), intraperitoneal, subcutaneous, or intramuscular form, all using forms well known to those of ordinary skill in the pharmaceutical arts. A currently preferred administration of the HDAC inhibitor is oral administration.

The HDAC inhibitors can also be administered in the form of a depot injection or implant preparation, which may be formulated in such a manner as to permit a sustained release of the active ingredient. The active ingredient can be compressed into pellets or small cylinders and implanted subcutaneously or intramuscularly as depot injections or implants. Implants may employ inert materials such as biodegradable polymers or synthetic silicones, for example, Silastic, silicone rubber or other polymers manufactured by the Dow-Corning Corporation.

The HDAC inhibitor can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The HDAC inhibitors can also be delivered by the use of monoclonal antibodies as individual carriers to which the compound molecules are coupled.

The HDAC inhibitors can also be prepared with soluble polymers as targetable drug carriers. Such polymers can include polyvinylpyrrolidone, pyran copolymer, polyhydroxy-propyl-methacrylamide-phenol, polyhydroxyethyl-aspartamide-phenol, or polyethyleneoxide-polylysine substituted with palmitoyl residues. Furthermore, the HDAC inhibitors can be prepared with biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and cross linked or amphipathic block copolymers of hydrogels.

In a currently preferred embodiment, the HDAC inhibitor, e.g. SAHA, is administered orally in a gelatin capsule, which can comprise excipients such as microcrystalline cellulose, croscarmellose sodium and magnesium stearate. A further preferred embodiment is 200 mg of solid SAHA with 89.5 mg of microcrystalline cellulose, 9 mg of sodium

croscarmellose and 1.5 mg of magnesium stearate contained in a gelatin capsule.

Dosages and Dosage Schedules

The dosage regimen utilizing the HDAC inhibitors can be selected in accordance with a variety of factors including type, species, age, weight, sex and the type of cancer being treated; the severity (i.e., stage) of the cancer to be treated; the route of administration; the renal and hepatic function of the patient; and the particular compound or salt thereof employed. An ordinarily skilled physician or veterinarian can readily determine and prescribe the effective amount of the drug required to treat, for example, to prevent, inhibit (fully or partially) or arrest the progress of the disease.

For example, SAHA or any one of the HDAC inhibitors can be administered in a total daily dose of up to 800 mg, The HDAC inhibitor can be administered once daily (QD), or divided into multiple daily doses such as twice daily (BID), and three times daily (TID). The HDAC inhibitor can be administered at a total daily dosage of up to 800 mg, e.g., 200 mg, 300 mg, 400 mg, 600 mg or 800 mg, which can be administered in one daily dose or can be divided into multiple daily doses as described above. Preferably, the administration is oral.

In addition, the administration can be continuous, i.e., every day, or intermittently. The terms "intermittent" or "intermittently" as used herein means stopping and starting at either regular or irregular intervals. For example, intermittent administration of an HDAC inhibitor may be administration one to six days per week or it may mean administration in cycles (e.g. daily administration for two to eight consecutive weeks, then a rest period with no administration for up to one week) or it may mean administration on alternate days.

SAHA or any of the HDAC inhibitors are administered to the patient at a total daily dosage of between 25-4000 $mg/m^2$. A currently preferred treatment protocol comprises continuous administration (i.e., every day), once, twice or three times daily at a total daily dose in the range of about 200 mg to about 600 mg.

Another currently preferred treatment protocol comprises intermittent administration of between three to five days a week, once, twice or three times daily at a total daily dose in the range of about 200 mg to about 600 mg.

In one particular embodiment, the HDAC inhibitor is administered continuously once daily at a dose of 400 mg or twice daily at a dose of 200 mg.

In another particular embodiment, the HDAC inhibitor is administered intermittently three days a week, once daily at a dose of 400 mg or twice daily at a dose of 200 mg.

In another particular embodiment, the HDAC inhibitor is administered intermittently four days a week, once daily at a dose of 400 mg or twice daily at a dose of 200 mg.

In another particular embodiment, the HDAC inhibitor is administered intermittently five days a week, once daily at a dose of 400 mg or twice daily at a dose of 200 mg.

In one particular embodiment, the HDAC inhibitor is administered continuously once daily at a dose of 600 mg, twice daily at a dose of 300 mg, or three times daily at a dose of 200 mg.

In another particular embodiment, the HDAC inhibitor is administered intermittently three days a week, once daily at a dose of 600 mg, twice daily at a dose of 300 mg, or three times daily at a dose of 200 mg.

In another particular embodiment, the HDAC inhibitor is administered intermittently four days a week, once daily at a dose of 600 mg, twice daily at a dose of 300 mg, or three times daily at a dose of 200 mg.

In another particular embodiment, the HDAC inhibitor is administered intermittently five days a week, once daily at a dose of 600 mg, twice daily at a dose of 300 mg, or three times daily at a dose of 200 mg.

In addition, the HDAC inhibitor may be administered according to any of the schedules described above, consecutively for a few weeks, followed by a rest period. For example, the HDAC inhibitor may be administered according to any one of the schedules described above from two to eight weeks, followed by a rest period of one week, or twice daily at a dose of 300 mg for three to five days a week. In another particular embodiment, the HDAC inhibitor is administered three times daily for two consecutive weeks, followed by one week of rest.

Intravenously or subcutaneously, the patient would receive the HDAC inhibitor in quantities sufficient to deliver between about 3-1500 $mg/m^2$ per day, for example, about 3, 30, 60, 90, 180, 300, 600, 900, 1200 or 1500 $mg/m^2$ per day. Such quantities may be administered in a number of suitable ways, e.g. large volumes of low concentrations of HDAC inhibitor during one extended period of time or several times a day. The quantities can be administered for one or more consecutive days, intermittent days or a combination thereof per week (7 day period). Alternatively, low volumes of high concentrations of HDAC inhibitor during a short period of time, e.g. once a day for one or more days either consecutively, intermittently or a combination thereof per week (7 day period). For example, a dose of 300 $mg/m^2$ per day can be administered for 5 consecutive days for a total of 1500 $mg/m^2$ per treatment. In another dosing regimen, the number of consecutive days can also be 5, with treatment lasting for 2 or 3 consecutive weeks for a total of 3000 $mg/m^2$ and 4500 $mg/m^2$ total treatment.

Typically, an intravenous formulation may be prepared which contains a concentration of HDAC inhibitor of between about 1.0 mg/mL to about 10 mg/mL, e.g. 2.0 mg/mL, 3.0 mg/mL, 4.0 mg/mL, 5.0 mg/mL, 6.0 mg/mL, 7.0 mg/mL, 8.0 mg/mL, 9.0 mg/mL and 10 mg/mL and administered in amounts to achieve the doses described above. In

one example, a sufficient volume of intravenous formulation can be administered to a patient in a day such that the total dose for the day is between about 300 and about 1500 mg/$m^2$.

Subcutaneous formulations, preferably prepared according to procedures well known in the art at a pH in the range between about 5 and about 12, also include suitable buffers and isotonicity agents, as described below. They can be formulated to deliver a daily dose of HDAC inhibitor in one or more daily subcutaneous administrations, e.g., one, two or three times each day.

The HDAC inhibitors can also be administered in intranasal form via topical use of suitable intranasal vehicles, or via transdermal routes, using those forms of transdermal skin patches well known to those of ordinary skill in that art. To be administered in the form of a transdermal delivery system, the dosage administration will, or course, be continuous rather than intermittent throughout the dosage regime.

It should be apparent to a person skilled in the art that the various modes of administration, dosages and dosing schedules described herein merely set forth specific embodiments and should not be construed as limiting the broad scope of the invention. Any permutations, variations and combinations of the dosages and dosing schedules are included within the scope of the present invention.

## Administration of Anti-Cancer Agent

Any one or more of the specific dosages and dosage schedules of the HDAC inhibitors, is also applicable to any one or more of the anti-cancer agents to be used in the combination treatment.

Moreover, the specific dosage and dosage schedule of the anti-cancer agent can further vary, and the optimal dose, dosing schedule and route of administration will be determined based upon the specific anti-cancer agent that is being used.

Of course, the route of administration of SAHA or any one of the other HDAC inhibitors is independent of the route of administration of the anti-cancer agent. A currently preferred route of administration for SAHA is oral administration. Thus, in accordance with this embodiment, SAHA is administered orally, and the second agent (anti-cancer agent) can be administered orally, parenterally, intraperitoneally, intravenously, intraarterially, transdermally, sublingually, intramuscularly, rectally, transbuccally, intranasally, liposomally, via inhalation, vaginally, intraoccularly, via local delivery by catheter or stent, subcutaneously, intraadiposally, intraarticularly, intrathecally, or in a slow release dosage form.

In addition, the HDAC inhibitor and anti-cancer agent may be administered by the same mode of administration, i.e. both agents administered e.g. orally, by IV. However, it is also within the scope of the present invention to administer the HDAC inhibitor by one mode of administration, e.g. oral, and to administer the anti-cancer agent by another mode of administration, e.g. IV or any other ones of the administration modes described hereinabove.

Commonly used anti-cancer agents and daily dosages usually administered include but are not restricted to:

| | | |
|---|---|---|
| Antimetabolites: | 1. Methotrexate | 20-40 mg/$m^2$ i.v. |
| | | 4-6 mg/$m^2$ p.o. |
| | | 12000 mg/$m^2$ high dose therapy |
| | 2. 6-Mercaptopurine: | 100 mg/$m^2$ |
| | 3. 6- Thioguanine: | 1-2 x 80 mg/$m^2$ p.o. |
| | 4. Pentostatin | 4 mg/$m^2$ i.v. |
| | 5. Fludarabinphosphate: | 25 mg/$m^2$ i.v. |
| | 6. Cladribine: | 0.14 mg/kg BW i.v. |
| | 7. 5-Fluorouracil | 500-2600 mg/$m^2$ i.v. |
| | 8. Capecitabine: | 1250 mg/$m^2$ p.o. |
| | 9. Cytarabin: | 200 mg/$m^2$ i.v. |
| | | 3000 mg/$m^2$ i.v. high dose therapy |
| | 10. Gemcitabine: | 800-1250 mg/$m^2$ i.v. |
| | 11. Hydroxyurea: | 800-4000 mg/$m^2$ p.o. |
| Antibiotics: | 12. Actinomycin D | 0.6 mg/$m^2$ i.v. |
| | 13. Daunorubicin | 45-6.0 mg/$m^2$ i.v. |
| | 14. Doxorubicin | 45-60 mg/$m^2$ i.v. |
| | 15. Epirubicin | 60-80 mg/$m^2$ i.v. |
| | 16. Idarubicin | 10-12 mg/$m^2$ i.v. |
| | | 35-50 mg/$m^2$ p.o. |

(continued)

| | | |
|---|---|---|
| | 17. Mitoxantron | 10-12 mg/$m^2$ i.v. |
| | 18. Bleomycin | 10-15 mg/$m^2$ i.v., i.m., s.c. |
| | 19.Mitomycin C | 10-20 mg/$^2$ i.v. |
| | 20. Irinotecan (CPT -11) | 350 mg/$m^2$ i.v. |
| | 21. Topotecan | 1.5 mg/$m^2$ i.v. |
| Alkylating Agents: | 22. Mustargen | 6 mg/$m^2$ i.v. |
| | 23. Estramustinphosphate | 150-200 mg/$m^2$ i.v.<br>480-550 mg/$m^2$ p.o. |
| | 24. Melphalan | 8-10 mg/$m^2$ i.v.<br>15 mg/$m^2$ i.v. |
| | 25. Chlorambucil | 3-6 mg/$m^2$ i.v. |
| | 26. Prednimustine | 40-100 mg/$m^2$ p.o. |
| | 27. Cyclophosphamide | 750-1200 mg/$m^2$ i.v.<br>50-100 mg/$m^2$ p.o. |
| | 28. Ifosfamide | 1500-2000 mg/$m^2$ i. v. |
| | 29. Trofosfamide | 25-200 mg/$m^2$ p.o. |
| | 30. Busulfan | 2-6 mg/$m^2$ p.o. |
| | 31. Treosulfan | 5000-8000 mg/$m^2$ i.v.<br>750-1500 mg/$m^2$ p.o. |
| | 32. Thiotepa | 12-16 mg/$m^2$ i.v. |
| | 33. Carmustin (BCNU) | 100 mg/$m^2$ i.v. |
| | 34. Lomustin (CCNU) | 100-130 mg/$m^2$ p.o. |
| | 35. Nimustin (ACNU) | 90-100 mg/$m^2$ i.v. |
| | 36. Dacarbazine (OTIC) | 100-375 mg/$m^2$ i.v. |
| | 37. Procarbazine | 100 mg/$m^2$ p.o. |
| | 38. Cisplatin | 20-120 mg/$m^2$ i.v. |
| | 39. Carboplatin | 300-400 mg/$m^2$ i.v. |
| Anti-mitotic agents: | 40. Vincristine | 1.5-2 mg/$m^2$ i.v. |
| | 41. Vinblastine | 4-8 mg/$m^2$ i.v. |
| | 42. Vindesine | 2-3 mg/$m^2$ i.v. |
| | 43. Etoposide (VP16) | 100-200 mg/$m^2$ i.v.<br>100 mg p.o. |
| | 44. Teniposide (VM26) | 20-30 mg/$m^2$ i.v. |
| | 45. Paclitaxel (Taxol) | 175-250 mg/$m^2$ i.v. |
| | 46. Docetaxel (Taxotere) | 100-150 mg/$m^2$ i.v. |
| Hormones, Cytokines and Vitamins: | | |
| | 47. Interferon-a | 2-10 x $10^6$ IU/$_m{}^2$ |
| | 48. Prednisone | 40-100 mg/$m^2$ p.o. |
| | 49. Dexamethasone | 8-24 mg p.o. |
| | 50. G-CSF | 5-20 μg/kg BW s.c. |
| | 51. al/-trans Retinoic Acid | 45 mg/$m^2$ |
| | 52. Interleukin-2 | 18 x $10^6$ IU/$m^2$ |
| | 53. GM-CSF | 250 mg/$m^2$ |
| | 54. erythropoietin | 150 IU/kg tiw |

## Combination Administration

The first treatment procedure, administration of an HDAC inhibitor, can take place prior to the second treatment procedure, i.e., the anti-cancer agent, after the treatment with the anti-cancer agent, at the same time as the treatment

with the anti-cancer agent, or a combination thereof. For example, a total treatment period can be decided for the HDAC inhibitor. The anti-cancer agent can be administered prior to onset of treatment with the inhibitor or following treatment with the inhibitor. In addition, anti-cancer treatment can be administered during the period of inhibitor administration but does not need to occur over the entire inhibitor treatment period.

SAHA or any one of the HDAC inhibitors can be administered in accordance with any dose and dosing schedule that, together with the effect of the anti-cancer agent, achieves a dose effective to treat cancer.

## Pharmaceutical compositions

As described above, the compositions comprising the HDAC inhibitor and/or the anti-cancer agent can be formulated in any dosage form suitable for oral, parenteral, intraperitoneal, intravenous, intraarterial, transdermal, sublingual, intramuscular, rectal, transbuccal, intranasal, liposomal, via inhalation, vaginal, or intraocular administration, for administration via local delivery by catheter or stent, or for subcutaneous, intraadiposal, intraarticular, intrathecal administration, or for administration in a slow release dosage form.

The HDAC inhibitor and the anti-cancer agent can be formulated in the same formulation for simultaneous administration, or they can be in two separate dosage forms, which may be administered simultaneously or sequentially as described above.

The invention also encompasses pharmaceutical compositions comprising pharmaceutically acceptable salts of the HDAC inhibitors and/or the anti-cancer agents. Suitable pharmaceutically acceptable salts of the compounds described herein and suitable for use in the method of the invention, are conventional non-toxic salts and can include a salt with a base or an acid addition salt such as a salt with an inorganic base, for example, an alkali metal salt (e.g., lithium salt, sodium salt, potassium salt, etc.), an alkaline earth metal salt (e.g., calcium salt, magnesium salt, etc.), an ammonium salt; a salt with an organic base, for example, an organic amine salt (e.g., triethylamine salt, pyridine salt, picoline salt, ethanolamine salt, triethanolamine salt, dicyclohexylamine salt, N,N'-dibenzylethylenediamine salt, etc.) etc.; an inorganic acid addition salt (e.g., hydrochloride, hydrobromide, sulfate, phosphate, etc.); an organic carboxylic or sulfonic acid addition salt (e.g., formate, acetate, trifluoroacetate, maleate, tartrate, methanesulfonate, benzenesulfonate, p-toluenesulfonate, etc.); a salt with a basic or acidic amino acid (e.g., arginine, aspartic acid, glutamic acid, etc.) and the like.

The invention also encompasses pharmaceutical compositions comprising hydrates of the HDAC inhibitors and/or the anti-cancer agents. The term "hydrate" includes but is not limited to hemihydrate, monohydrate, dihydrate, trihydrate and the like.

In addition, this invention also encompasses pharmaceutical compositions comprising any solid or liquid physical form of SAHA or any of the other HDAC inhibitors. For example, The HDAC inhibitors can be in a crystalline form, in amorphous form, and have any particle size. The HDAC inhibitor particles may be micronized, or may be agglomerated, particulate granules, powders, oils, oily suspensions or any other form of solid or liquid physical form.

For oral administration, the pharmaceutical compositions can be liquid or solid. Suitable solid oral formulations include tablets, capsules, pills, granules, pellets and the like. Suitable liquid oral formulations include solutions, suspensions, dispersions, emulsions, oils and the like.

Any inert excipient that is commonly used as a carrier or diluent may be used in the formulations of the present invention, such as for example, a gum, a starch, a sugar, a cellulosic material, an acrylate, or mixtures thereof. The compositions may further comprise a disintegrating agent and a lubricant, and in addition may comprise one or more additives selected from a binder, a buffer, a protease inhibitor, a surfactant, a solubilizing agent, a plasticizer, an emulsifier, a stabilizing agent, a viscosity increasing agent, a sweetener, a film forming agent, or any combination thereof. Furthermore, the compositions of the present invention may be in the form of controlled release or immediate release formulations.

The HDAC inhibitors can be administered as active ingredients in admixture with suitable pharmaceutical diluents, excipients or carriers (collectively referred to herein as "carrier" materials or "pharmaceutically acceptable carriers") suitably selected with respect to the intended form of administration. As used herein, "pharmaceutically acceptable carrier" is intended to include any and all solvents, dispersion media, coatings, antibacterial and antifungal agents, isotonic and absorption delaying agents, and the like, compatible with pharmaceutical administration.

Suitable carriers are described in the most recent edition of Remington's Pharmaceutical Sciences, a standard reference text in the field, which is incorporated herein by reference.

For liquid formulations, pharmaceutically acceptable carriers may be aqueous or non-aqueous solutions, suspensions, emulsions or oils. Examples of non-aqueous solvents are propylene glycol, polyethylene glycol, and injectable organic esters such as ethyl oleate. Aqueous carriers include water, alcoholic/aqueous solutions, emulsions or suspensions, including saline and buffered media. Examples of oils are those of petroleum, animal, vegetable, or synthetic origin, for example, peanut oil, soybean oil, mineral oil, olive oil, sunflower oil, and fish-liver oil. Solutions or suspensions can also include the following components: a sterile diluent such as water for injection, saline solution, fixed oils, polyethylene glycols, glycerine, propylene glycol or other synthetic solvents; antibacterial agents such as benzyl alcohol or

methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The pH can be adjusted with acids or bases, such as hydrochloric acid or sodium hydroxide.

Liposomes and non-aqueous vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

Solid carriers/diluents include, but are not limited to, a gum, a starch (e.g., corn starch, pregelatinized starch), a sugar (e.g., lactose, mannitol, sucrose, dextrose), a cellulosic material (e.g., microcrystalline cellulose), an acrylate (e.g., polymethylacrylate), calcium carbonate, magnesium oxide, talc, or mixtures thereof.

In addition, the compositions may further comprise binders (e.g., acacia, cornstarch, gelatin, carbomer, ethyl cellulose, guar gum, hydroxypropyl cellulose, hydroxypropyl methyl cellulose, povidone), disintegrating agents (e.g., cornstarch, potato starch, alginic acid, silicon dioxide, croscarmellose sodium, crospovidone, guar gum, sodium starch glycolate, Primogel), buffers (e.g., tris-HCI, acetate, phosphate) of various pH and ionic strength, additives such as albumin or gelatin to prevent absorption to surfaces, detergents (e.g., Tween 20, Tween 80, Pluronic F68, bile acid salts), protease inhibitors, surfactants (e.g., sodium lauryl sulfate), permeation enhancers, solubilizing agents (e.g., glycerol, polyethylene glycerol), a glidant (e.g., colloidal silicon dioxide), anti-oxidants (e.g., ascorbic acid, sodium metabisulfite, butylated hydroxyanisole), stabilizers (e.g., hydroxypropyl cellulose, hyroxypropylmethyl cellulose), viscosity increasing agents (e.g., carbomer, colloidal silicon dioxide, ethyl cellulose, guar gum), sweeteners (e.g., sucrose, aspartame, citric acid), flavoring agents (e.g., peppermint, methyl salicylate, or orange flavoring), preservatives (e.g., Thimerosal, benzyl alcohol, parabens), lubricants (e.g., stearic acid, magnesium stearate, polyethylene glycol, sodium lauryl sulfate), flow-aids (e.g., colloidal silicon dioxide), plasticizers (e.g., diethyl phthalate, triethyl citrate), emulsifiers (e.g., carbomer, hydroxypropyl cellulose, sodium lauryl sulfate), polymer coatings (e.g., poloxamers or poloxamines), coating and film forming agents (e.g., ethyl cellulose, acrylates, polymethacrylates) and/or adjuvants.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The pharmaceutical compositions can be included in a container, pack, or dispenser together with instructions for administration.

The preparation of pharmaceutical compositions that contain an active component is well understood in the art, for example, by mixing, granulating, or tablet-forming processes. The active therapeutic ingredient is often mixed with excipients that are pharmaceutically acceptable and compatible with the active ingredient. For oral administration, the active agents are mixed with additives customary for this purpose, such as vehicles, stabilizers, or inert diluents, and converted by customary methods into suitable forms for administration, such as tablets, coated tablets, hard or soft gelatin capsules, aqueous, alcoholic or oily solutions and the like as detailed above.

The amount of the compound administered to the patient is less than an amount that would cause toxicity in the patient. In the certain embodiments, the amount of the compound that is administered to the patient is less than the amount that causes a concentration of the compound in the patient's plasma to equal or exceed the toxic level of the compound. Preferably, the concentration of the compound in the patient's plasma is maintained at about 10 nM. In another embodiment, the concentration of the compound in the patient's plasma is maintained at about 25 nM. In another embodiment, the concentration of the compound in the patient's plasma is maintained at about 50 nM. In another embodiment, the concentration of the compound in the patient's plasma is maintained at about 100 nM. In another embodiment, the concentration of the compound in the patient's plasma is maintained at about 500 nM. In another embodiment, the concentration of the compound in the patient's plasma is maintained at about 1000 nM. In another embodiment, the concentration of the compound in the patient's plasma is maintained at about 2500 nM. In another

embodiment, the concentration of the compound in the patient's plasma is maintained at about 5000 nM. It has been found with HMBA that administration of the compound in an amount from about 5 gm/$m^2$/day to about 30 gm/$m^2$/day, particularly about 20 gm/$m^2$/day, is effective without producing toxicity in the patient. The optimal amount of the compound that should be administered to the patient in the practice of the present invention will depend on the particular compound used and the type of cancer being treated.

The percentage of the active ingredient and various excipients in the formulations may vary. For example, the composition may comprise between 20 and 90%, preferably between 50-70% by weight of the active agent:

For IV administration, Glucuronic acid, L-lactic acid, acetic acid, citric acid or any pharmaceutically acceptable acid/conjugate base with reasonable buffering capacity in the pH range acceptable for intravenous administration can be used as buffers. Sodium chloride solution wherein the pH has been adjusted to the desired range with either acid or base, for example, hydrochloric acid or sodium hydroxide, can also be employed. Typically, a pH range for the intravenous formulation can be in the range of from about 5 to about 12. A preferred pH range for intravenous formulation comprising an HDAC inhibitor, wherein the HDAC inhibitor has a hydroxamic acid moiety, can be about 9 to about 12.

Subcutaneous formulations, preferably prepared according to procedures well known in the art at a pH in the range between about 5 and about 12, also include suitable buffers and isotonicity agents. They can be formulated to deliver a daily dose of the active agent in one or more daily subcutaneous administrations. The choice of appropriate buffer and pH of a formulation, depending on solubility of the HDAC inhibitor to be administered, is readily made by a person having ordinary skill in the art. Sodium chloride solution wherein the pH has been adjusted to the desired range with either acid or base, for example, hydrochloric acid or sodium hydroxide, can also be employed in the subcutaneous formulation. Typically, a pH range for the subcutaneous formulation can be in the range of from about 5 to about 12. A preferred pH range for subcutaneous formulation of an HDAC inhibitor a hydroxamic acid moiety, can be about 9 to about 12.

The compositions of the present invention can also be administered in intranasal form via topical use of suitable intranasal vehicles, or via transdermal routes, using those forms of transdermal skin patches well known to those of ordinary skill in that art. To be administered in the form of a transdermal delivery system, the dosage administration will, or course, be continuous rather than intermittent throughout the dosage regime.

The present invention also provides in-vitro methods for selectively inducing terminal differentiation, cell growth arrest and/or apoptosis of neoplastic cells, thereby inhibiting proliferation of such cells, by contacting the cells with a first amount of suberoylanilide hydroxamic acid (SAHA) or a pharmaceutically acceptable salt or hydrate thereof, and a second amount of an anti-cancer agent, wherein the first and second amounts together comprise an amount effective to induce terminal differentiation, cell growth arrest of apoptosis of the cells.

Although the methods of the present invention can be practiced *in vitro,* it is contemplated that the preferred embodiment for the methods of selectively inducing terminal differentiation, cell growth arrest and/or apoptosis of neoplastic cells will comprise contacting the cells in vivo, i.e., by administering the compounds to a subject harboring neoplastic cells or tumor cells in need of treatment.

As such, the present invention also provides methods for selectively inducing terminal differentiation, cell growth arrest and/or apoptosis of neoplastic cells, thereby inhibiting proliferation of such cells in a subject by administering to the subject a first amount of suberoylanilide hydroxamic acid (SAHA) or a pharmaceutically acceptable salt or hydrate thereof, in a first treatment procedure, and a second amount of an anti-cancer agent in a second treatment procedure, wherein the first and second amounts together comprise an amount effective to induce terminal differentiation, cell growth arrest of apoptosis of the cells.

The invention is illustrated in the examples in the Experimental Details Section that follows. This section is set forth to aid in an understanding of the invention but is not intended to, and should not be construed to limit in any way the invention as set forth in the claims which follow thereafter.

## EXPERIMENTAL DETAILS SECTION

### EXAMPLE 1:

### Synthesis of SAHA

SAHA can be synthesized according to the method outlined below, or according to the method set forth in US Patent 5,369,108, the contents of which are incorporated by reference in their entirety, or according to any other method.

Synthesis of SAHA

Step 1- Synthesis of Suberanilic acid

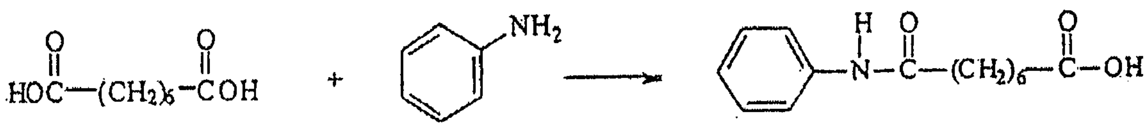

In a 22 L flask was placed 3,500 g (20.09 moles) of suberic acid, and the acid melted with heat. The temperature was raised to 175˚C, and then 2,040 g (21.92 moles) of aniline was added. The temperature was raised to 190˚C and held at that temperature for 20 minutes. The melt was poured into a Nalgene tank that contained 4,017 g of potassium hydroxide dissolved in 50 L of water. The mixture was stirred for 20 minutes following the addition of the melt. The reaction was repeated at the same scale, and the second melt was poured into the same solution of potassium hydroxide. After the mixture was thoroughly stirred, the stirrer was turned off, and the mixture was allowed to settle. The mixture was then filtered through a pad of Celite (4,200 g) (the product was filtered to remove the neutral by-product (from attack by aniline on both ends of suberic acid). The filtrate contained the salt of the product, and also the salt of unreacted suberic acid. The mixture was allowed to settle because the filtration was very slow, taking several days.). The filtrate was acidified using 5 L of concentrated hydrochloric acid; the mixture was stirred for one hour, and then allowed to settle overnight. The product was collected by filtration, and washed on the funnel with deionized water (4 x 5 L). The wet filter cake was placed in a 72 L flask with 44 L of deionized water, the mixture heated to 50˚C, and the solid isolated by a hot filtration (the desired product was contaminated with suberic acid which is has a much greater solubility in hot water. Several hot triturations were done to remove suberic acid. The product was checked by NMR [$D_6$DMSO] to monitor the removal of suberic acid). The hot trituration was repeated with 44 L of water at 50˚C. The product was again isolated by filtration, and rinsed with 4 L of hot water. It was dried over the weekend in a vacuum oven at 65˚C using a Nash pump as the vacuum source (the Nash pump is a liquid ring pump (water) and pulls a vacuum of about 29 inch of mercury. An intermittent argon purge was used to help carry off water); 4,182.8 g of suberanilic acid was obtained.

The product still contained a small amount of suberic acid; therefore the hot trituration was done portionwise at 65˚C, using about 300 g of product at a time. Each portion was filtered, and rinsed thoroughly with additional hot water (a total of about 6 L). This was repeated to purify the entire batch. This completely removed suberic acid from the product. The solid product was combined in a flask and stirred with 6 L of methanol/water (1:2), and then isolated by filtration and air dried on the filter over the week end. It was placed in trays and dried in a vacuum oven at 65˚C for 45 hours using the Nash pump and an argon bleed. The final product has a weight of 3,278.4 g (32.7% yield).

Step 2 -Synthesis of Methyl Suberanilate

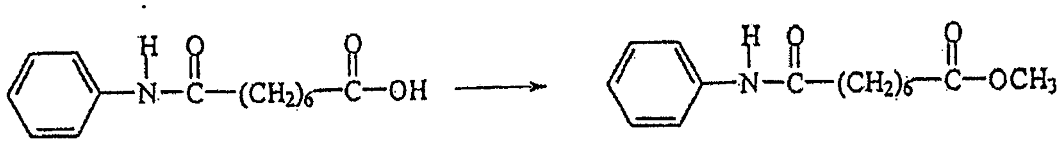

To a 50 L flask fitted with a mechanical stirrer, and condenser was placed 3,229 g of suberanilic acid from the previous step, 20 L of methanol, and 398.7 g of Dowex 50WX2-400 resin. The mixture was heated to reflux and held at reflux for 18 hours. The mixture was filtered to remove the resin beads, and the filtrate was taken to a residue on a rotary evaporator.

The residue from the rotary evaporator was transferred into a 50 L flask fitted with a condenser and mechanical stirrer. To the flask was added 6 L of methanol, and the mixture heated to give a solution. Then 2 L of deionized water was added, and the heat turned off. The stirred mixture was allowed to cool, and then the flask was placed in an ice bath, and the mixture cooled. The solid product was isolated by filtration, and the filter cake was rinsed with 4 L of cold

methanol/water (1:1). The product was dried at 45˚C in a vacuum oven using a Nash pump for a total of 64 hours to give 2,850.2 g (84% yield) of methyl suberanilate, CSL Lot # 98-794-92-3 1.

Step 3 - Synthesis of Crude SAHA

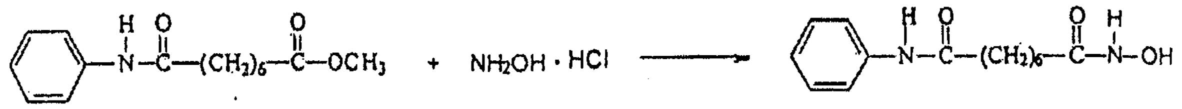

To a 50 L flask with a mechanical stirrer, thermocouple, and inlet for inert atmosphere was added 1,451.9 g of hydroxylamine hydrochloride, 19 L of anhydrous methanol, and a 3.93 L of a 30% sodium methoxide solution in methanol. The flask was then charged with 2,748.0 g of methyl suberanilate, followed by 1.9 L of a 30% sodium methoxide solution in methanol. The mixture was allowed to stir for 16 hr and 10 minutes. Approximately one half of the reaction mixture was transferred from the reaction flask (flask 1) to a 50 L flask (flask 2) fitted with a mechanical stirrer. Then 27 L of deionized water was added to flask 1 and the mixture was stirrer for 10 minutes. The pH was taken using a pH meter; the pH was 11.56. The pH of the mixture was adjusted to 12.02 by the addition of 100 ml of the 30% sodium methoxide solution in methanol; this gave a clear solution (the reaction mixture at this time contained a small amount of solid. The pH was adjusted to give a clear solution from which the precipitation the product would be precipitated). The reaction mixture in flask 2 was diluted in the same manner; 27 L of deionized water was added, and the pH adjusted by the addition of 100 ml of a 30 % sodium methoxide solution to the mixture, to give a pH of 12.01 (clear solution).

The reaction mixture in each flask was acidified by the addition of glacial acetic acid to precipitate the product. Flask 1 had a final pH of 8.98, and Flask 2 had a final pH of 8.70. The product from both flasks was isolated by filtration using a Buchner funnel and filter cloth. The filter cake was washed with 15 L of deionized water, and the funnel was covered and the product was partially dried on the funnel under vacuum for 15.5 hr. The product was removed and placed into five glass trays. The trays were placed in a vacuum oven and the product was dried to constant weight. The first drying period was for 22 hours at 60˚C using a Nash pump as the vacuum source with an argon bleed. The trays were removed from the vacuum oven and weighed. The trays were returned to the oven and the product dried for an additional 4 hr and 10 minutes using an oil pump as the vacuum source and with no argon bleed. The material was packaged in double 4-mill polyethylene bags, and placed in a plastic outer container. The final weight after sampling was 2633.4 g (95.6%).

Step 4 - Recrystallization of Crude SAHA

The crude SAHA was recrystallized from methanol/water. A 50 L flask with a mechanical stirrer, thermocouple, condenser, and inlet for inert atmosphere was charged with the crude SAHA to be crystallized (2,525.7 g), followed by 2,625 ml of deionized water and 15,755 ml of methanol. The material was heated to reflux to give a solution. Then 5,250 ml of deionized water was added to the reaction mixture. The heat was turned off, and the mixture was allowed to cool. When the mixture had cooled sufficiently so that the flask could be safely handled (28˚C), the flask was removed from the heating mantle, and placed in a tub for use as a cooling bath. Ice/water was added to the tub to cool the mixture to -5˚C. The mixture was held below that temperature for 2 hours. The product was isolated by filtration, and the filter cake washed with 1.5 L of cold methanol/water (2:1). The funnel was covered, and the product was partially dried under vacuum for 1.75 hr. The product was removed from the funnel and placed in 6 glass trays. The trays were placed in a vacuum oven, and the product was dried for 64.75 hr at 60˚C using a Nash pump as the vacuum source, and using an argon bleed. The trays were removed for weighing, and then returned to the oven and dried for an additional 4 hours at 60˚C to give a constant weight. The vacuum source for the second drying period was a oil pump, and no argon bleed was used. The material was packaged in double 4-mill polyethylene bags, and placed in a plastic outer container. The final weight after sampling was 2,540.9 g (92.5%).

## EXAMPLE 2-

### Effect of SAHA and Gemcitabine Combinations in T24 Cell Line

SAHA was used in combination with gemcitabine, leading to an observed combinatorial synergistic effect that is greater than the additive effect that would have been obtained by using each of the agents alone.

Materials and Methods:

Cells were plated at a density of 1.25 x $10^4$ cells/ml in MEM alpha medium with 10% FCS, and were allowed to adhere to wells.

Gemcitabine was reconstituted in MEM alpha medium and the pH was adjusted to 7 using 1N NaOH. Concentrations of gemcitabine were prepared by serial dilution of gemcitabine in complete medium. Concentrations of SAHA were prepared from 1 mM stock solutions.

Cells were left untreated, treated with SAHA alone, gemcitabine alone, or simultaneously with a combination of SAHA and gemcitabine by aspirating wells and refilling with the relevant medium at the indicated concentrations. The cells were then cultured with medium containing the compound or combination of compounds.

To assay for proliferation and viability, triplicate samples of cells were harvested and counted for proliferation and viability at the indicated time points. To harvest, the contents of each well were removed with 0.5 ml trypsin, transferred to a 15 ml tube, and cells were centrifuges and re-suspended in 1 ml medium. Harvested cells were counted on a hemocytometer for proliferation. Viability was determined by trypan blue exclusion.

Results:

T24 cells were cultured in complete medium (control), with 2 nM gemcitabine, with 5 μM SAHA, or with a combination of 2 nM gemcitabine and 5μM SAHA for 96 hours.

The results are depicted in Figure 1A (showing cell proliferation), and Figure 1B (showing cell viability). As shown in Figure 1, treatment of cells with the combination of SAHA and gemcitabine inhibits the proliferation of significantly more cells than either SAHA or gemcitabine alone.

The combination of gemcitabine and SAHA produces a significantly better result than the additive effects of each constituent when it is administered alone - i.e. a synergistic response, providing the added advantage over an additive response.

## EXAMPLE 3 -

### Effect of SAHA and Gemcitabine Combinations in a LnCap Cell Line

Materials and Methods:

Cells were plated at a density of 2.5 x $10^4$ cells/ml in RMPI medium with 10% FCS, and were allowed to adhere to wells.

Gemcitabine was reconstituted in medium and the pH was adjusted to 7 using 1N NaOH. Concentrations of gemcitabine were prepared by serial dilution of gemcitabine in complete medium. Concentrations of SAHA were prepared from 1 mM stock solutions.

Cells were left untreated, treated with SAHA alone, gemcitabine alone, or simultaneously with a combination of SAHA and gemcitabine by aspirating wells and refilling with the relevant medium at the indicated concentrations. The cells were then cultured with medium containing the compound or combination of compounds.

To assay for proliferation and viability, triplicate samples of cells were harvested and counted for proliferation and viability at the indicated time points as described above in Example 2.

Results:

LnCap cells were cultured in complete medium (control), with 2 nM gemcitabine, with 5 μM SAHA, or with a combination of 2 nM gemcitabine and 5μM SAHA for 72 hours.

The results are depicted in Figure 2A (showing cell proliferation), and Figure 2B (showing cell viability). As shown in Figure 2, treatment of cells with gemcitabine alone produced a small effect on the cells, whereas SAHA treatment significantly inhibited proliferation. Treatment with a combination of SAHA and gemcitabine produced an additive effect.

**EXAMPLE 4-**

**Effect of SAHA and 5-azacytidine Combinations in a T24 Cell Line**

Materials and Methods:

Cells were cultured in T-150 flasks at 37˚C in RPMI with 10% FCS. Cells were diluted in a complete medium to a density of 5.0 x $10^4$ cells/ml. The cells were incubated at 37˚C for 14 hours before treatment with 5-azacytidine to allow cells to adhere to the wells.

Concentrations of 5-azacytidine were prepared by serial dilution of from a 1 mM stock. After 14-hour incubation in complete medium, wells were aspirated and the medium was replaced to 1 ml of 5-azacytidine at the indicated concentration. The cells were pre-incubated for 27.5 hours in 5-azacytidine before addition of SAHA.

Concentrations of SAHA were prepared from 1 mM stock solutions.

After pre-incubation in medium alone (control) or with 5-azacytidine, wells were aspirated and well contents were replaced by 1 ml of medium alone (control), medium containing 5-azacytidine alone, medium containing SAHA alone, or medium containing a combination of 5-azacytidine and SAHA.

To assay for proliferation and viability, triplicate samples of cells were harvested and counted for proliferation and viability at the indicated time points as described above in Example 2.

Results:

T24 cells were cultured in complete medium (control), with 200 nM 5-azacytidine, with 5 μM SAHA, or with a combination of 200 nM 5-azacytidine and 5μM SAHA according to the method described above.

The results are depicted in Figure 3A (showing cell proliferation), and Figure 3B (showing cell viability). As shown in Figure 3, treatment of cells with 5-azacytidine alone or SAHA alone significantly inhibited proliferation. Treatment with a combination of SAHA and 5-azacytidine produced an additive effect achieving essentially a complete inhibition of proliferation relative to the initial cell count.

**EXAMPLE 5-**

**Effect of SAHA in Combination with Etoposide, Doxorubicin, 5-Fluorouracil, Mitoxantrone, and Oxaliplatin in Breast, Glioblastoma and Prostate Cancer Cell Lines**

Study Objective:

The purpose of these studies was to determine if SAHA in combination with the therapeutic agents listed in Table 1 would more effectively inhibit cell growth and colony formation that either drug alone. All combination agents are commercially available and were purchased through Sigma. For these studies, 5 different cell lines representing three common cancer types were tested (Table 2). Anti-proliferative effects were observed with several agents in both assays, in general additive effects were observed.

Table 1. Drugs Used in Combination with SAHA.

| Therapeutic Agents |
|---|
| Etoposide (Eto) |
| Doxorubicin (Dox) |
| 5-Fluorouracil (5-FU) |
| Mitoxantrone (Mitox) |
| Oxaliplatin (Oxal) |

Table 2. Cell Lines Used for SAHA Combination Studies.

| Cell Lines |
|---|
| MDA-231 breast |

(continued)

| Cell Lines |
|---|
| U-118 glioblastoma |
| DU-145 prostate |
| PC-3 prostate |
| LnCap prostate |

Cell Growth Assay:

The cell growth inhibition assay used the commercially available MTS assay, also referred to as the Cell Titer 96 Aqueous One Solution Cell Proliferation Assay. The MTS reagent contains a novel tetrazolium compound [3-(4,5-dimethylthiazol-2-yl)-5-(3-carboxymethoxyphenyl)-2-(4-sulfophenyl)-2H-tetrazolium, inner salt] and electron-coupling reagent (phenazine ethosulfate;PES). Assays were performed by adding a small amount of the MTS reagent directly to culture wells, incubating for 1-4 hours and then recording the absorbance at 490nM with a 96-well plate reader. The quantity of formazan product as measured by the amount of 490nM absorbance is directly proportional to the number of living cells in culture. Treatment regimens for the MTS assays were performed in two different ways. In one method, the plated cells were pretreated with SAHA for 4 hours and then washed free of SAHA before the combination agent was added for the remainder of the 48-hour incubation. In the other method, the cells were treated with SAHA for 48 hours prior to adding the second agent for 4 hours. The cells were then washed and allowed to grow for 48 hours.

Colony Formation Assay:

The colony formation assay was performed as follows. Cells were by plating in 6cm dishes at 200-300 cells/dish and allowed to adhere for 24 hours. Cells were treated with SAHA for 48 hours and then the combination drug was added for an additional 4 hours. The cells were then washed and colonies were allowed to grow for 2-3 week and then stained with 2% crystal violet in methanol. All colonies of a threshold size (~0.2mm) in each dish were counted. Duplicate dishes were counted per treatment group and the range in colony number/dish is shown as error bars.

Results:

A. Effects of SAHA Combinations on MDA-231 Cell Proliferation (Figure 4)

In one experiment, MDA-231 breast cancer cells were pretreated with the indicated concentration of SAHA for 4 hours, washed, and then the second agent was added for 48 hours. Cell growth was quantitated using the MTS assay. The results are depicted in Figure 4A.

In another experiment, Cells were pretreated with the indicated concentration of SAHA for 48 hours, the second agent was added for 4 hours, and then the cells were washed. Cell growth was quantitated 48 hours later using the MTS assay. The results are depicted in Figure 4B.

As shown in Figure 4, combination treatment of SAHA with the therapeutic agents Etoposide, Doxorubicin, 5-Fluorouracil, Mitoxantrone and Oxaliplatin at the indicated concentrations produced an anti-proliferative effect that is greater than treatment with each agent alone. The effect appears to be additive.

B. Effects of SAHA Combinations on DU145 Cell Proliferation (Figure 5)

Cells were pretreated with the indicated concentration of SAHA for 48 hours, the second agent was added for 4 hours, and then the cells were washed. Cell growth was quantitated 48 hours later using the MTS assay.

As shown in Figure 5, combination treatment with SAHA and the therapeutic agents Etoposide, Doxorubicin, 5-Fluorouracil, Mitoxantrone and Oxaliplatin at the indicated concentrations produced an anti-proliferative effect that is greater than treatment with each agent alone. The effect appears to be additive.

C. Effects of SAHA Combinations on DU145 Cell Clonogenicity (Figure 6)

Cells were treated with SAHA for 48 hours, the second agent was then added for 4 hours and then the cells were washed. Colony formation was evaluated 2-3 weeks later.

As shown in Figure 6, combination treatment with SAHA and the therapeutic agents Etoposide, Doxorubicin,

and Oxaliplatin at the indicated concentrations reduced the number of colonies to a greater extent than treatment with each agent alone. The effect appears to be additive.

D. Effects of SAHA Combinations on MDA-231 Cell Clonogenicity (Figure 7)

Cells were treated with SAHA for 48 hours, the second agent was then added for 4 hours and then the cells were washed. Colony formation was evaluated 2-3 weeks later.
As shown in Figure 7, combination treatment with SAHA and the therapeutic agents Etoposide, Doxorubicin, 5-Fluorouracil and Oxaliplatin at the indicated concentrations reduced the number of colonies to a greater extent than treatment with each agent alone. The effect appears to be additive.

E. Effects of SAHA Combinations on U118 Cell Clonogenicity (Figure 8)

Cells were treated with SAHA for 48 hours, the second agent was then added for 4 hours and then the cells were washed. Colony formation was evaluated 2-3 weeks later.
As shown in Figure 8, combination treatment with SAHA and the therapeutic agents Etoposide, Doxorubicin, 5-Fluorouracil and Oxaliplatin at the indicated concentrations reduced the number of colonies to a greater extent than treatment with each agent alone. The effect appears to be additive.

**EXAMPLE 6**-

**Effect of SAHA in Combination with Chemotherapeutic Agents Irinotecan, 5-Fluorouracil and Docetaxel**

Study Objective and Summary:

The purpose of these studies was to evaluate the effects of SAHA on transformed bladder carcinoma (T24), prostate cancer (LnCap), breast cancer (MCF7), non-Hodgkin's lymphoma (DLCL) and colon carcinoid (LCC18) cell lines *in vitro* when administered in paired combination with three clinically implemented anticancer agents: Irinotecan, 5-Fluorouraci (5-FU)1 and Docetaxel.
Transformed cells were treated with various combinations of SAHA and one of these agents in order to assess if each pair (SAHA plus agent) is able to additively, synergistically or antagonistically exert an anti-proliferative effect. The results suggest the effects of SAHA in combination with Irinotecan, 5-Fluorouracil and Docetaxel are mostly additive. In some experiments, a synergistic effect was observed. The most pronounce synergistic effect occurred in LnCap cells, using a SAHA and Docetaxel combination, as described hereinbelow.

Irinotecan, 5-Fluorouracil and Docetaxel

Each of these three agents is currently used clinically in cancer chemotherapy and have all been extensively characterized.
Irinotecan works to stabilize nuclear topoisomerase I/DNA complexes, which results in the accumulation of single-stranded breaks in DNA and thus leads to apoptosis. Clinically, it has been used to treat a wide variety of cancers, including breast, colorectal, cervical, ovarian and small cell/non-small cell lung (Hardman W.E. et al. (1999) Br J Cancer 81, 440-448).
5-Fluorouracil (5-FU) acts as a pyrimidine antagonist that inhibits the methylation of deoxyuridylic acid to thymidylic acid and subsequently the synthesis of DNA and RNA (http://www.nursespdr.com/members/database/ndrhtml/fluorouracil.html). This drug has been used extensively as a chemotherapy agent over the last decades and has also been administered clinically in combination with other anticancer agents including Irinotecan (Awada A. et al. (2002) Eur J. Cancer 38, 773-778).
Docetaxel is used clinically as an antineoplastic agent that disrupts the microtubular network within tumor cells, which can aid in suppressing cell division. By binding to free tubulin, it enhances the assembly and inhibits the depolymerization of microtubules (Chou T. et al. (1991) Synergism and Antagonism in Chemotherapy. New York: Academic Press).

Materials and Methods:

Drug combination experiments were performed on five human cancer cell lines: T24 (bladder carcinoma), LnCap (prostate), MCF7 (breast), DLCL (non-Hodgkin's lymphoma) and LCC18 (colon carcinoid). Each cell line was cultured and incubated at 37˚C in its required medium: MEM α (10% FCS), RPMI 1640 (10% FCS), DME HG (10%

FCS), enhanced RPMI (10% FCS), and Hites medium (5% FCS), respectively.

Adherent cell lines (T24, LnCap, MCF7 and LCC18) were plated on 96 well plates 24 hours before treatment with SAHA and anticancer agents, to allow time for the cells to attach to the well bottoms. DLCL, a suspension cell line, was plated on 96 well plates on the same day of the experiment. For T24, LnCap, DLCL and LCC18, 200 μL containing 2000 cells were plated into each well. For MCF7, 4000 cells/well were plated to account for the line's slower cell cycle. Cells were plated onto two 96 well plates for each SAHA and anticancer agent combination experiment.

All SAHA and combination drug treatments were prepared using cell-line specific media on the same day of treatment. Cells that received no treatment served as a control group.

To administer treatment to adherent cell lines (T24, LnCap, MCF7), media in each well was aspirated and replaced with 200 μL of media containing desired concentration of drug or drugs. For all other cell lines (OCC18 and DLCL), 22 μL of media containing 10 times the desired concentration of drug or drugs was added to 200 μL of media in each well.

After treatment, cells were incubated at 37°C for 4 days. On the fourth day, each well was treated with 20 μL of alamarBlur™, an aqueous dye, and incubated for 4 hours at 37°C. The reduction of this dye, when absorbed by cells, is greater in proliferating cells than in non-proliferating cells because of an increased concentration of NADPH, FADH, FMNH and NADH. The reduction was measured by fluorescence using a SpectaMax GeminiXS® spectrofluorometric microtiter well plate reader (Molecular Devices Corporation, Sunnyvale, CA). Data was expressed as fluorescence emission intensity units as a function of time of incubation and analyzed using SOFTmax PRO® v. 4.0 software (Molecular Devices Corp. Sunnyvale, CA). In order to assess the percent inhibition of cell growth four days after drug treatment, the following formula was used:

$$100 - \frac{\text{(Mean Intensity Units/Well with Identical Treatment)} * 100}{\text{(Mean Intensity Units/Well in Control Group)}}$$

The percent standard error for each percent inhibition assessment was calculated using the following formula:

$$100 - \frac{\text{(Std. Error of Intensity Units/Well with Identical Treatment)} * 100}{\text{(Std. ErrorIntensity Units/Well in Control Group)}}$$

The concentrations used for treatment with each drug are based on the dose that inhibits 50% of proliferation for each drug. The following concentrations were tested:

a) 50% effective dose/4; b) 50% effective dose/2; c) 50% effective dose; d) 50% effective dose*2; and e) 50% effective dose*4. The dose that inhibits 50% of proliferation for each drug was determined in preliminary experiments in which cells were treated with a wide range of concentrations of SAHA alone and Irinotecan, 5-FU and Docetaxel alone. A polynomial relationship was defined between drug concentration and percent inhibition using various concentrations, and this function was used to predict the concentration of each drug required to inhibit proliferation of 50% of the cells.

The following criteria were established for assessing additive, synergistic and antagonistic interactions:

An additive interaction was observed if the % inhibition of cells treated with SAHA plus agent combination was greater than the % inhibition of cells treated with SAHA alone or anticancer agent alone, but less than or equal to the expected % inhibition of cells treated with drug combination if purely additive (i.e. % inhibition with SAHA alone + % inhibition with agent alone).

Synergy was observed if the % inhibition of cells treated with SAHA plus agent combination was greater than the expected % inhibition of cells treated with drug combination if purely additive (% inhibition with SAHA alone + % inhibition with agent alone).

Antagonistic interaction was observed if the % inhibition of cells treated with SAHA alone or agent alone was greater than the expected % inhibition of cells treated with drug combination.

**Results:**

A. Determination of 50% cell proliferation effective doses:

The 50% cell proliferation effective doses of Irinotecan, 5-FU and Docetaxel in combination with increasing SAHA concentrations were analyzed. The results are depicted in Tables 3 and 4. The combination of SAHA and each of the anticancer agents results in a lowering of the required concentration of the agents to inhibit 50% of cell growth. In almost every experiment, an increase in SAHA concentration resulted in a lower concentration of the agent requirement to reach this amount of inhibition. For example, 5.1 nM of Irinotecan alone was required to inhibit 50% of T24 bladder cancer cells; however, when 0.625 μM of SAHA is administered in combination with Irinotecan, the concentration of drug required decreases to 4.1 nM. When the concentration of SAHA was further increased to 2.5 μM, for example, the required Irinotecan concentration continues to decrease (1.9 nM).

**Table 3: T24 50% inhibition. Effective Dose for Representative Combination Treatment**

| | Alone | SAHA (0.625*) | SAHA (1.25) | SAHA (2.5) | SAHA (5.0) | SAHA (10.0) |
|---|---|---|---|---|---|---|
| Irinotecan (nM) | 5.1 | 4.1 | 2.8 | 1.9 | 1.4 | 0.28 |
| 5-FU (μM) | 14.0 | 13.1 | 11.5 | -- | 2.7 | 1.5 |
| Docetaxel (nM) | 2.1 | 0.51 | 0.72 | 0.59 | 0.29 | 0.05 |
| * All concentrations of SAHA are in μM. SAHA alone: 5.0 μM | | | | | | |

**Table 4: LnCap 50% inhibition. Effective Dose for Representative Combination Treatment**

| | Alone | SAHA (0.125*) | SAHA (0.25) | SAHA (0.5) | SAHA (1.0) | SAHA (2.0) |
|---|---|---|---|---|---|---|
| Irinotecan (nM) | 5.1 | 5.1 | 4.1 | 3.8 | 1.9 | 1.9 |
| 5-FU (μM) | 3.0 | 5.2 | -- | 1.6 | 1.9 | 0.99 |
| Docetaxel (nM) | 2.1 | 1.2 | 1.2 | 0.6 | 0.34 | 0.12 |
| * All concentrations of SAHA are in μM. SAHA alone: 1.7 μM | | | | | | |

B. SAHA and Irinotecan:

SAHA and Irinotecan combinations were tested in four cell lines: T24, LnCap, DLCL and LCC18. Interactions were assessed to be mostly additive in all four cell lines.

The results are depicted in terms of the percent of each type of interaction (additive, synergistic and antagonistic) out of the total (100%) of the tested combination treatments.

**Table 5: Interaction observed in different cell lines after combination treatment with SAHA and Irinotecan**

| Type of Cell | Additive (%) | Synergistic (%) | Antagonistic (%) |
|---|---|---|---|
| T24 | 86 | 0 | 14 |
| LCC18 | 62 | 19 | 19 |
| DLCL | 50 | 10 | 40 |
| LnCap | 59 | 25 | 16 |

In T24 bladder cancer cells, 86% of the combination treatments resulted in additive interactions. No synergy was reported. 14% of the treatments resulted in an antagonistic interaction. Additive interactions occurred in treatments of high and low concentrations of both SAHA and Irinotecan (SAHA: 0.625-10μM, Irinotecan: 2.6-10 nM).

In LnCap prostate cell lines, 25% of the combination treatments resulted in a synergistic effect. 59% of the treatments resulted in additive interactions, which mostly occurred at middle and high concentrations of SAHA and low

through high concentrations of Irinotecan (SAHA: 0.5-2 μM; Irinotecan: 2.6-10 nM).

Half of the combination treatments on DLCL resulted in additive interaction and 40% were antagonistic. Antagonism mostly occurred at higher concentrations.

Sixty two percent of treatments on LCC18 cells showed additive interactions, while 19% interacted synergistically and an equal percent antagonistically. Interactions were not specific to any concentration range of SAHA, but all synergistic interactions occurred at low Irinotecan concentrations (1.9 nM).

A representative graph showing the individual responses is depicted in Figure 9 (LnCap). Similar graphs were plotted for the other cell lines (not shown).

B. SAHA and 5-Fluorouracil:

As with Irinotecan, most SAHA and 5-FU combination treatments resulted in additive interactions. SAHA and 5-FU combinations were tested in four cell lines: T24, LnCap and LCC18.

The results are depicted in terms of the percent of each type of interaction (additive, synergistic and antagonistic) out of the total (100%) of the tested combination treatments.

**Table 6: Interaction observed in different cell lines after combination treatment with SAHA and 5-FU**

| Type of Cell | Additive (%) | Synergistic (%) | Antagonistic (%) |
|---|---|---|---|
| T24 | 80 | 6 | 14 |
| LCC18 | 49 | 7 | 44 |
| LnCap | 60 | 33 | 7 |

In T24 bladder cancer cells, 80% of the combination treatments resulted in additive interactions. These occurred mostly at middle and high concentrations of SAHA (2.5 μM - 10 μM).

In LnCap prostate cell lines, 33% of the combination treatments resulted in a synergistic effect. 60% of the treatments resulted in additive interactions, which mostly occurred at middle and high concentrations of SAHA (1.0-2.0 μM). Most synergy occurred at low SAHA concentrations (0.5 μM)

49% of LCC18 treatments resulted in an additive effect. 44% of LCC18 treatments resulted in an antagonistic effect. Neither interaction occurred at any specific concentration range.

A representative graph showing the individual responses is depicted in Figure 10 (LnCap). Similar graphs were plotted for the other cell lines (not shown).

C. SAHA and Docetaxel:

SAHA and Docetaxel combination experiments were performed on T24, LnCap and LCC18 cells.

The results are depicted in terms of the percent of each type of interaction (additive, synergistic and antagonistic) out of the total (100%) of the tested combination treatments.

**Table 7: Interaction observed in different cell lines after combination treatment with SAHA and Docetaxel**

| Type of Cell | Additive (%) | Synergistic (%) | Antagonistic (%) |
|---|---|---|---|
| T24 | 80 | 5 | 15 |
| LCC18 | 72 | 8 | 20 |
| LnCap | 38 | 56 | 6 |

In T24 bladder cancer cells, 80% of the combination treatments resulted in additive interactions and 5% of the interactions were synergistic.

The greatest synergistic effect occurred in the SAHA/Docetaxel combination experiments in LnCap cells (56%). This synergy primarily occurred at low and middle-range concentration of SAHA (0.25-1 μM). Thirty eight percent of the treatments resulted in additive interaction at mostly high SAHA concentrations (2.0 μM).

Additive interactions and synergy resulted in 72% and 8% of combination treatments in LCC18 cells, respectively.

A representative graph showing the individual responses is depicted in Figure 11 (LnCap). Similar graphs were

plotted for the other cell lines (not shown).

In summary, the results show that in general SAHA interacts mostly additively with Irinotecan, 5-FU and Docetaxel. However, importantly, SAHA interacts mostly synergistically with Docetaxel in LnCap cells. Other synergistic effects were seen in several of the concentrations tested, in particular in LnCap cell lines.

**CONCLUSIONS:**

The results of all the combination studies described hereinabove indicate that combination treatment with SAHA and other anti-cancer agents may be useful for cancer therapy, since the dosage of each agent in a combination therapy can be reduced as compared with monotherapy with the agent, while still achieving an overall anti-tumor effect. The combination treatment is particularly useful when a synergistic effect between the two agents is observed, as depicted hereinabove.

While this invention has been particularly shown and described with references to preferred embodiments thereof, it will be understood by those skilled in the art that various changes in form and details may be made therein without departing from the meaning of the invention described. Rather, the scope of the invention is defined by the claims that follow:

## Claims

1. Suberoylanilide hydroxamic acid (SAHA), represented by the structure:

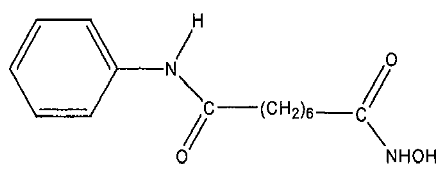

or a pharmaceutically acceptable salt or hydrate thereof, and an antimetabolic agent, for use in the treatment of cancer.

2. The SAHA or pharmaceutically acceptable salt or hydrate thereof, and antimetabolic agent of claim 1, wherein the antimetabolic agent is selected from floxuridine, fluorouracil, methotrexate, leucovorin, hydroxyurea, thioguanine, mercaptopurine, cytarabine, pentostatin, fludarabine phosphate, cladribine, asparaginase, capecitabine and gemcitabine.

3. The SAHA or pharmaceutically acceptable salt or hydrate thereof, and antimetabolic agent of claim 2, wherein said antimetabolic agent is gemcitabine.

4. The SAHA or pharmaceutically acceptable salt or hydrate thereof, and antimetabolic agent of claim 3, wherein said gemcitabine is for intravenous administration.

5. The SAHA or pharmaceutically acceptable salt or hydrate thereof, and antimetabolic agent of claim 1, wherein SAHA or pharmaceutically acceptable salt or hydrate thereof is for administration orally.

6. The SAHA or pharmaceutically acceptable salt or hydrate thereof, and antimetabolic agent of any one of claims 1-5, wherein the cancer is selected from the group consisting of lung cancer, ovarian cancer, bladder cancer, and prostate cancer.

7. An *in-vitro* method of selectively inducing terminal differentiation of neoplastic cells and thereby inhibiting proliferation of said cells, said method comprising contacting the cells with suberoylanilide hydroxamic acid (SAHA) represented by the structure:

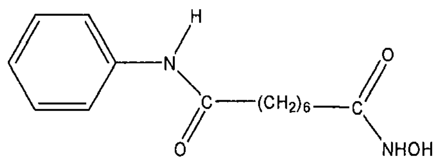

or a pharmaceutically acceptable salt or hydrate thereof, and an antimetabolic agent, thereby inducing terminal differentiation of said cells.

**8.** Use of suberoylanilide hydroxamic acid (SAHA) represented by the structure:

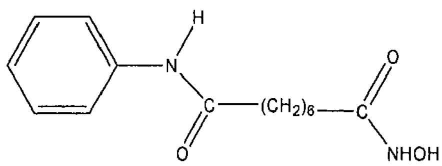

or a pharmaceutically acceptable salt or hydrate thereof, in the manufacture of a medicament for administration in combination with an antimetabolic agent for the treatment of cancer.

**9.** Use of an antimetabolic agent in the manufacture of a medicament, for administration in combination with suberoylanilide hydroxamic acid (SAHA) represented by the structure:

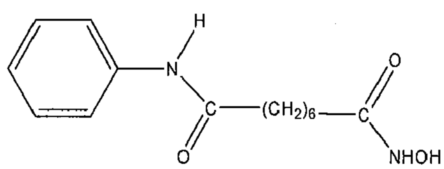

or a pharmaceutically acceptable salt or hydrate thereof, for the treatment of cancer.

**10.** The use of claim 8 or 9, wherein the antimetabolic agent is selected from floxuridine, fluorouracil, methotrexate, leucovorin, hydroxyurea, thioguanine, mercaptopurine, cytarabine, pentostatin, fludarabine phosphate, caldribine, asparaginase, capecitabine, and gemcitabine.

**11.** The use of claim 10, wherein said antimetabolic agent is gemcitabine.

**12.** The use of claim 11, wherein gemcitabine is for intravenous administration.

**13.** The use of claim 12, wherein SAHA or pharmaceutically acceptable salt or hydrate thereof is for administration orally.

**14.** The use of any one of claims 8 to 13, wherein the cancer is selected from the group consisting of lung cancer, ovarian cancer, bladder cancer, and prostate cancer.

# FIG. 1A

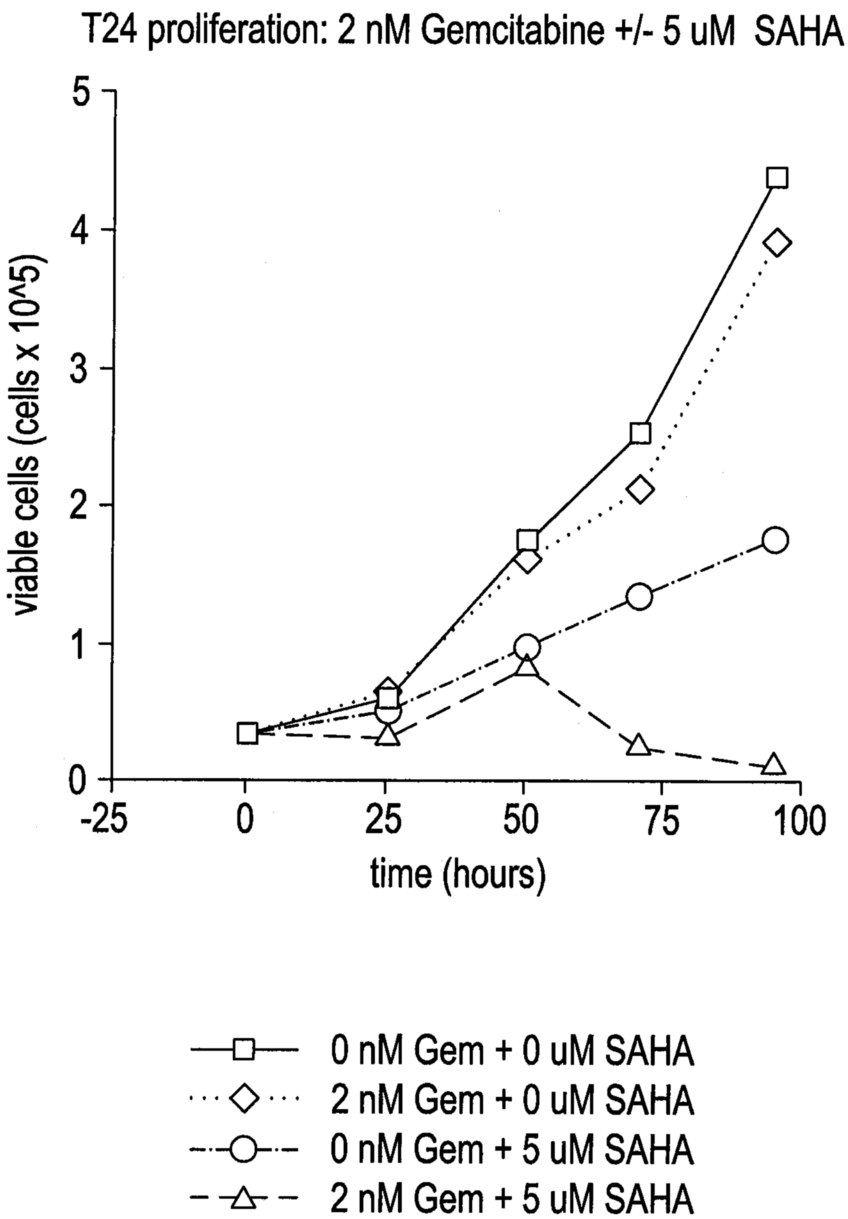
T24 proliferation: 2 nM Gemcitabine +/- 5 uM SAHA
viable cells (cells x 10^5)
5
4
3
2
1
0
-25
0
25
50
75
100
time (hours)
0 nM Gem + 0 uM SAHA
2 nM Gem + 0 uM SAHA
0 nM Gem + 5 uM SAHA
2 nM Gem + 5 uM SAHA

# FIG. 1B

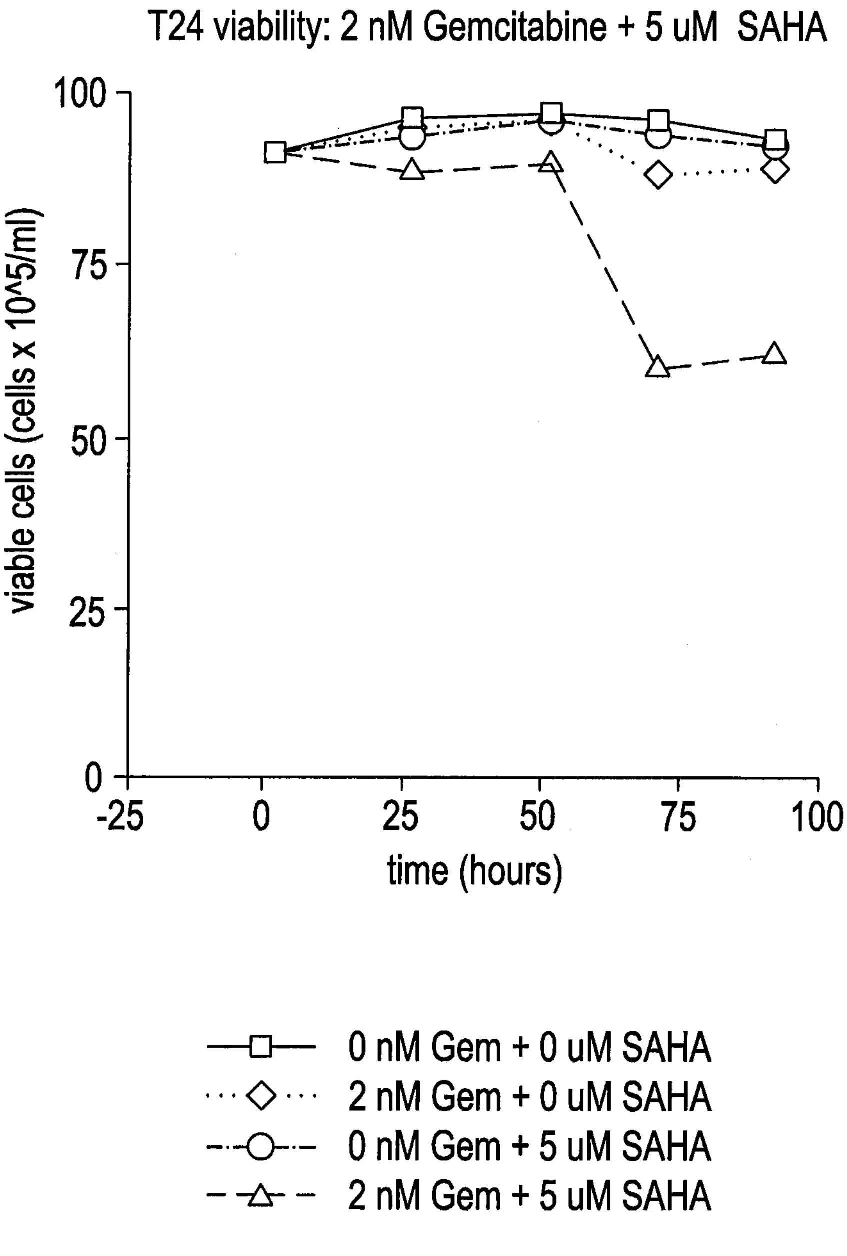
T24 viability: 2 nM Gemcitabine + 5 uM SAHA
viable cells (cells x 10^5/ml)
100
75
50
25
0
-25
0
25
50
75
100
time (hours)
0 nM Gem + 0 uM SAHA
2 nM Gem + 0 uM SAHA
0 nM Gem + 5 uM SAHA
2 nM Gem + 5 uM SAHA

# FIG. 2A

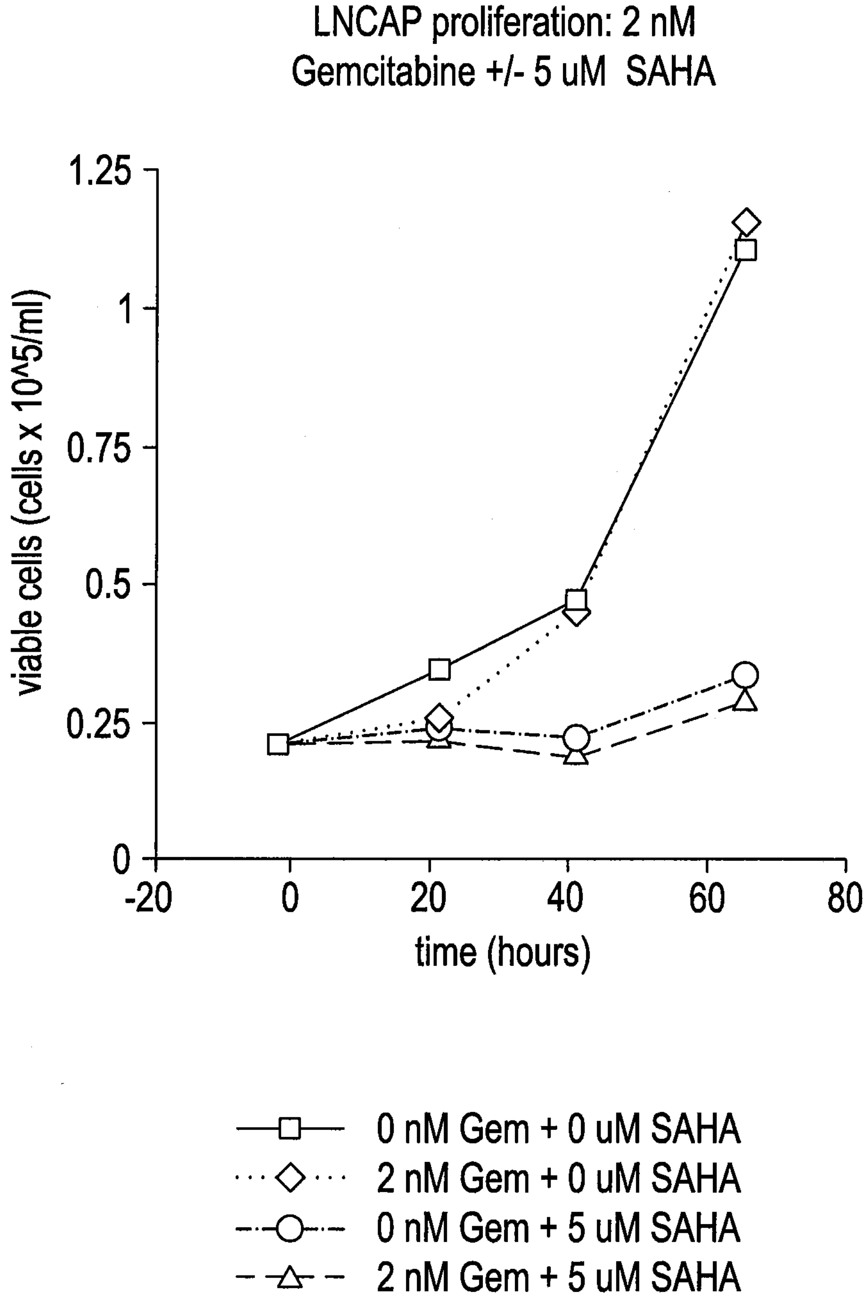
LNCAP proliferation: 2 nM
Gemcitabine +/- 5 uM SAHA
viable cells (cells x 10^5/ml)
1.25
1
0.75
0.5
0.25
0
-20
0
20
40
60
80
time (hours)
0 nM Gem + 0 uM SAHA
2 nM Gem + 0 uM SAHA
0 nM Gem + 5 uM SAHA
2 nM Gem + 5 uM SAHA

# FIG. 2B

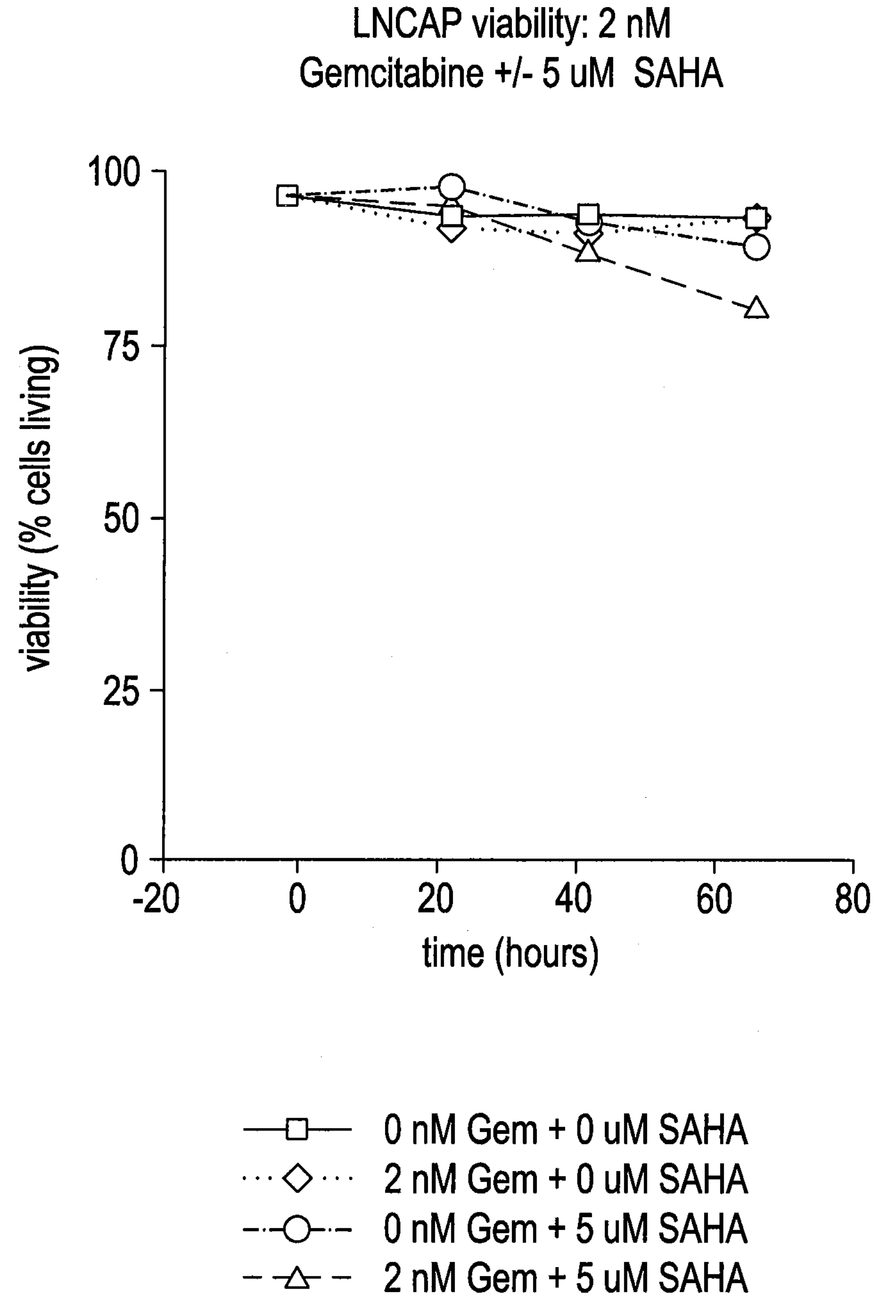
LNCAP viability: 2 nM
Gemcitabine +/- 5 uM SAHA
100
75
50
25
0
viability (% cells living)
-20
0
20
40
60
80
time (hours)
0 nM Gem + 0 uM SAHA
2 nM Gem + 0 uM SAHA
0 nM Gem + 5 uM SAHA
2 nM Gem + 5 uM SAHA

# FIG. 3A

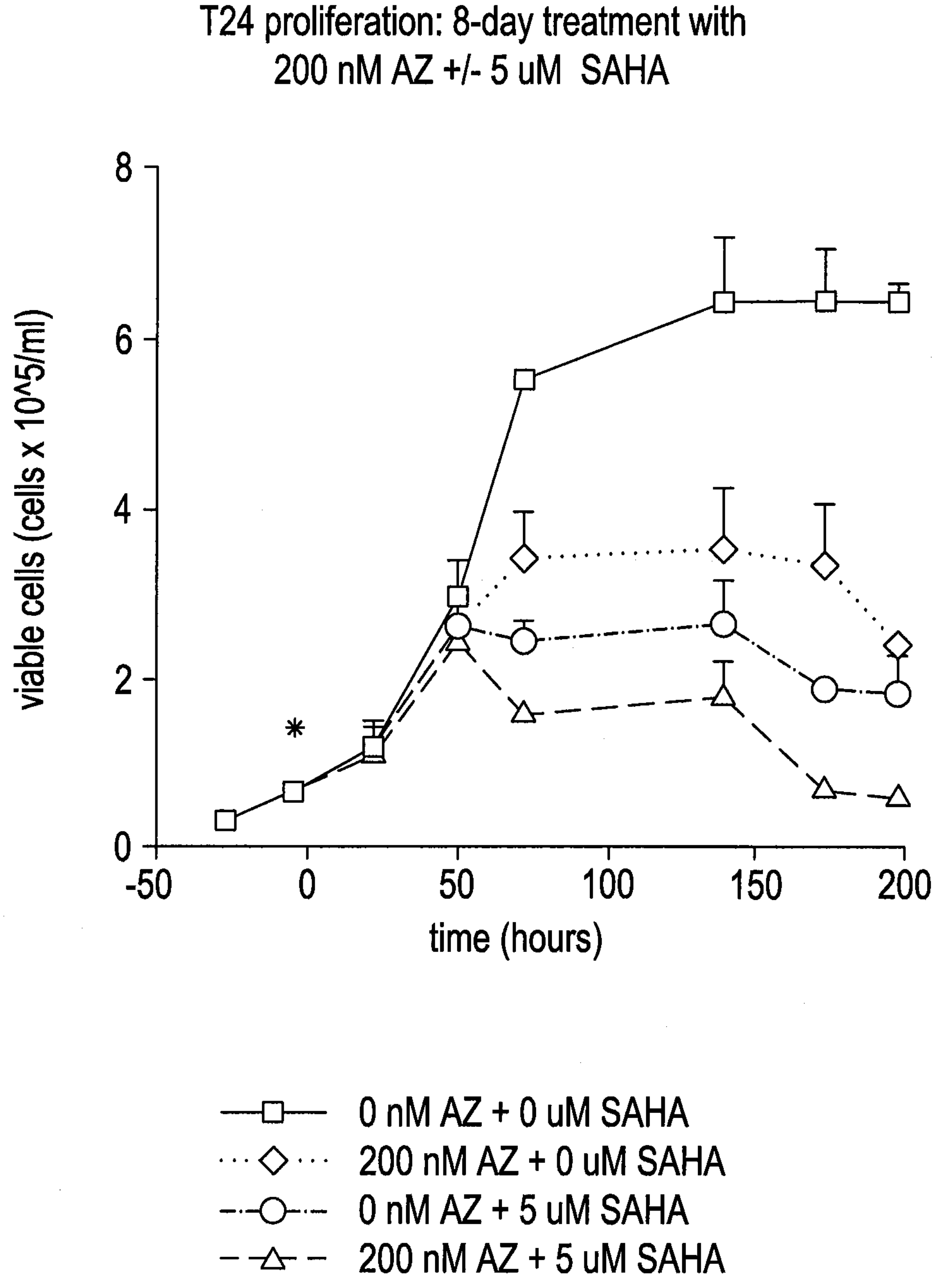
T24 proliferation: 8-day treatment with
200 nM AZ +/- 5 uM SAHA
viable cells (cells x 10^5/ml)
0
2
4
6
8
-50
0
50
100
150
200
time (hours)
*
0 nM AZ + 0 uM SAHA
200 nM AZ + 0 uM SAHA
0 nM AZ + 5 uM SAHA
200 nM AZ + 5 uM SAHA

FIG. 3B

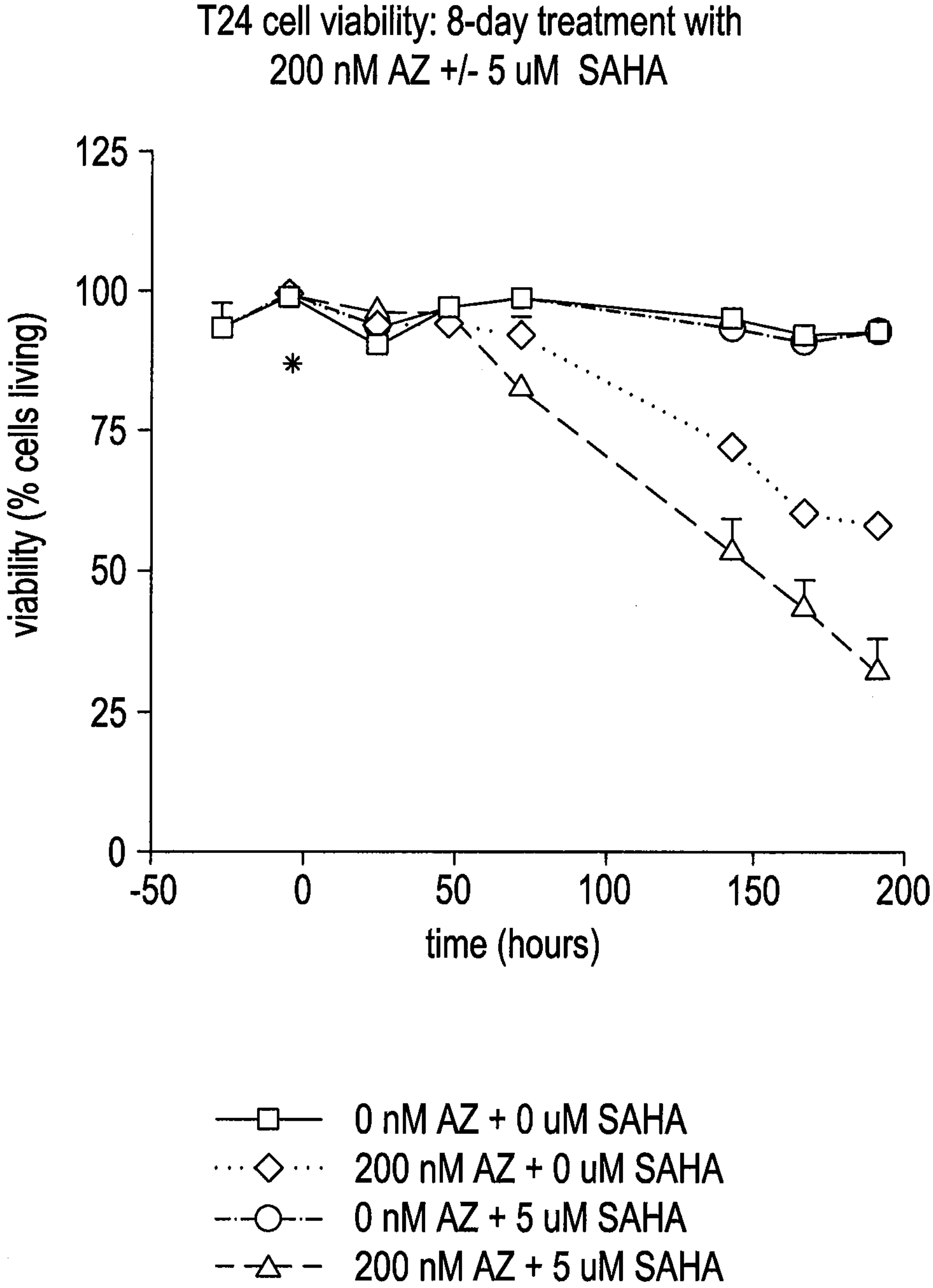
T24 cell viability: 8-day treatment with
200 nM AZ +/- 5 uM SAHA
viability (% cells living)
125
100
75
50
25
0
-50
0
50
100
150
200
time (hours)
*
0 nM AZ + 0 uM SAHA
200 nM AZ + 0 uM SAHA
0 nM AZ + 5 uM SAHA
200 nM AZ + 5 uM SAHA

FIG. 4A

Growth Inhibition of MDA cells
Combination Treatment (SAHA 4hrs + 2nd drug 48 hrs)

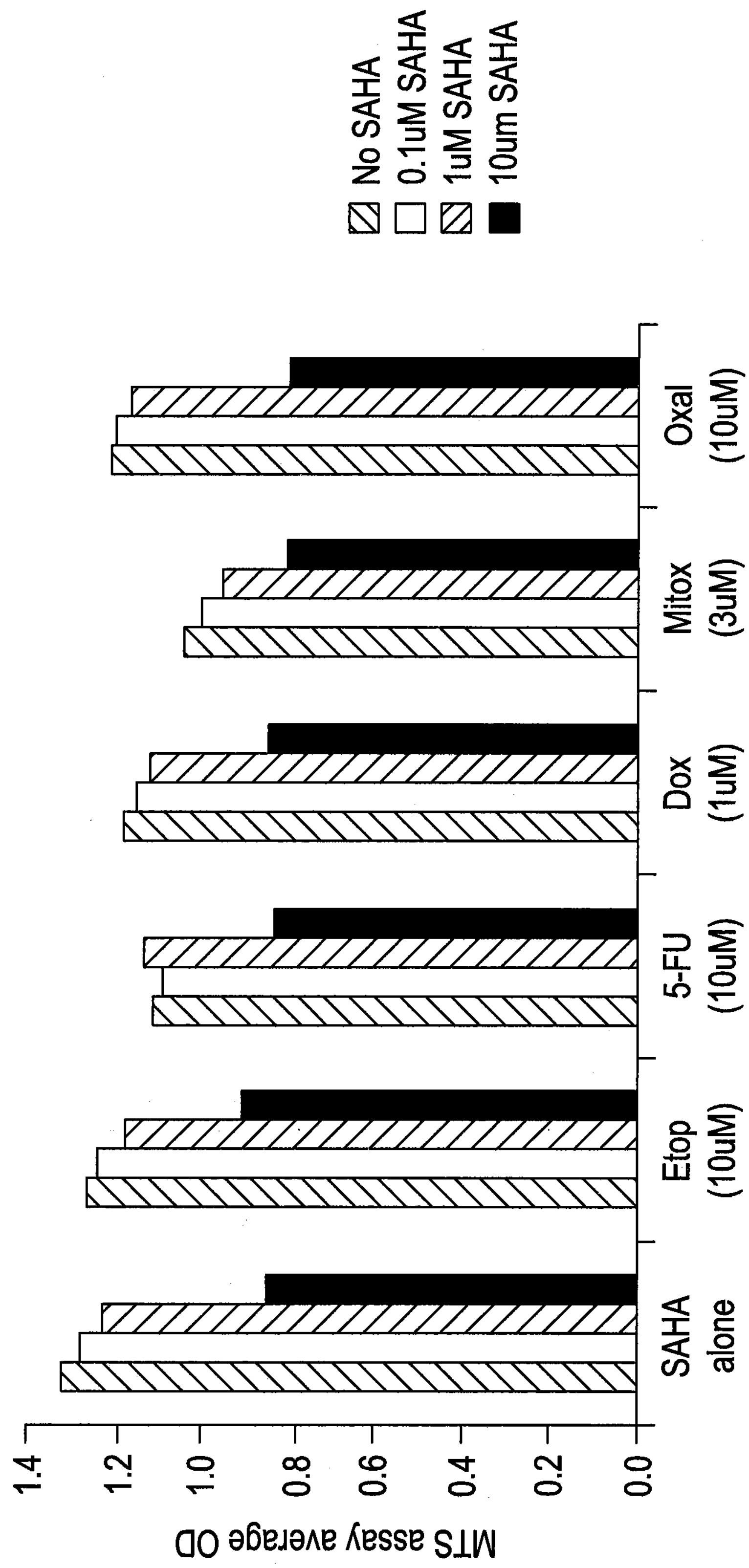

FIG. 4B

Effect of combination drug treatment on MDA cell growth

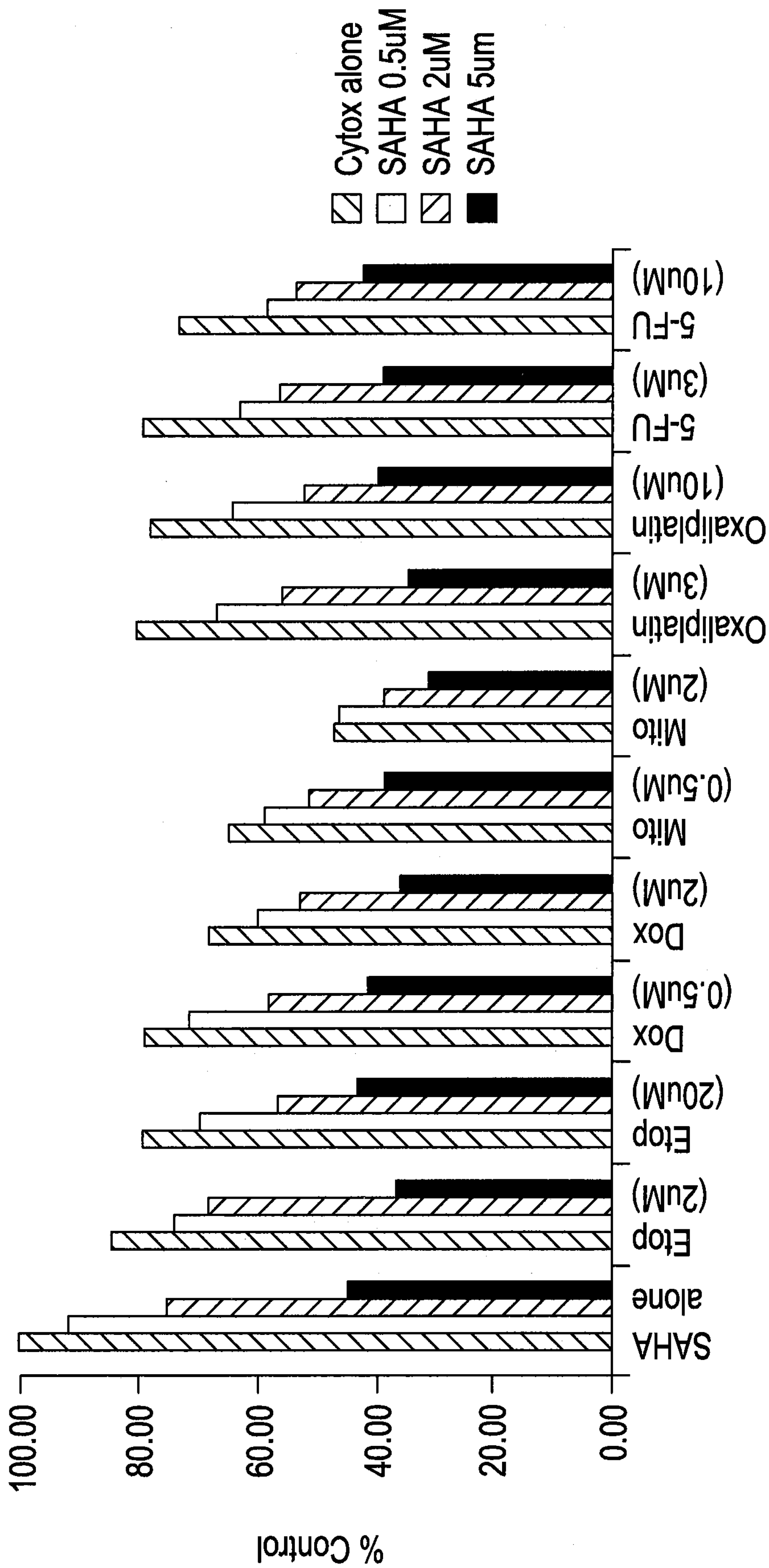

FIG. 5

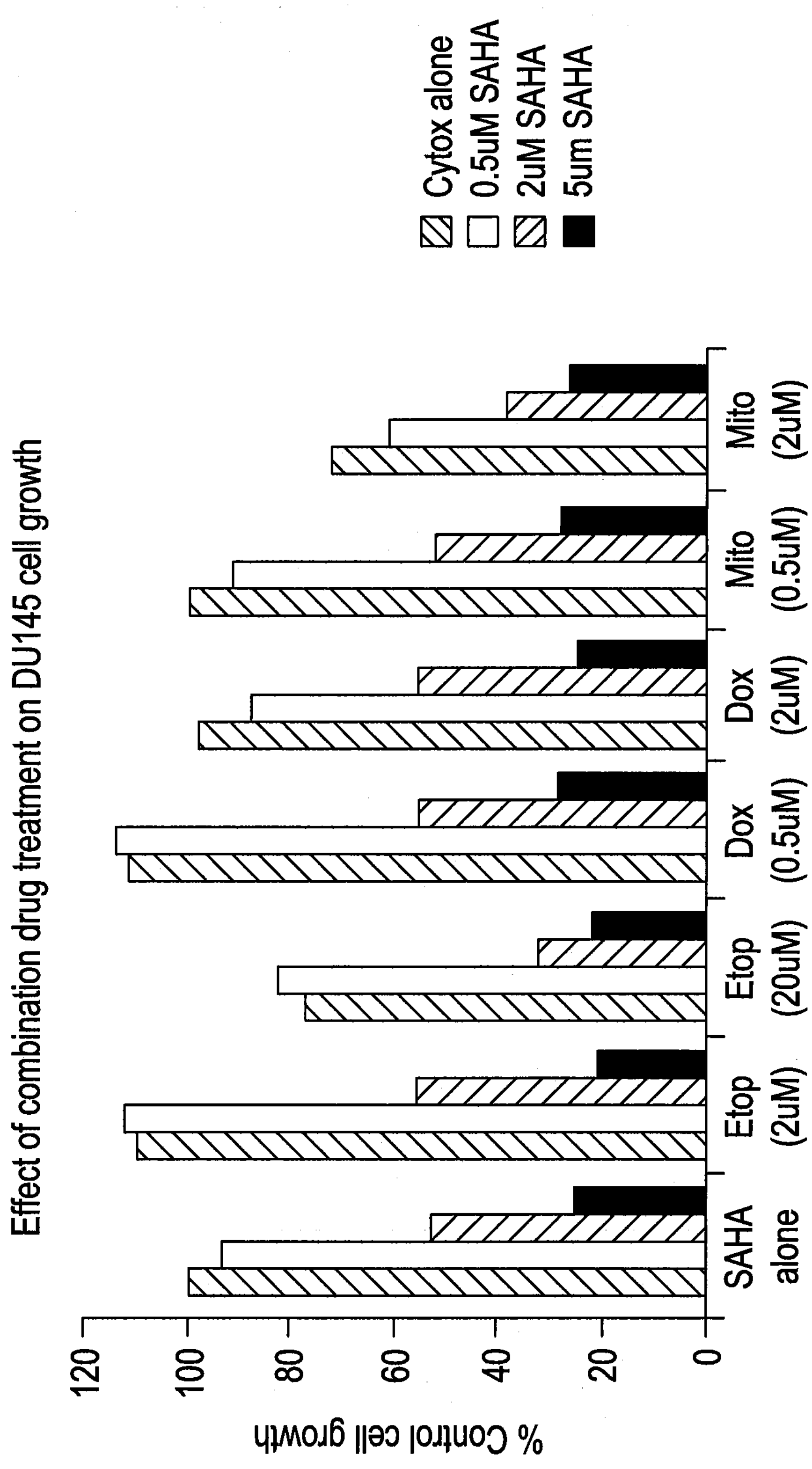
Effect of combination drug treatment on DU145 cell growth
% Control cell growth
0
20
40
60
80
100
120
SAHA alone
Etop (2uM)
Etop (20uM)
Dox (0.5uM)
Dox (2uM)
Mito (0.5uM)
Mito (2uM)
Cytox alone
0.5uM SAHA
2uM SAHA
5um SAHA

FIG. 6

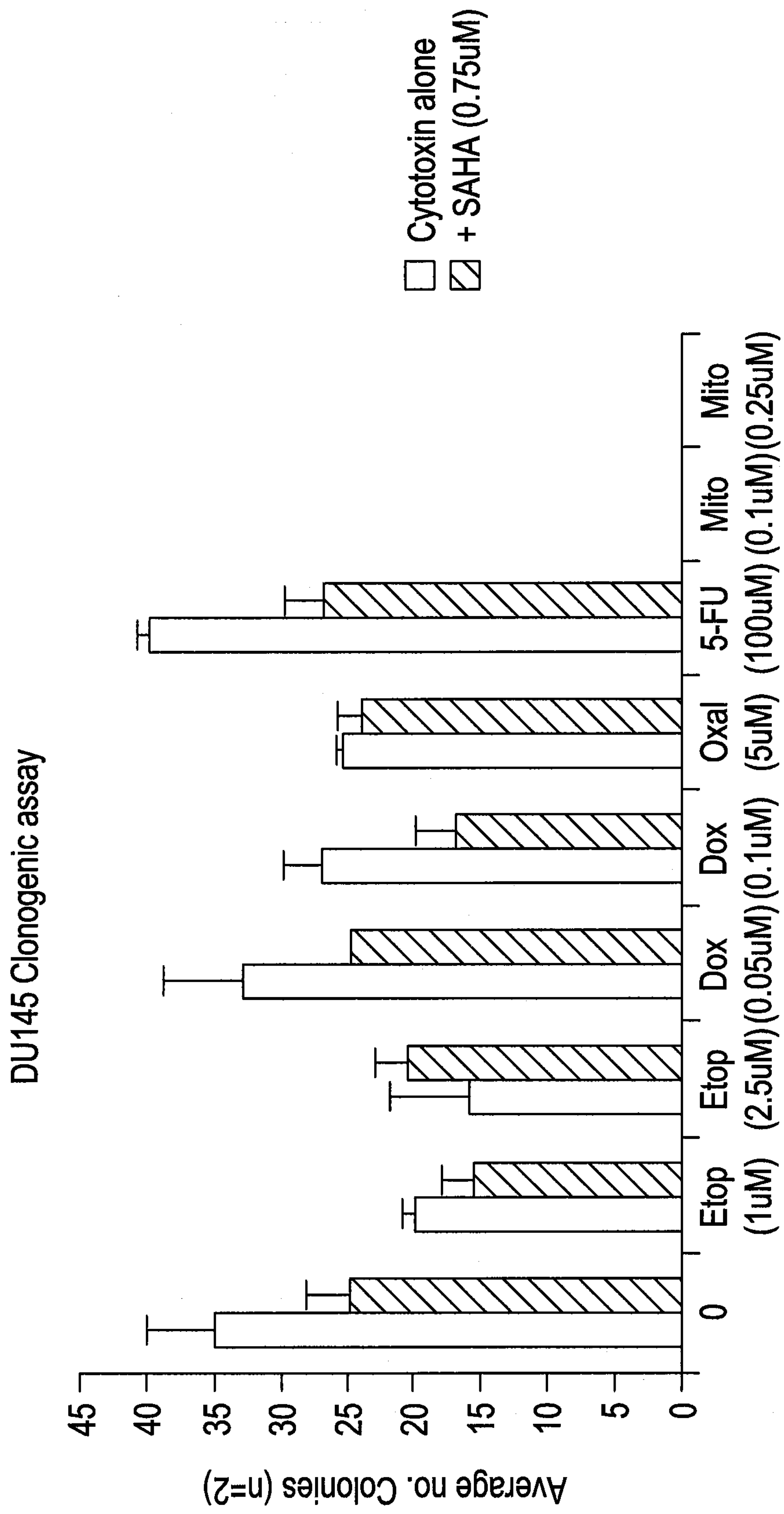
DU145 Clonogenic assay
Average no. Colonies (n=2)
0
5
10
15
20
25
30
35
40
45
0
Etop (1uM)
Etop (2.5uM)
Dox (0.05uM)
Dox (0.1uM)
Oxal (5uM)
5-FU (100uM)
Mito (0.1uM)
Mito (0.25uM)
Cytotoxin alone
+ SAHA (0.75uM)

FIG. 7

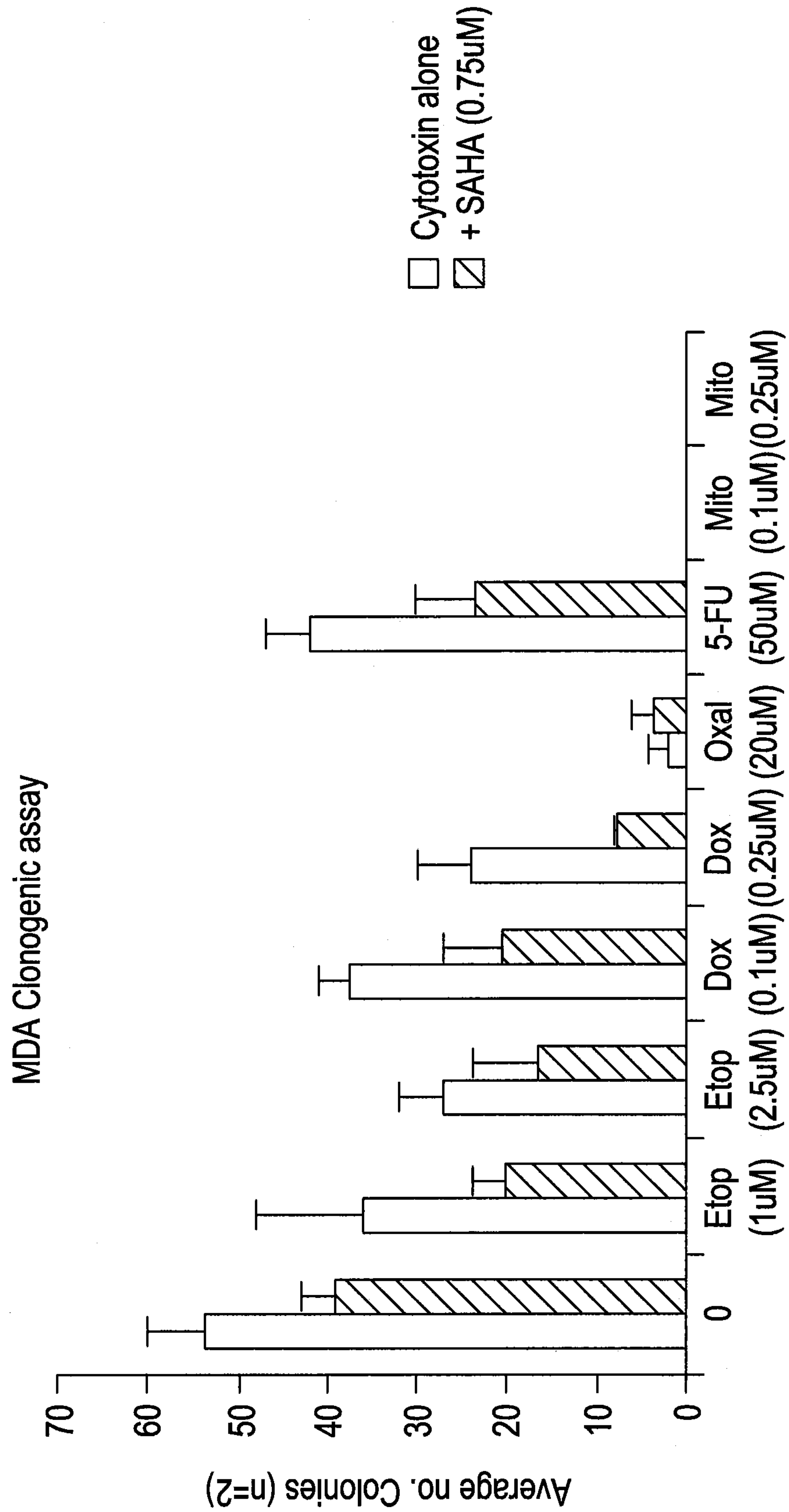
MDA Clonogenic assay
Average no. Colonies (n=2)
0
10
20
30
40
50
60
70
0
Etop (1uM)
Etop (2.5uM)
Dox (0.1uM)
Dox (0.25uM)
Oxal (20uM)
5-FU (50uM)
Mito (0.1uM)
Mito (0.25uM)
Cytotoxin alone
+ SAHA (0.75uM)

FIG. 8

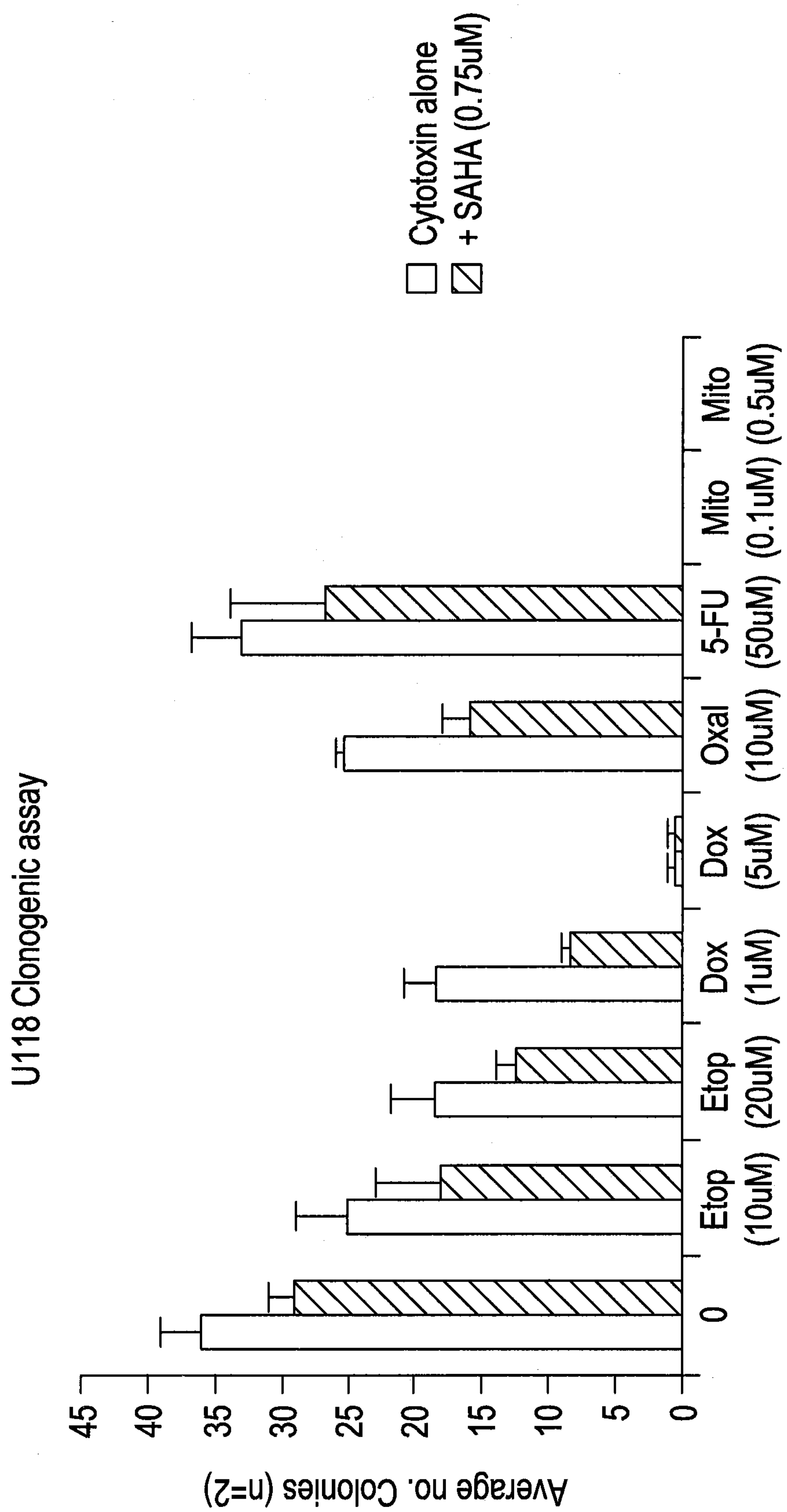
U118 Clonogenic assay
Average no. Colonies (n=2)
0
5
10
15
20
25
30
35
40
45
0
Etop (10uM)
Etop (20uM)
Dox (1uM)
Dox (5uM)
Oxal (10uM)
5-FU (50uM)
Mito (0.1uM)
Mito (0.5uM)
Cytotoxin alone
+ SAHA (0.75uM)

FIG. 9

Percent Inhibition of LNCap Cells Treated with SAHA and Irinotecan

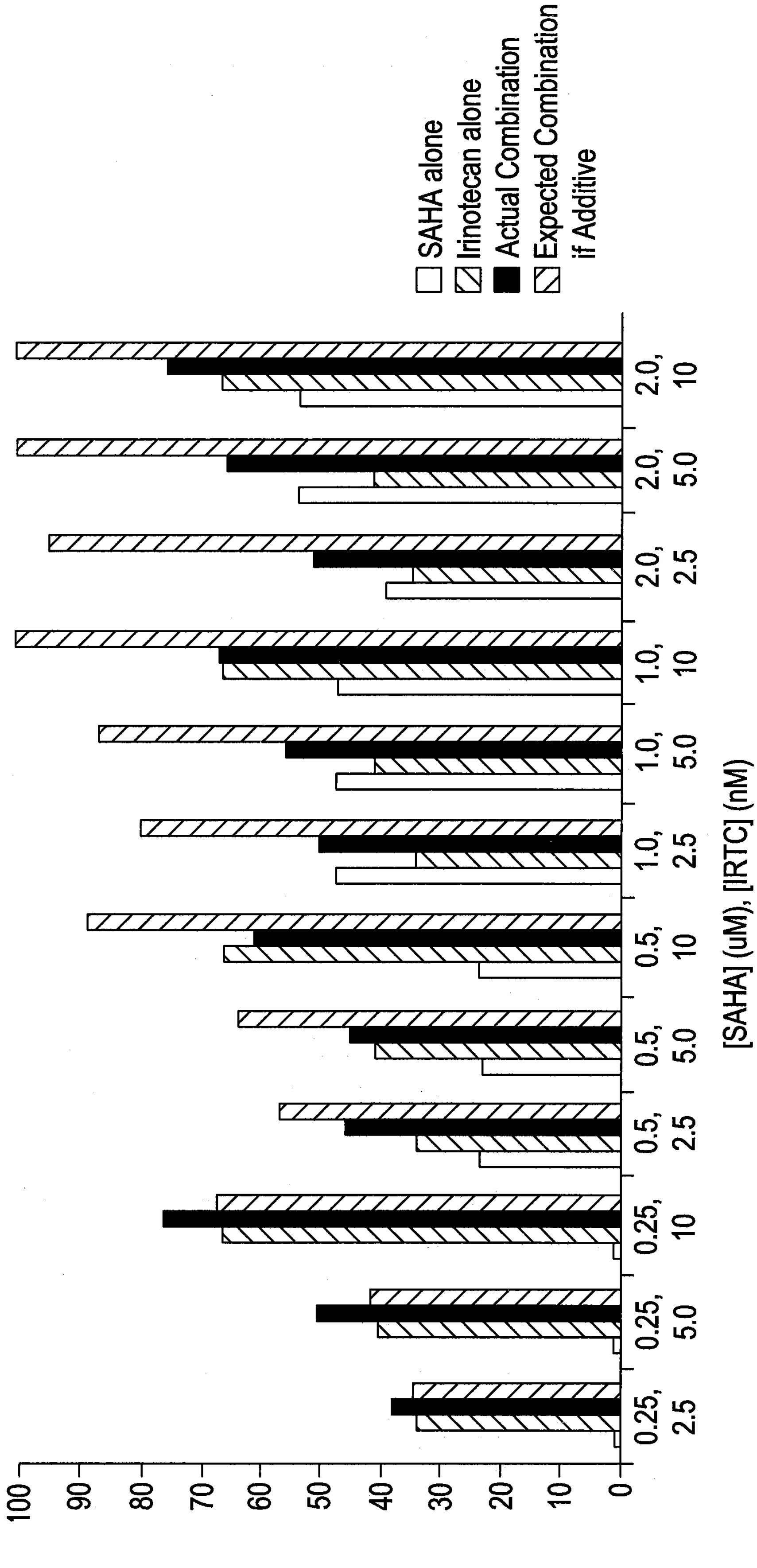

FIG. 10

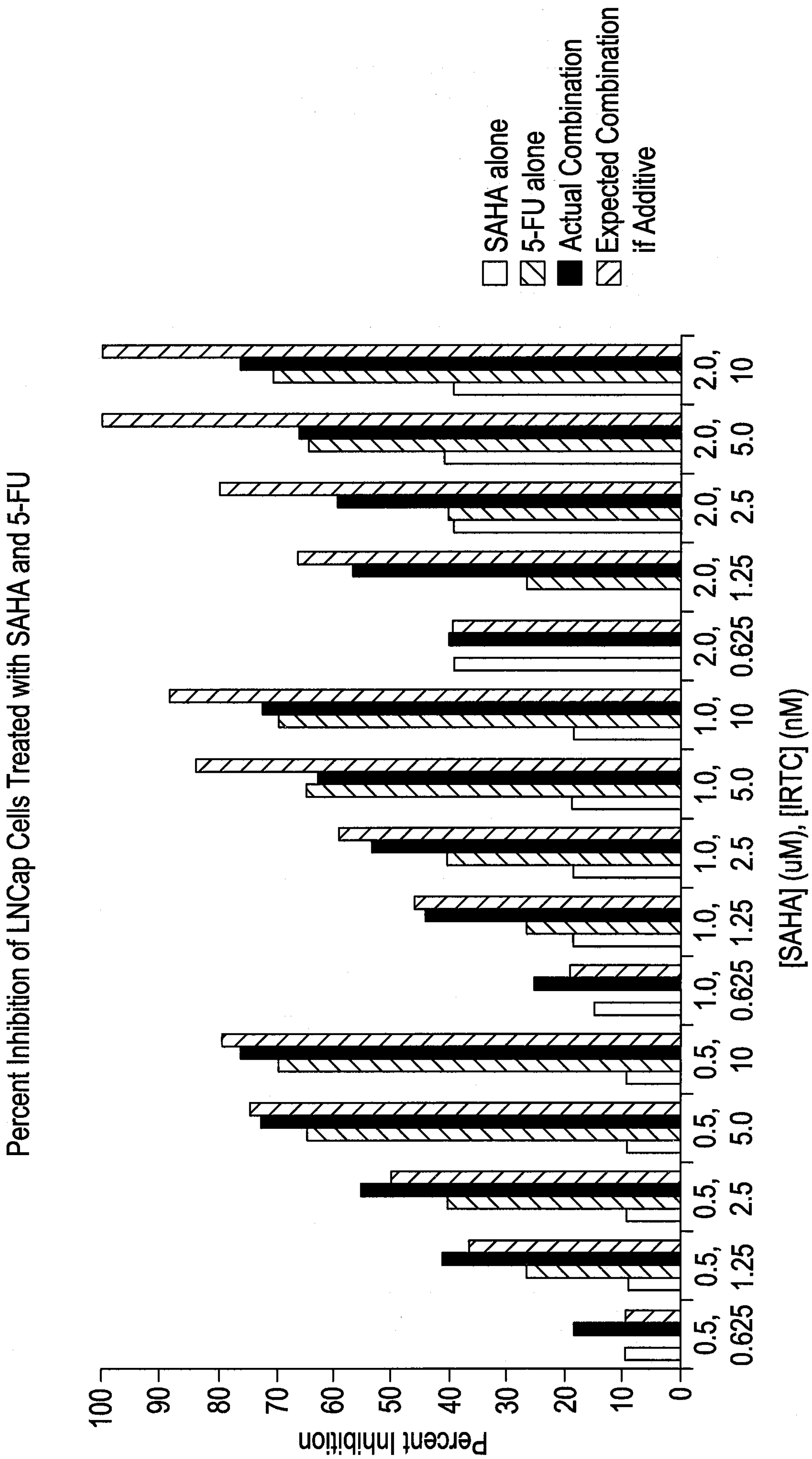
Percent Inhibition of LNCap Cells Treated with SAHA and 5-FU
Percent Inhibition
100
90
80
70
60
50
40
30
20
10
0
0.5, 0.625
0.5, 1.25
0.5, 2.5
0.5, 5.0
0.5, 10
1.0, 0.625
1.0, 1.25
1.0, 2.5
1.0, 5.0
1.0, 10
2.0, 0.625
2.0, 1.25
2.0, 2.5
2.0, 5.0
2.0, 10
[SAHA] (uM), [IRTC] (nM)
SAHA alone
5-FU alone
Actual Combination
Expected Combination if Additive

# FIG. 11

Percent Inhibition of LNCap Cells Treated with SAHA and Docetaxel

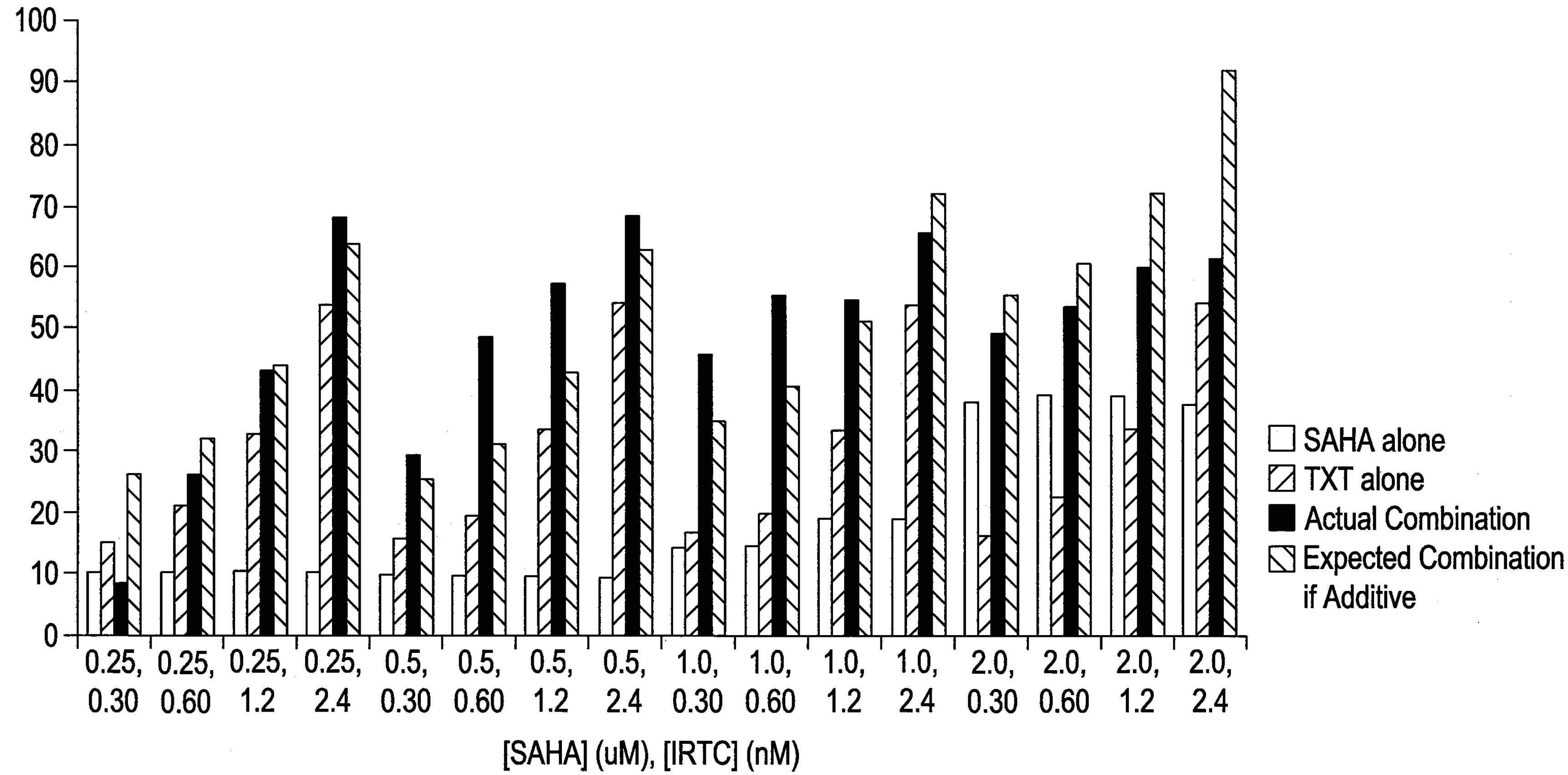

Europäisches Patentamt
European Patent Office
Office européen des brevets

# EUROPEAN SEARCH REPORT

Application Number
EP 10 15 8227

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/085400 A (SUPERGEN INC [US]; DIMARTINO JORGE [US]) 31 October 2002 (2002-10-31) * the whole document, in particular page 7, line 19 - page 8, line 8 and claim 31 * | 1-14 | INV. A61K31/167 A61K31/7068 A61K45/06 A61P35/00 |
| X | "Growth arrest, apoptosis and potentiation of 5-fluorouracil and Raltitrexed cytotoxic effect induced by histone deacetylase inhibitor SAHA in colorectal cancer cells" EUROPEAN JOURNAL OF CANCER, PERGAMON PRESS, OXFORD, GB, vol. 38, 1 November 2002 (2002-11-01), page S29, XP004403521 ISSN: 0959-8049 * abstract * | 1,2,5, 7-10 | |
| X | WEI-GUO ZHU ET AL: "The interaction of histone deacetylase inhibitors and DNA methyltransferase inhibitors in the treatment of human cancer cells" CURRENT MEDICINAL CHEMISTRY. ANTI-CANCER AGENTS, BENTHAM SCIENCE PUBLISHERS, HILVERSUM, NL, vol. 3, no. 3, 1 January 2003 (2003-01-01) , pages 187-199, XP008022883 ISSN: 1568-0118 * the whole document * | 1,5-9,14 | TECHNICAL FIELDS SEARCHED (IPC) |
| | | | A61K |
| Y | US 5 369 108 A (BRESLOW RONALD [US] ET AL) 29 November 1994 (1994-11-29) * column 6, line 14 - line 27 * * column 11, line 3 - line 13 * * column 13, line 26 - line 36 * * column 26, compound * * tables 1,2 * | 7 | |

-/--

The present search report has been drawn up for all claims

1

| Place of search | Date of completion of the search | Examiner |
|---|---|---|
| Munich | 29 July 2010 | Albrecht, Silke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 15 8227

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | HATSE S ET AL: "Role of antimetabolites of purine and pyrimidine nucleotide metabolism in tumor cell differentiation" BIOCHEMICAL PHARMACOLOGY, PERGAMON, OXFORD, GB LNKD- DOI:10.1016/S0006-2952(99)00035-0, vol. 58, no. 4, 15 August 1999 (1999-08-15), pages 539-555, XP007903567 ISSN: 0006-2952 * the whole document * ----- | 7 | |

TECHNICAL FIELDS SEARCHED (IPC)

1

The present search report has been drawn up for all claims

| Place of search | Date of completion of the search | Examiner |
|---|---|---|
| Munich | 29 July 2010 | Albrecht, Silke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 15 8227

This annex lists the patent family members relating to the patent documents cited in the above-mentioned European search report.
The members are as contained in the European Patent Office EDP file on
The European Patent Office is in no way liable for these particulars which are merely given for the purpose of information.

29-07-2010

| Patent document cited in search report | Publication date | Patent family member(s) | Publication date |
|---|---|---|---|
| WO 02085400 A | 31-10-2002 | CA 2443560 A1 | 31-10-2002 |
| | | EP 1389127 A1 | 18-02-2004 |
| | | US 2005159347 A1 | 21-07-2005 |
| | | US 2004204339 A1 | 14-10-2004 |
| US 5369108 A | 29-11-1994 | AT 183185 T | 15-08-1999 |
| | | AU 668696 B2 | 16-05-1996 |
| | | AU 2870392 A | 03-05-1993 |
| | | AU 6206396 A | 17-10-1996 |
| | | CA 2120619 A1 | 15-04-1993 |
| | | DE 69229800 D1 | 16-09-1999 |
| | | DE 69229800 T2 | 27-04-2000 |
| | | EP 0642509 A1 | 15-03-1995 |
| | | ES 2134815 T3 | 16-10-1999 |
| | | FI 941537 A | 31-05-1994 |
| | | HU 67421 A2 | 28-04-1995 |
| | | JP 3432823 B2 | 04-08-2003 |
| | | JP 7502494 T | 16-03-1995 |
| | | JP 2003226680 A | 12-08-2003 |
| | | JP 2007291110 A | 08-11-2007 |
| | | JP 2008069158 A | 27-03-2008 |
| | | KR 100263264 B1 | 01-08-2000 |
| | | KR 100258680 B1 | 15-05-2000 |
| | | RU 2128643 C1 | 10-04-1999 |
| | | WO 9307148 A1 | 15-04-1993 |
| | | US 5932616 A | 03-08-1999 |
| | | US 5700811 A | 23-12-1997 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5369108 A
- US 5932616 A
- US 5700811 A
- US 6087367 A
- US 6511990 B
- US 5608108 A
- FR 901228
- FR 225497
- WO 0008048 A, H. Mori
- WO 9711366 A
- WO 9848825 A
- US 5773474 A
- US 5055608 A
- US 5175191 A
- WO 0118171 A
- WO 02246144 A, Hofmiann-La Roche
- WO 0222577 A
- WO 0230879 A
- WO 0138322 A
- WO 0170675 A
- WO 0071703 A
- WO 0021979 A
- WO 9840080 A
- US 4522811 A

### Non-patent literature cited in the description


- **M. B., Roberts, A. B. ; Driscoll, J. S.** Cancer: Principles and Practice of Oncology. J. B. Lippincott, 1985, 49
- **Breitman, T. R. ; Selonick, S. E. ; Collins, S. J.** *Proc. Natl. Acad. Sci. USA,* 1980, vol. 77, 2936-2940
- **Olsson, I. L. ; Breitman, T. R.** *Cancer Res.,* 1982, vol. 42, 3924-3927
- **Schwartz, E. L. ; Sartorelli, A. C.** *Cancer Res.,* 1982, vol. 42, 2651-2655
- **Sporn ; Marks, P. A. ; Sheffery, M. ; Rifkind, R. A. et al.** *Cancer Res.,* 1987, vol. 47, 659
- **Sachs, L.** *Nature (Lond.),* 1978, vol. 274, 535
- **Friend, C. ; Scher, W. ; Holland, J. W. ; Sato, T.** *Proc. Natl. Acad. Sci. (USA),* 1971, vol. 68, 378-382
- **Tanaka, M. ; Levy, J. ; Terada, M. ; Breslow, R. ; Rifkind, R. A. ; Marks, P. A.** *Proc. Natl. Acad. Sci. (USA),* 1975, vol. 72, 1003-1006
- **Reuben, R. C. ; Wife, R. L. ; Breslow, R. ; Rifkind, R. A. ; Marks, P. A.** *Proc. Natl. Acad. Sci. (USA),* 1976, vol. 73, 862-866
- **Abe, E. ; Miyaura, C. ; Sakagami, H. ; Takeda, M. ; Konno, K. ; Yamazaki, T. ; Yoshika, S. ; Suda, T.** *Proc. Natl, Acad, Sci. (USA),* 1981, vol. 78, 4990-4994
- **Schwartz, E. L. ; Snoddy, J. R. ; Kreutter, D. ; Rasmussen, H. ; Sartorelli, A. C.** *Proc. Am. Assoc. Cancer Res.,* 1983, vol. 24, 18
- **Tanenaga, K. ; Hozumi, M. ; Sakagami, Y.** *Cancer Res.,* 1980, vol. 40, 914-919
- **Lotem, J. ; Sachs, L.** *Int. J. Cancer,* 1975, vol. 15, 731-740
- **Metcalf, D.** *Science,* 1985, vol. 229, 16-22
- **Scher, W. ; Scher, B. M. ; Waxman, S.** *Exp. Hematol.,* 1983, vol. 11, 490-498
- **Scher, W. ; Scher, B. M. ; Waxman, S.** *Biochem. & Biophys. Res. Comm.,* 1982, vol. 109, 348-354
- **Huberman, E. ; Callaham, M. F.** *Proc. Natl. Acad. Sci. (USA),* 1979, vol. 76, 1293-1297
- **Lottem, J. ; Sachs, L.** *Proc. Natl. Acad. Sci. (USA),* 1979, vol. 76, 5158-5162
- **Terada, M. ; Epner, E. ; Nudel, U. ; Salmon, J. ; Fibach, E. ; Rifkind, R. A. ; Marks, P. A.** *Proc. Natl. Acad. Sci. (USA),* 1978, vol. 75, 2795-2799
- **Morin, M. J. ; Sartorelli, A. C.** *Cancer Res.,* 1984, vol. 44, 2807-2812
- **Schwartz, E. L. ; Brown, B. J. ; Nierenberg, M. ; Marsh, J. C. ; Sartorelli, A. C.** *Cancer Res.,* 1983, vol. 43, 2725-2730
- **Sugano, H. ; Furusawa, M. ; Kawaguchi, T. ; Ikawa, Y.** *Bibl. Hematol.,* 1973, vol. 39, 943-954
- **Ebert, P. S. ; Wars, I. ; Buell, D. N.** *Cancer Res.,* 1976, vol. 36, 1809-1813
- **Hayashi, M. ; Okabe, J. ; Hozumi, M.** *Gann,* 1979, vol. 70, 235-238
- **Richon, V.M. ; Webb, Y. ; Merger, R. et al.** *PNAS,* 1996, vol. 93, 5705-8

- **Cohen, L.A. ; Amin, S. ; Marks, P.A. ; Rifkind, R.A. ; Desai, D. ; Richon, V.M.** *Anticancer Research,* 1999, vol. 19, 4999-5006
- **Grunstein, M.** *Nature,* 1997, vol. 389, 349-52
- **Finnin, M.S. ; Donigian, J.R. ; Cohen, A. et al.** *Nature,* 1999, vol. 401, 188-193
- **Van Lint, C. ; Emiliani, S. ; Verdin, E.** *Gene Expression,* 1996, vol. 5, 245-53
- **Archer, S. Shufen ; M. Shei, A. ; Hodin, R.** *PNAS,* 1998, vol. 95, 6791-96
- **Dressel, U. ; Renkawitz, R. ; Baniahmad, A.** *Anticancer Research,* 2000, vol. 20 (2A), 1017-22
- **Lin, R.J. ; Nagy, L. ; Inoue, S. et al.** *Nature,* 1998, vol. 391, 811-14
- **Marks, P.A. et al.** *J. Natl. Cancer Inst.,* 2000, vol. 92, 1210-1215
- **Butler, L.M. et al.** *Cancer Res.,* 2000, vol. 60, 5165-5170
- **Richon, V. M. et al.** *Proc. Natl. Acad. Sci., USA,* 1998, vol. 95, 3003-3007
- **Yoshida, M. et al.** *J. Biol. Chem.,* 1990, vol. 265, 17174-17179
- **Yoshida et al.** *J. Biol. Chem.,* 1990, vol. 265, 17174-17179
- **Richon et al.** *Proc. Natl. Acad. Sci. USA,* 1998, vol. 95, 3003-3007
- **Koghe et al.** *Biochem. Pharmacol.,* 1998, vol. 56, 1359-1364
- **Andrews et al.** *International J. Parasitology,* 2000, vol. 30, 761-768
- **Qiu et al.** *Mol. Biol. Cell,* 2000, vol. 11, 2069-2083
- **Kim et al.** *Oncogene,* 1999, vol. 18, 2461-2470
- **Su et al.** *Cancer Research,* 2000, vol. 60, 3137-3142
- **Kijima et al.** *J Biol. Chem.,* 1993, vol. 268, 22429-22435
- **Nakajima et al.** *Ex. Cell Res.,* 1998, vol. 241, 126-133
- **Darkin-Rattray et al.** *Proc. Natl. Acad. Sci. USA,* 1996, vol. 93, 13143-13147
- **Bosch et al.** *Plant Cell,* 1995, vol. 7, 1941-1950
- **Cousens et al.** *J. Biol. Chem.,* 1979, vol. 254, 1716-1723
- **McBain et al.** *Biochem. Pharm.,* 1997, vol. 53, 1357-1368
- **Lea ; Tulsyan.** *Anticancer Research,* 1995, vol. 15, 879-873
- **Wang et al.** *Cancer Research,* 1999, vol. 59, 2766-2799
- **Guan et al.** *Cancer Research,* 2000, vol. 60, 749-755
- **Saito et al.** *Proc. Natl. Acad. Sci. USA,* 1999, vol. 96, 4592-4597
- **Frey et al.** *Bioorganic & Med. Chem. Lett.,* 2002, vol. 12, 3443-3447
- **Kwon et al.** *PNAS,* 1998, vol. 95, 3356-3361
- **Yoshida, M. et al.** *Bioassays,* 1995, vol. 17, 423-430
- **Saito, A. et al.** *PNAS USA,* 1999, vol. 96, 4592-4597
- **Furamai R. et al.** *PNAS USA,* 2001, vol. 98 (1), 87-92
- **Komatsu, Y. et al.** *Cancer Res.,* 2001, vol. 61 (11), 4459-4466
- **Su, G.H. et al.** *Cancer Res.,* 2000, vol. 60, 3137-3142
- **Lee, B.I. et al.** *Cancer Res.,* vol. 61 (3), 931-934
- **Suzuki, T. et al.** *J. Med. Chem.,* 1999, vol. 42 (15), 3001-3003
- *Expert Opin. Ther. Patents,* 2002, vol. 12 (9), 1375-1384
- **David Kozma.** CRC Handbook of Optical Resolutions via Diastereomeric Salt Formation. CRC Press, 2001
- Cancer Chemotherapy: Principles and Practice. JB Lippincott, 1990
- **Black ; Livingston.** *Drugs,* 1990, vol. 39, 489-501
- *DRUGS,* vol. 39, 652-673
- **Chen ; Grem.** *Curr. Opin. Oncol.,* 1992, vol. 4, 1089-1098
- **Abe, E. ; Miyaura, C. ; Sakagami, H. ; Takeda, M. ; Konno, K. ; Yamazaki, T. ; Yoshika, S. ; Suda, T.** *Proc. Natl. Acad. Sci. (USA),* 1981, vol. 78, 4990-4994
- **Huberman, E. ; Callaham, M. F.** *Proc. Natl. Acad Sci. (USA),* 1979, vol. 76, 1293-1297
- **Ebert, P. S. ; Wars, I. ; Buell, D. N.** *Cancer Res,* 1976, vol. 36, 1809-1813
- **Hardman W.E. et al.** *Br J Cancer,* 1999, vol. 81, 440-448
- **Awada A. et al.** *Eur J. Cancer,* 2002, vol. 38, 773-778
- **Chou T. et al.** *Synergism and Antagonism in Chemotherapy,* 1991